(19) Europäisches Patentamt
European Patent Office
Office européen des brevets

(11) EP 1 923 473 B1

(12) EUROPEAN PATENT SPECIFICATION

(45) Date of publication and mention of the grant of the patent:
**23.06.2010 Bulletin 2010/25**

(21) Application number: **06380268.0**

(22) Date of filing: **17.10.2006**

(51) Int Cl.:
***C22B 3/26*** *(2006.01)* ***G21C 19/46*** *(2006.01)*
***C07C 235/08*** *(2006.01)* ***C07C 235/10*** *(2006.01)*
***C07D 311/82*** *(2006.01)* ***C22B 60/02*** *(2006.01)*

(54) **Bis-diglycolamides (BISDGA) as new extractants for lanthanides [Ln(III)] and actinides [An(III)] from aqueous high-level wastes**

Bis-Diglykolamide (BISDGA) als neue Extraktionsmittel für Lanthanoide [La(III)] und Actinide [An(III)] in wässrigem, hochradioaktivem Abfall

Bis-diglycolamides (BISDGA) en tant qu'agents d'extraction de lanthanides [La(III)] et d'actinides [An (III)] des dechets aqueux à activité elevée

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**21.05.2008 Bulletin 2008/21**

(73) Proprietors:
- **Universidad Autónoma de Madrid 28049 Madrid (ES)**
- **Institut Catala D'Investigacio Quimica 43007 Tarragona (ES)**
- **Centro de Investigaciones Energéticas, Medioambientales y Tecnológicas 28040 Madrid (ES)**
- **Forschungszentrum Jülich GmbH 52425 Jülich (DE)**

(72) Inventors:
- **Murillo Mendoza, M. Teresa C/.Einstein 3 28049 Madrid (ES)**
- **Sánchez Quesada, Jorge C/. Einstein, 3 28049 Madrid (ES)**
- **Almaraz González, Marta C/. Einstein, 3 28049 Madrid (ES)**
- **Mendoza Sans, Javier 43007 Tarragona (ES)**
- **González Espartero, Amparo 28040 Madrid (ES)**
- **Modolo, Giuseppe 52425 Jülich (DE)**
- **Prados Hernando, Pilar C/. Einstein, 3 28049 Madrid (ES)**

(74) Representative: **Carpintero Lopez, Francisco et al Herrero & Asociados, S.L. Alcalá 35 28014 Madrid (ES)**

(56) References cited:
**FR-A1- 2 810 679 JP-A- 2003 322 699**

- **DATABASE WPI Week 200533 Derwent Publications Ltd., London, GB; AN 2005-319661 XP002423579 & JP 2005 114448 A (JAPAN ATOMIC ENERGY RES INST) 28 April 2005 (2005-04-28)**
- **YUJI SASAKI AND SHOICHI TACHIMORI: "EXTRACTION OF ACTINIDES(III), (IV), (V), (VI) AND LANTHANIDES(III) BY STRUCTURALLY TAILORED DIAMIDES" SOLVENT EXTRACTION AND ION EXCHANGE, vol. 20, no. 1, 2002, pages 21-34, XP009080173**
- **YUJI SASAKI ET AL: "THE NOVEL EXTRACTANTS, DIGLYCOLAMIDES, FOR THE EXTRACTION OF LANTHANIDES AND ACTINIDES IN HNO3-n-DODECANE SYSTEM" SOLVENT EXTRACTION AND ION EXCHANGE, vol. 19, no. 1, 2001, pages 91-103, XP009080172**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 923 473 B1

## Description

### FIELD OF THE INVENTION

The present invention relates to the field of recovery of radioactive materials. More specifically, it relates to the synthesis of new bis-diglycolamides and their use as extractants for lanthanides and actinides from aqueous nitric solutions in general, and from high-level radioactive liquid wastes in particular.

### BACKGROUND OF THE INVENTION

One of the options for handling irradiated fuel discharged from nuclear reactors is the so-called "closed cycle" option in which after a cooling period, the fuel elements are subject to a hydrometallurgical process called *PUREX* (Plutonium Uranium Refining by Extraction) for the purpose of recovering the non fissioned uranium and the generated plutonium and using them to manufacture new nuclear fuels. This process consists in dissolving the bars of the irradiated fuel elements in concentrated nitric acid (3 mol/L - 6 mol/L), releasing gas products (Kr, Xe, I, etc.) and eliminating the insoluble precipitates by filtration. In these conditions, uranium and plutonium oxidize to U(VI) and Pu(IV), separating them by extraction from the remaining elements, using an organic phase formed by a kerosene-type aliphatic solvent which contains 30% tri-n-butyl phosphate (TBP), the trivalent actinides (americium and curium) together with most of the fission and activation products remaining in the aqueous phase. (See: a) McKay, H. A. C. The PUREX Process, Part I, Introduction in Science and Technology of Tributyl Phosphate, Ed.: Schulz, W. W.; Navratil, J. D., CRC Press: Boca Raton 1984; b) Miles, J. H. The PUREX Process, Part II, Separation of Plutonium and Uranium in Science and Technology of Tributyl Phosphate, Ed.: Schulz, W. W.; Navratil, J. D., CRC Press: Boca Raton 1984; c) McKibben, J. M. Radiochim. Acta 1984, 36, 3; and d) Washitani, T.; Ozawa, M.; Kawada, T.; Hayashi, S. 1993, JP5093795)

The option for handling the aqueous residue called High *Level Liquid Waste (HLLW)*, which has more international consensus from the technical and safety point of view, is its immobilization in a glass matrix and subsequent storage in a deep geological repository (DGR).

This option is questioned by several opinion groups who argue its potential hazards due to the presence of radionuclides having a long half-life period and high radiotoxicity. This has brought about the study and consideration of new management alternatives focused on decreasing the radiotoxicity present in HLLW, mainly due to the presence of the so-called minor actinides (MA): Am, Cm, Bk and Cf.

Two HLLW management strategies are currently contemplated: the so-called "separation and transmutation (S&T)" consisting in separating the radionuclides having a long half-life period to prepare blanks with high chemical purity, which allow their transmutation in elements with a lower half-life period or which are stable; and "separation and conditioning (S&C)", consisting in separating the radionuclides with a long half-life period to immobilize them in stable crystalline matrixes and subsequently storing them. In both cases it is necessary to have sufficiently selective and effective separation methods. Minor actinides are present in the HLLW coming from the PUREX process in a trivalent oxidation state [An(III)]. The selective extraction of these An(III) is a complex problem due to the high concentration of trivalent lanthanides [Ln(III)] in the HLLW and due to the fact that these two families of cations have very similar chemical properties.

For this reason, their separation has been approached by means of an extraction process using steps. Therefore, the DIAMEX (DIAMide EXtraction) process, which uses *N,N,N',N'*-tetra-alkyl malonamides, allows jointly extracting An (III)/Ln(III), being separated from the other fission products. (See: a) Hubert, H.; Musikas, C. 1986, US 4572802; b) Thiollet, G.; Lafosse, L.; Musikas, C.; Hoel, P. 1987, FR 2585700; c) Musikas, C.; Hoel, P.; Thiollet, G.; Lafosse, L. 1990, US 4938871; and d) Spjuth, L.; Liljenzin, J. O.; Hudson, M. J.; Drew, M. G. B.; Iveson, P. B.; Madic, C. Solvent Extr. Ion Exch. 2000, 18, 1). Then the SANEX (Selective ActiNide Extraction) process is applied to selectively separate the An (III) from the Ln (III) without the presence of the fission products and at diluted nitric acid concentrations, using extractants having soft atoms such as nitrogen or sulfur. (See: a) Fitoussi, R.; Musikas, C.; Ranarivello, H. 1982, EP 0043765; b) Fitoussi, R.; Musikas, C.; Ranarivello, H. 1984, US 4461747; c) Bonnin, M.; Musikas, C.; Vitorge P. 1985, CA 1197382; d) Smith, B. F.; Jarvinen, G. D.; Ryan, R. R. 1989, US 4867951; and e) Cordier P.-Y., Cuillierdier C., Musikas C., 1993, US 5256383).

On 2001, Sasaki et al. described that diglycolamides and especially N,N,N',N'-tetraoctyl diglycolamide (TODGA), have distribution coefficients which are greater than those of malonamides for the co-extraction of An(III) and Ln(III). These coefficients increase when the nitric acid concentration of the aqueous solution increases. This ligand is further resistant to hydrolysis and to radiolysis although it forms third phases at high metal concentrations, which makes it difficult to separate An(III) and Ln(III). In addition to TODGA, other diglycolamides have particularly been described which form complexes with Am (III) and Cm (III), such as TDDGA (N,N,N',N'-tetradecyl-3-oxapentane-diamide), TDoDGA (N,N,N',N'-tetradodecyl-3-oxapentane-diamide), TMDGA (N,N,N',N'-tetramethyl-diglycolamide), TBDGA (N,N,N',N'-tetrabutyl-3-oxapentane-diamide or DMDODGA (N,N-dimethyl-N',N'-dioctyl-oxapentane-diamide (See: a) Sasaki, Y.;

Yaita, T.; Narira, H.; Tachimori, S. 2001, FR 2810679; b) Sasaki, Y.; Sugo, Y.; Suzuki, S.; Tachimori, S. Solvent Extraction and Ion Exchange, 2001, 19(1), 91-103; c) Sasaki, Y.; Tachimori, S.; Solvent Extraction and Ion Exchange, 2002, 20 (1), 21-34; d) Tachimori, S.; Sasaki, Y.; Suzuki, S. Solvent Extraction and Ion Exchange, 2002, 20(6), 687-699; e) Tatemori K., Sasaki Y., 2003, JP 2003322699; f) Sasaki Y., Suzuki S., Kimura T., 2005, JP 2005114448; g) Ye, G. -A. ; He, J. -Y. ; Jiang, Y. -Q. He Huaxue Yu Fangshe Huaxue, 2000, 22(2), 65-72; h) Ye, G.-A.; He, J.-Y. ; Luo, F.-X. He Huaxue Yu Fangshe Huaxue, 2000, 22(3), 136-143.; i) Chen, W.-J.; Zhu, L.; Ding, S.-D.; Liu, Z.-Q.; Chen, S.-J.; Jin, Y.-D. Gaodeng Xuexiao Huaxue Xuebao, 1998, 19(11), 1724-1726; j) Chen, W.; Wen, Y.; Fan, Q.; He Huaxue Yu Fangshe Huaxue, 1999, 21(3), 136-141; and k) Ding, S.-D.; Chen, W.J.; Xiao, C.-J.; Lu, Q.; Luo, H.-Y.; Liu, J.-F.; Ye, G.-A.; Zhu, Z.-X.; Tang, H.-B.; Luo, F.-X. He Huaxue Yu Fangshe Huaxue, 2003, 25 (3), 188-192).

However, most of said extractants have as their main problems low solubility in aliphatic solvents, third phase formation, and the extraction of considerable amounts of Sr(II), Zr(II) and Pd (II) together with An(III). Co-extractants are added to prevent any of these drawbacks, which complicates the process by increasing the amount of radioactive wastes.

Therefore, in the state of the art there is still a need to provide alternative extracting agents for Ln(III) and An (III) that overcome the previously mentioned drawbacks.

The authors have discovered that by using certain bis-diglycolamides, which are bound through aromatic or aliphatic spacers, good distribution coefficients are achieved during the process of extraction of An(III) and Ln(III) from aqueous nitric acid solutions in a wide range of concentrations (from 0.01 M to 7 M), with reduced extraction times (less than 5 minutes) and preventing the formation of the third phase modifying slightly the solvent used for the extraction. Furthermore, these compounds have a greater hydrolytic and radiolytic stability than TODGA in the same conditions, as well as a greater selectivity in the extraction of An(III) and Ln(III) compared to the other fission products present in the high-level liquid waste such as, for example, Sr(II), which avoids an additional step in the process. Finally, An(III) and Ln(III) are recovered easily from the organic phase to an aqueous phase, the organic phase being regenerated for new extraction cycles, thus reducing the costs of the industrial process.

Therefore, the present invention provides new alterative compounds for the extraction of lanthanides [Ln(III)] and actinides [An(III)] from aqueous nitric solutions, particularly HLLW, with better properties than those described to date.

## OBJECT OF THE INVENTION

An object of the present invention is to provide new bis-diglycolamide compounds having formula (I) suitable for the extraction of lanthanides [Ln(III)] and actinides [An(III)] from aqueous nitric solutions.

Another object of the present invention is to provide a process for the preparation of said bis-diglycolamides compounds having formula (I).

Another object of the present invention is to provide the use of said bis-diglycolamides compounds having formula (I) for the extraction of lanthanides [Ln(III)] and actinides [An(III)] from aqueous nitric solutions.

Another object of the present invention is to also provide a method for the extraction of lanthanides [Ln(III)] and actinides [An(III)] from an aqueous nitric solution using said bis-diglycolamides compounds having formula (I).

Finally, another object of the present invention is to provide an extracting solution for the joint separation of lanthanides [Ln(III)] and actinides [An(III)] from aqueous nitric solutions and, preferably high-level radioactive liquid wastes, comprising said bis-diglycolamide compound having general formula (I) in a mixture of organic solvents.

## DESCRIPTION OF THE DRAWINGS

Figure 1 shows the kinetics of Am(III)/Eu(III) extraction from a 3 mol/L aqueous nitric acid solution by a 0.1 mol/L organic solution of compound 3 in 1-octanol and in a "TPH/1-octanol $(50:50)_{\%vol}$" mixture.

Figure 2 shows the kinetics of Am(III)/Eu(III) extraction from a 3 mol/L aqueous nitric acid solution by a 0.1 mol/L organic solution of compound 4 in n-heptane and in 1-octanol.

Figure 3 shows the kinetics of Am(III)/Eu(III) extraction from a 3 mol/L aqueous nitric acid solution by a 0.1 mol/L organic solution of compound 6 in a " TPH/1-octanol $(50:50)_{\%vol}$" mixture, and by a 0.1 mol/L organic solution of each of the compounds 9, 10 and 11 in a "TPH/1-octanol $(75:25)_{\%vol}$" mixture.

Figure 4 shows the kinetics of Am(III)/Eu(III) extraction from a 3 mol/L aqueous nitric acid solution by a 0.1 mol/L organic solution of compound 14 in 1-octanol and in a "TPH/1-octanol $(50:50)_{\%vol}$" mixture.

Figure 5 shows the kinetics of Am(III)/Eu(III) extraction from a 3 mol/L aqueous nitric acid solution by a 0.1 mol/L organic solution of compound 15 in 1-octanol.

Figure 6 shows the kinetics of Am(III)/Eu(III) extraction from a 3 mol/L aqueous nitric acid solution by a 0.1 mol/L organic solution of compound 16 in a "TPH/1-octanol $(50 : 50)_{\%vol}$" mixture and by a 0.1 mol/L organic solution of

compound 18 in a "TPH/1-octanol $(50:50)_{\%vol}$" mixture.
Figure 7 shows the kinetics of Am(III)/Eu(III) extraction from a 3 mol/L aqueous nitric acid solution by a 0.1 mol/L organic solution of compound 19 in 1-octanol.
Figure 8 shows the extraction capability of compound 4 dissolved at 0.1 mol/L in 1-octanol and in "TPH/1-octanol $(50:50)\%_{vol}$, $(75:25)_{\%vol}$, and $(90:10)_{\%vol}$" mixtures, for different nitric acid concentrations (0.06 mol/L < $HNO_3$ < 6.7 mol/L) of the solution containing Am(III)/Eu(III).

Figure 9 shows the extraction capability of compound 16 dissolved at 0.1 mol/L in a "TPH/1-octanol $(50:50)_{\%vol}$" mixture and in a "TPH/1-octanol $(95:5)_{\%vol}$" mixture, for different nitric acid concentrations (0.06 mol/L < $HNO_3$ < 6.7 mol/L) of the solution containing Am(III)/Eu(III).

Figure 10 shows the extraction capability of compound 18 dissolved at 0.1 mol/L in a "TPH/1-octanol $(50:50)_{\%vol})$" mixture and in a "TPH/1-octanol $(95:5)_{\%vol}$" mixture, for different nitric acid concentrations (0.06 mol/L < $HNO_3$ < 6.7 mol/L) of the solution containing Am(III)/Eu(III).

Figure 11 shows the loading capacity of compound 4 dissolved at 0.2 mol/L in a "TPH/1-octanol $(95:5)_{\%vol}$" mixture for different concentrations of Eu(III) in 3 mol/L nitric acid solution.

Figure 12 shows the loading capacity of compounds 16 and 18 each dissolved at 0.2 mol/L in a "TPH/1-octanol $(95:5)_{\%vol}$" mixture for different concentrations of Eu(III) in 3 mol/L nitric acid solution.

Figure 13 shows the extraction capability of compound 4 dissolved at 0.1 mol/L in a "n-dodecane/1-octanol $(90:10)_{\%vol}$" mixture of Am(III), Cm(III), Cf(III) and Eu(III) from a 3 mol/L nitric acid solution to which different amounts of oxalic acid have been added.

Figure 14 shows the evolution of the concentration of Am(III) and Eu(III) in the organic phase formed by a 0.1 mol/L solution of compound 4 in a "TPH/1-octanol (95: 5) %vol" mixture in the different steps of extraction, scrubbing and re-extraction of Am(III) and Eu(III) with 0.01 mol/L nitric acid solutions.

Figure 15 shows the evolution of the concentration of nitric acid in the aqueous phase in the different steps of extraction, scrubbing and re-extraction of Am(III) and Eu(III) with 3.0, 1.0 and 0.01 mol/L nitric acid solutions respectively from an organic phase formed by a 0.1 mol/L solution of compound 4 in a "TPH/1-octanol $(95:5)_{\%vol}$" mixture.

Figure 16 shows the evolution of the concentration of Am(III) and Eu(III) in the organic phase formed by a 0.1 mol/L solution of compound 18 in a "TPH/1-octanol $(95: 5)_{\%vol}$" mixture in the different steps of extraction, scrubbing and re-extraction of Am(III) and Eu(III) with 0.01 mol/L nitric acid solutions.

Figure 17 shows the evolution of the concentration of nitric acid in the aqueous phase in the different steps of extraction, scrubbing and re-extraction of Am(III) and Eu(III) with 3.0, 1.0 and 0.01 mol/L nitric acid solutions respectively from an organic phase formed by a 0.1 mol/L solution of compound 18 in a "TPH/1-octanol $(95:5)_{\%vol}$" mixture.

Figure 18 shows the hydrolytic stability of 0.1 mol/L organic solutions of compounds 4 and 18 in the "n-dodecane/1-octanol $(95:5)_{\%vol}$" mixture in permanent contact time periods of 66 days and 42 days respectively, with 3 mol/L nitric acid solutions.

Figure 19 shows the radiolytic stability of 0.1 mol/L organic solutions of compound 4 without solvent and dissolved in the "n-dodecane/1-octanol $(95:5)_{\%vol}$" mixture up to an integrated dose of 1000 kGy at a dose rate of 4 kGy/h.

Figure 20 shows the radiolytic stability of 0.1 mol/L organic solutions of compound 18 without solvent and dissolved in the "n-dodecane/1-octanol $(95:5)_{\%vol}$" mixture up to an integrated dose of 1000 kGy at a dose rate of 4 kGy/h.

## DETAILED DESCRIPTION OF THE INVENTION

The invention provides a compound having general formula (I)

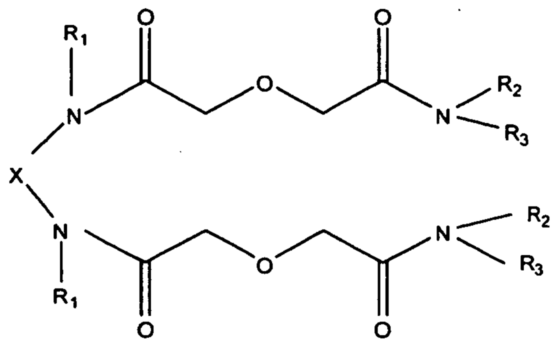

(I)

wherein
X represents a spacer selected from:

a) a xylylene radical non-substituted or optionally substituted with an $R_4O$ alcoxy group, wherein $R_4$ is a linear or branched $C_6$-$C_{12}$ alkyl group,
b) a $C_5$-$C_7$ alkylidene radical,
c) a $R_5$-O-$R_5$ group, wherein $R_5$ is a $C_2$-$C_3$ alkylidene radical, and
d) a xanthene-derived group having formula (II)

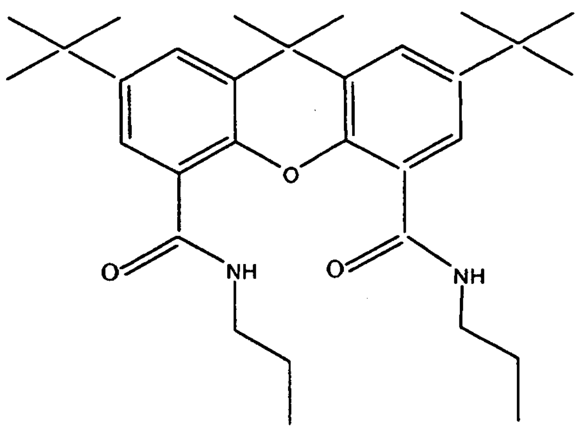

(II)

$R_1$ is an H atom or a $C_1$-$C_3$ alkyl radical,
$R_2$ is an H atom or a linear or branched $C_1$-$C_8$ alkyl radical, and
$R_3$ is a linear or branched $C_4$-$C_8$ alkyl radical,

In the context of the present invention, the term "xylylene" refers both to an m-xylylene radical and to a p-xylylene radical. Likewise, the term "alkyl" refers both to a linear chain alkyl group and to a branched chain alkyl group.

Without intending to be limited to a particular theory, it is believed that the binding of more than one diglycolamide subunit by means of the suitable aromatic or aliphatic spacer, would pre-organize the ligands in the complexing of Ln (III) and An(III), thus reducing the energy of the coordination process and probably improving the distribution coefficients or even the selectivity of one of them.

In a particular embodiment, the compounds of the invention are compounds having formula (I) wherein X is a non-substituted xylylene radical, $R_1$ is an H atom or a methyl radical, $R_2$ is an H atom or an alkyl radical selected from methyl, butyl, isopentyl and octyl, and $R_3$ is an alkyl radical selected from butyl, isopentyl and octyl.

In another particular embodiment, the compounds of the invention are compounds having formula (I) wherein

X is a xylylene radical substituted with an $R_4O$ alcoxy group, wherein $R_4$ is an alkyl radical selected from hexyl, sec-heptyl and dodecyl, $R_1$ is an H atom, and $R_2$ and $R_3$ are each a butyl radical.

In another particular embodiment, the compounds of the invention are compounds having formula (I) wherein X is a $C_5$-$C_7$ alkylidene radical, $R_1$ is an H atom, and $R_2$ and $R_3$ are each an alkyl radical selected from butyl and octyl.

In another particular embodiment, the compounds of the invention are compounds having formula (I) wherein X is a $R_5$-O-$R_5$ group, wherein $R_5$ is a $C_2$-$C_3$ alkylidene radical, $R_1$ is an H atom, and $R_2$ and $R_3$ are each an octyl radical.

In another particular embodiment, the compounds of the invention are compounds having formula (I) wherein X is a xanthene-derived group having formula (II), $R_1$ is an H atom, and $R_2$ and $R_3$ are each a butyl radical.

Table 1 shows illustrative examples of the compounds of the present invention:

**TABLE 1- Compounds having general formula (I)**

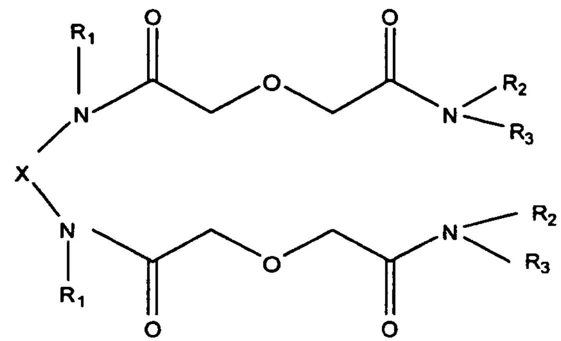

| Compound no. | X | $R_1$ | $R_2$ | $R_3$ |
|---|---|---|---|---|
| 1 | 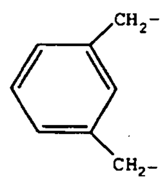 | H | H | $-(CH_2)_3-CH_3$ |
| 2 | 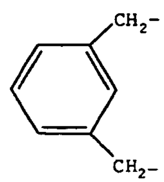 | H | $-CH_3$ | $-(CH_2)_3-CH_3$ |
| 3 | 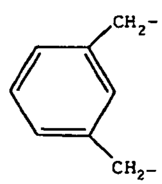 | H | $-(CH_2)_3-CH_3$ | $-(CH_2)_3-CH_3$ |

(continued)

| Compound no. | X | $R_1$ | $R_2$ | $R_3$ |
|---|---|---|---|---|
| 4 | 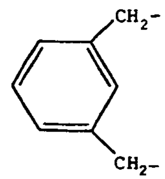 | H | $-(CH_2)_7-CH_3$ | $-(CH_2)_7-CH_3$ |
| 5 | 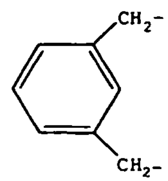 | H | $-CH_3$ | $-(CH_2)_7-CH_3$ |
| 6 | 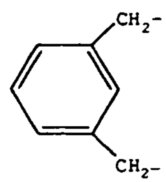 | H | 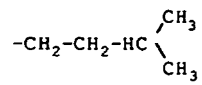 | 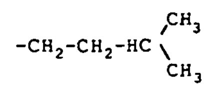 |
| 7 | 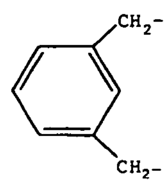 | $-CH_3$ | $-CH_3$ | $-(CH_2)_7-CH_3$ |
| 8 | 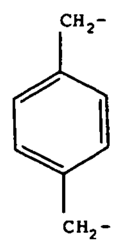 | H | $-(CH_2)_7-CH_3$ | $-(CH_2)_7-CH_3$ |

(continued)

| Compound no. | X | $R_1$ | $R_2$ | $R_3$ |
|---|---|---|---|---|
| 9 | 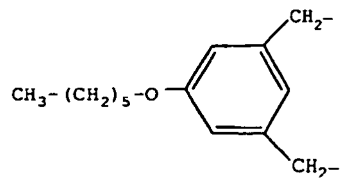 | H | $-(CH_2)_3-CH_3$ | $-(CH_2)_3-CH_3$ |
| 10 | 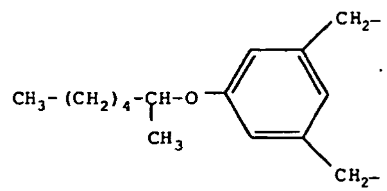 | H | $-(CH_2)_3-CH_3$ | $-(CH_2)_3-CH_3$ |
| 11 | 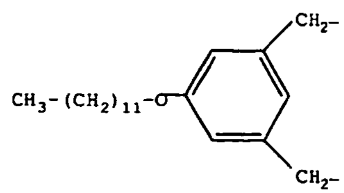 | H | $-(CH_2)_3-CH_3$ | $-(CH_2)_3-CH_3$ |
| 12 | 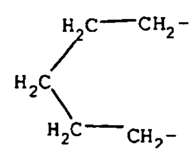 | H | $-(CH_2)_3-CH_3$ | $-(CH_2)_3-CH_3$ |
| 13 | 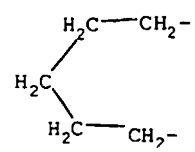 | H | $-(CH_2)_7-CH_3$ | $-(CH_2)_7-CH_3$ |

(continued)

| Compound no. | X | $R_1$ | $R_2$ | $R_3$ |
|---|---|---|---|---|
| 14 | 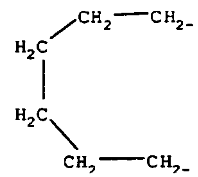 | H | $-(CH_2)_3-CH_3$ | $-(CH_2)_3-CH_3$ |
| 15 | 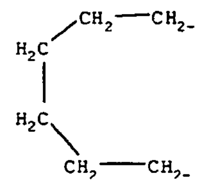 | H | $-(CH_2)_7-CH_3$ | $-(CH_2)_7-CH_3$ |
| 16 | 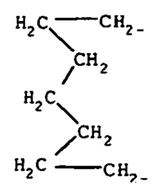 | H | $-(CH_2)_7-CH_3$ | $-(CH_2)_7-CH_3$ |
| 17 | 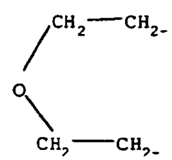 | H | $-(CH_2)_7-CH_3$ | $-(CH_2)_7-CH_3$ |
| 18 | 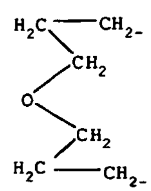 | H | $-(CH_2)_7-CH_3$ | $-(CH_2)_7-CH_3$ |

(continued)

| Compound no. | X | $R_1$ | $R_2$ | $R_3$ |
|---|---|---|---|---|
| 19 | 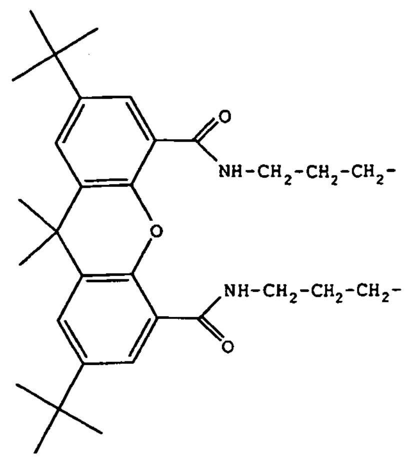 | H | $-(CH_2)_3-CH_3$ | $-(CH_2)_3-CH_3$ |

Thus, in a preferred embodiment of the present invention, the compounds having formula (I) are selected from the following group of compounds:

[1] 2-Butylcarbamoylmethoxy-*N*-[3-[(2-butylcarbamoylmethoxy-acetylamino)methyl]benzyl]acetamide,

[2] 2-[(Butyl-methylcarbamoyl)methoxy]-N-[3-[(2-butylmethyl-carbamoyl)methoxyacetylamino]methyl]benzyl] acetamide,

[3] 2-Dibutylcarbamoylmethoxy-N-[3-[(2-dibutylcarbamoyl-methoxy-acetylamino)methyl]benzyl]acetamide,

[4] 2-Dioctylcarbamoylmethoxy-N-[3-[(2-dioctylcarbamoyl-methoxy-acetylamino)methyl]benzyl]acetamide,

[5] 2-[(Methyloctylcarbamoyl)methoxy]-N-[3-[(2-[(methyl-octyl-carbamoyl)methoxy]acetylamino]methyl]benzyl]-acetamide,

[6] 2-[[Bis(3-methylbutyl)carbamoyl]methoxy]-N-[3-[(2-[[bis-(3-methylbutyl)carbamoyl]methoxy]acetylamino)methyl]-benzyl]-acetamide,

[7] N-Methyl-N-[3-[(methyl-[2-[(methyloctylcarbamoyl)-methoxy]-acetyl]amino)methyl]benzyl]-2-[(methyloctyl-carbamoyl)methoxy]acetamide,

[8] 2-[[4-([2-[Dioctylcarbamoyl)methoxy]acetylamino]methyl)-benzylcarbamoyl]methoxy]-N,N-dioctylacetamide,

[9] 2-Di-butylcarbamoylmethoxy-N-[3-[(2-dibutylcarbamoyl-methoxyacetylamino)methyl]-5-hexyloxybenzyl]acetamide,

[10] 2-Di-butylcarbamoylmethoxy-N-[3-[(2-dibutyl-carbamoyl-methoxyacetylamino)methyl]-5-(1-methylhexyloxy) benzyl]-acetamide,

[11] 2-Di-butylcarbamoylmethoxy-N-[3-[(2-dibutyl-carbamoyl-methoxyacetylamino)methyl]-5-dodecyloxybenzyl] acetamide,

[12] 2-Dibutylcarbamoylmethoxy-N-[5-(2-dibutylcarbamoyl-methoxyacetylamino)pentyl]acetamide,

[13] 2-Dioctylcarbamoylmethoxy-N-[5-(2-dioctylcarbamoyl-methoxyacetylamino)pentyl]acetamide,

[14] 2-Dibutylcarbamoylmethoxy-N-[6-(2-dibutylcarbamoyl-methoxyacetylamino)hexyl]acetamide,

[15] 2-Dioctylcarbamoylmethoxy-N-[6-(2-dioctylcarbamoyl-methoxyacetylamino)hexyl]acetamide,

[16] 2-Dioctylcarbamoylmethoxy-N-[7-(2-dioctylcarbamoyl-methoxyacetylamino)heptyl]acetamide,

[17] 2-Dioctylcarbamoylmethoxy-N-[2-[2-(2-dioctylcarbamoyl-methoxyacetylamino)ethoxy]ethyl]acetamide,

[18] 2-Dioctylcarbamoylmethoxy-N-[3-[3-(2-dioctylcarbamoyl-methoxyacetylamino)propoxy]propyl]acetamide, and

[19] 2,7-di-tert-butyl-9,9-dimethyl-9H-xanthen-4,5-dicarboxylic acid bis-[[3-(2-dibutylcarbamoylmethoxy-acetylamino)propyl]amide].

**Synthesis of bis-diglycolamides with aromatic spacers**

Compounds 1-7 and 9-11 have two diglycolamides bound to a m-xylylene aromatic ring, whereas compound 8 has a p-xylylene spacer providing a greater distance between the ligands.

Compounds 1-8 are obtained by reacting a suitable carboxylic acid (obtained in turn by the reaction of diglycolic anhydride with the selected aliphatic amine) with the corresponding m-xylylene- or p-xylylenediamine in a conventional inert solvent and in the presence of a suitable activating agent and/or base selected by a person skilled in the art. Thus, 1-ethyl-3-[3-dimethylaminopropyl]carbodiimide (EDC) or acids activated via acid chloride in solvents such as dichloromethane or benzene can be used as activating agents. The activation is also possible by means of other agents such

as benzotriazol-1-yloxy-tris(dimethyl-amino)phosphonium hexafluorophosphate (BOP), (benzotriazol-1-yloxy)tripyrrolidinophosphonium hexafluorophosphate (PyBOP), 1,1'-carbonyl-diimidazole (CDI), dicyclohexylcarbodiimide (DCC), and other activating agents used in the synthesis of peptides (amide bonds), optionally in the presence of an organic base such as a tertiary amine, preferably triethylamine, the used solvents being dimethylformamide (DMF), N-methylpyrrolidone (NMP), dimethylsulphoxide (DMSO), chlorinated solvents or tetrahydrofuran (THF), or any other aprotic polar organic solvent, for example. Finally, it is also possible, although less recommended, to obtain the compounds by aminolysis of the diglycolamide acid ester.

Thus, another aspect of the present invention provides a process for obtaining compounds having formula (I), wherein X is a non-substituted xylylene radical, comprising reacting a compound having formula (III)

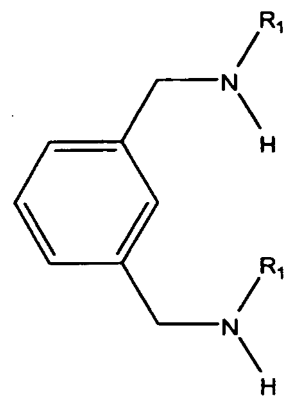

(III)

with a compound having formula (IV)

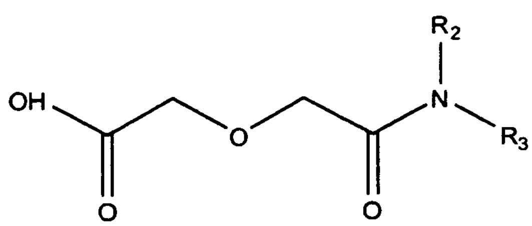

(IV)

in an inert solvent and in the presence of an activating agent, $R_1$, $R_2$ and $R_3$ having the previously mentioned meaning. EDC is preferably used as an activating agent and dichloromethane as a solvent.

Compounds 9-11 are also obtained in a similar manner from the suitable diamine (with a protected hydroxyl group) which is reacted with the suitable carboxylic acid and is then subjected to hydrogenolysis and subsequent introduction of an alkylic chain to form the alcoxy group of the xylylene ring.

Thus, a process is provided for obtaining compounds having formula (I) wherein X is a xylylene radical substituted with an $R_4O$ alcoxy group comprising reacting a compound having formula (VIII),

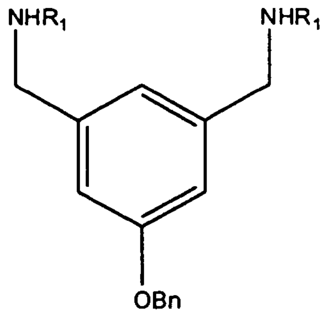

(VIII)

with a compound having formula (IV) in an inert solvent and in the presence of an activating agent, followed by a hydrogenation reaction, in an inert solvent and in the presence of a hydrogenation catalyst, giving rise to a compound having formula (IX),

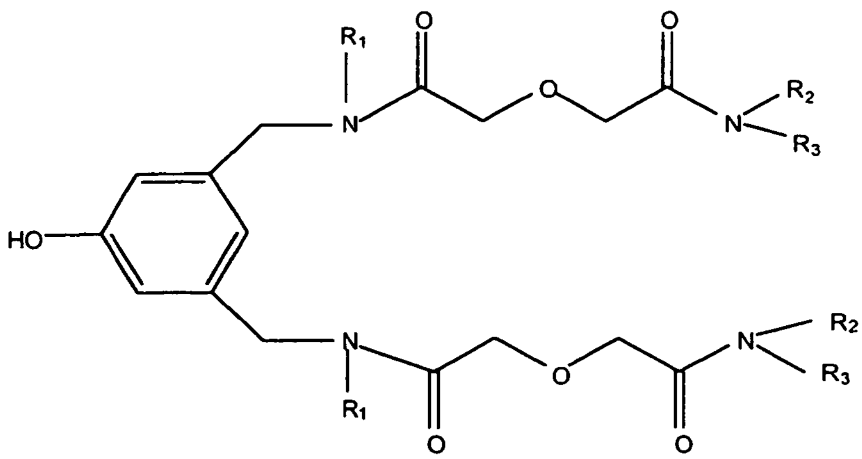

(IX)

followed by an O-alkylation reaction with $BrR_4$, in an inert solvent and in the presence of a base, $R_1$, $R_2$, $R_3$ and $R_4$ having the previously mentioned meaning.

The reaction of the diamine having formula (VIII) with the acid having formula (IV) is preferably carried out using EDC.HCl as an activating agent and dichloromethane as a solvent. The hydrogenation reaction is also carried out preferably using 10% Pd on carbon as a hydrogenation catalyst and ethanol as a solvent. The O-alkylation is also carried out preferably using an inorganic base such as $K_2CO_3$ and acetonitrile as a solvent at a temperature of 80°C.

The diamine having formula (VIII) is obtained by O-benzylation of dimethyl 5-hydroxyisophthalate (commercially available) followed by the reduction of the ester groups with $LiAlH_4$ to obtain a dialcohol, which in turn is reacted with thionyl chloride to obtain a dichlorine derivative which is finally reacted with potassium phthalimide (Gabriel synthesis).

Thus, in a particular embodiment, the compound having formula (VIII) is obtained by O-benzylation with benzyl bromide (BrBn) of a compound having formula (V),

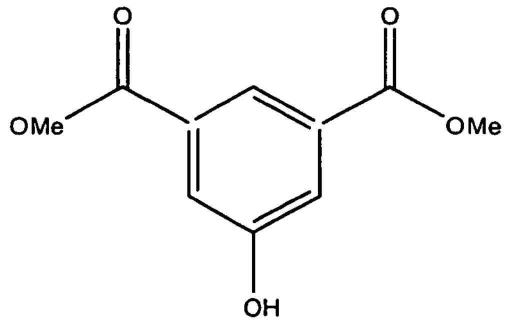

(V)

in an inert solvent and in the presence of a base, followed by a reduction reaction with $LiAlH_4$ in an inert solvent, and by the reaction of the formed dialcohol having formula (VI)

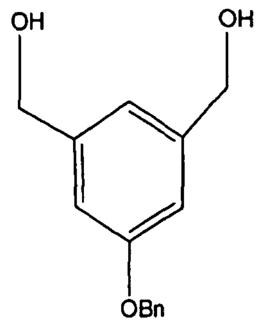

(VI)

with $SOCl_2$ giving rise to a compound having formula (VII)

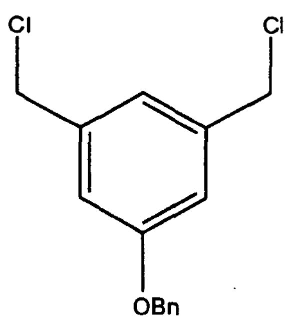

(VII)

which is subjected to a Gabriel synthesis reaction with potassium phthalimide, in an inert solvent and in the presence of sodium iodide, followed by treatment with hydrazine in an inert solvent, to obtain the compound having formula (VIII).

The O-benzylation of the compound having formula (V) is preferably carried out using an inorganic base such as $K_2CO_3$ and DMF as solvent. The subsequent reduction is also preferably carried out using THF as an inert solvent. Likewise, the reaction of the compound (VII) with potassium phthalimide (Gabriel synthesis) is preferably carried out with DMF as a solvent at a temperature of 80°C, whereas the treatment with hydrazine is preferably carried out in ethanol.

**Synthesis of bis-diglycolamides with alkyl and alcoxy spacers**

A new series of compounds was prepared by replacing the aromatic spacer with a flexible alkyl or alcoxy chain (Compounds 12-18). This new family of extractants belongs to one of the following categories:

a) Compounds including an alkyl diamine in which the nitrogen atoms are separated by five carbon atoms like in the m-xylylene (compounds 12 and 13), and compounds including an alkyl diamine in which this distance has been increased to six and seven carbon atoms between the diglycolamide subunits (Compounds 14 to 16).
Commercial penta-, hexa- and hepta-methylenediamines as starting materials. These amines are reacted with the corresponding carboxylic acids to provide the compounds 12-16 with moderate to good yields, using an activating agent and/or a base in a suitable solvent such as those specified previously.
b) Compounds having a methylene group of the alkyl spacer mentioned in a) replaced by an oxygen atom (Compounds 17-18). Compounds 17 and 18 were prepared with good yields from the diamines available in the market by reacting them with the suitable acid according to the process described previously, in the presence of an activating agent and/or a base in a suitable solvent such as those specified previously.

Therefore, a process is provided for obtaining compounds having formula (I) wherein X is a $C_5$-$C_7$ alkylidene comprising reacting a compound having formula (X)

$$HNR_1\text{-}(CH_2)n\text{-}NHR_1 \qquad (X)$$

with a compound having formula (IV) in an inert solvent and in the presence of an activating agent, $R_1$, $R_2$, and $R_3$ having the previously mentioned meaning and wherein n is 5, 6 or 7. Said reaction is preferably carried out using EDC as an activating agent and dichloromethane as a solvent.

On the other hand, a process is provided for obtaining compounds having formula (I) wherein X is a $R_5$-O-$R_5$ group, comprising reacting a compound having formula (XI)

$$HNR_1\text{-}(CH_2)n\text{-}O\text{-}(CH_2)n\text{-}NHR_1 \qquad (XI)$$

with a compound having formula (IV), in an inert solvent and in the presence of an activating agent, $R_1$, $R_2$, and $R_3$ having the previously mentioned meaning and wherein n is 2 or 3. Said reaction is preferably carried out using EDC as an activating agent and dichloromethane as a solvent.

**Synthesis of bis-diglycolamides with xanthene spacers**

Diglycolamide subunits are bound by means of a spacer based on xanthene containing additional amide groups. Compound 19 was synthesized with an acceptable yield from the suitable diamine and acid using a coupling agent such as EDC.

Therefore, a process is provided for obtaining compounds having formula (I) wherein X is a xanthene-derived group, comprising reacting a compound having formula (XIV)

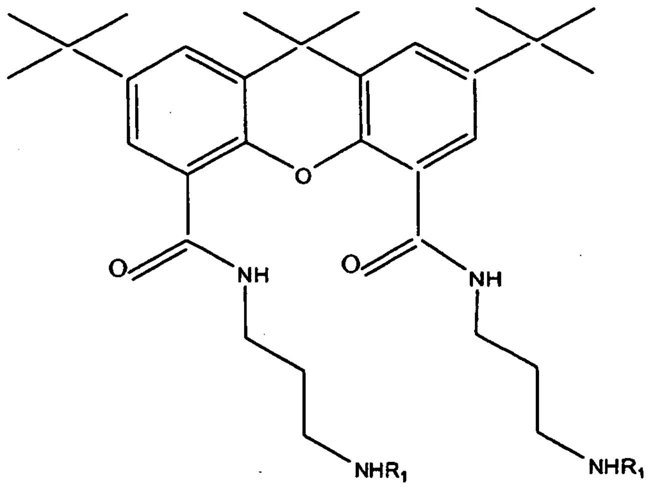

(XIV)

with a compound having formula (IV), in an inert solvent and in the presence of an activating agent, $R_1$, $R_2$ and $R_3$ having the previously mentioned meaning. Said reaction is preferably carried out using EDC as an activating agent and dichloromethane as a solvent.

In a particular embodiment, the compound having formula (XIV) is obtained by reacting the commercial compound having formula (XII)

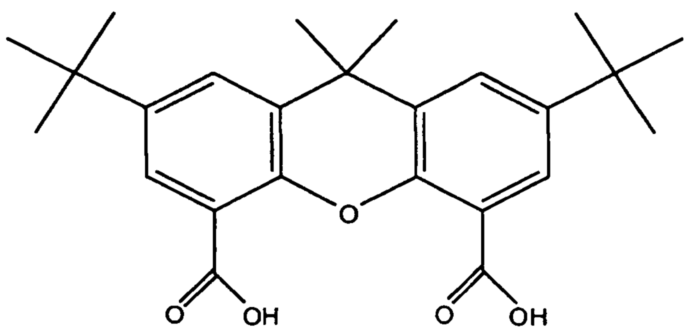

(XII)

with a compound having formula (XIII)

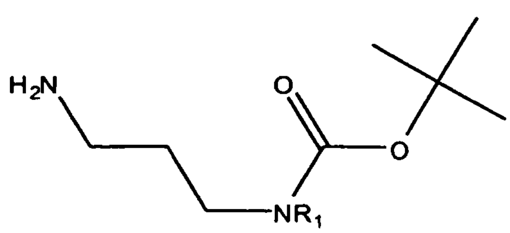

(XIII)

$R_1$ having the previously mentioned meaning, in an inert solvent and in the presence of an activating agent and of an

organic base, followed by treatment with acid. Said reaction is preferably carried out using dichloromethane as a solvent and in the presence of PyBOP as an activating agent and of a base such as triethylamine, followed by treatment with trifluoroacetic acid.

On the other hand, another aspect of the invention also provides the use of the previously described compounds having formula (I) for the extraction of lanthanides [Ln(III)] and actinides [An(III)] present in aqueous nitric solutions. In a preferred embodiment, said aqueous nitric solutions are high-level radioactive liquid wastes.

Another aspect of the present invention also provides a method for the extraction of lanthanides [Ln(III)] and actinides [An(III)] from an aqueous nitric solution comprising:

a) putting the aqueous nitric solution containing lanthanides [Ln(III)] and actinides [An(III)] in contact with an organic solution comprising an extractant having general formula (I) and an organic solvent, and
b) extracting the actinides [An(III)] and the lanthanides [Ln(III)] from the acid solution, after a contact time period.

In a particular embodiment of the method of the present invention, the contact time period of step b) is 1 to 5 minutes.

In another embodiment of the method of the present invention, the organic solvent of the organic solution is selected from tetrachloroethane, n-heptane, 1-octanol, TPH, n-dodecane and mixtures thereof. In a preferred embodiment, the solvent of the organic solution is selected from a mixture of TPH and 5-10% (v/v) of 1-octanol and a mixture of n-dodecane and 5-10% (v/v) of 1-octanol. In a more preferred embodiment, the solvent of the organic solution is a mixture of TPH and 5% (v/v) of 1-octanol.

Another aspect of the present invention provides an extracting organic solution for the extraction of lanthanides [Ln(III)] and actinides [An(III)] in aqueous nitric solutions, and preferably in high-level radioactive liquid wastes, comprising an extractant having formula general (I) as described previously, and a solvent selected from tetrachloroethane, n-heptane, 1-octanol, TPH, n-dodecane and mixtures thereof. In a preferred embodiment, the solvent is selected from a mixture of TPH and 5-10% (v/v) of 1-octanol and a mixture of n-dodecane and 5-10% (v/v) of 1-octanol. In a more preferred embodiment, the solvent is a mixture of TPH and 5% (v/v) of 1-octanol.

The following examples are set forth for the purpose of providing the person skilled in the art with a sufficiently clear and complete explanation of the present invention. In no case they must be considered as limiting the scope thereof.

<u>General processes</u>

Analysis. The melting points are determined in a Gallenkamp apparatus in open tubes. The $^1$H NMR and $^{13}$C NMR spectra were carried out in Bruker AC-300 ($^1$H-300 MHz) and DRX-500 ($^1$H-500 MHz) spectrometers in the solvents indicated in each case. The chemical shifts are expressed in parts per million (ppm, $\delta$) referred to the residual peak of the solvent. The mass spectra were recorded in a VG Autospec spectrometer, using the FAB$^+$ technique, a Reflex chromatograph for the MALDI-TOF ionization and an LCMS Hewlett Packard 1100 apparatus (quadrupole analyzer, 3000 uma) for the ESI and APCI ionization. The data are expressed in m/z units and the values between brackets correspond to the relative intensities of the different ions with respect to the base peak. The elemental analyses were carried out in a Perkin-Elmer 2400 CHN analyzer and are expressed in percentages.

Chromatography. Alugram Sil G/UV254 (Macherey-Nagel) plates were used for the thin layer chromatography (TLC), using ultraviolet light, phosphomolybdic acid (in EtOH or in $CeSO_4/H_2SO_4$ conc/$H_2O$), bromocresol green, ninhydrin and 50% sulphuric acid in EtOH as developing agents. SDS silica gel, Chromagel 60 AC.C, 40-60 $\mu$m, Chromagel 60 AC.C, 70-200 $\mu$m and Scharlau ASTM 60, 40-60 $\mu$m were used for column chromatography, following the process described by W. C. Still (Still, W. C.; Kahn, M.; Mitra, A. J. Org. Chem. 1978, 43, 2923).

**Synthesis**. All the commercial reagents (Acros, Aldrich, Fluka, NovaBiochem, Panreac) were used without any additional purification. The used solvents were dried and distilled according to conventional methods (See, for example, Perrin, D. D.; Armarego, W. L. F.; Perrin, D. R. Purification of Laboratory Chemicals, 2a Ed.; Pergamon Press: Oxford, 1980*).*

**General process for obtaining diglycolic acids**

The corresponding amine is added to a 0.8 M diglycolic anhydride solution in THF and is stirred for 48 h at room temperature. Afterwards, 1 M HC1 is added to the reaction and it is stirred until the appearance of a precipitate, which is filtered and washed with $H_2O$.

***N*-butyl-carbamoylmethoxy-acetic acid (20a)**

The general process described above was followed, from diglycolic anhydride (1 g, 8.6 mmol) and n-butylamine

(740 mg, 10.1 mmol). After drying the precipitate, acid 20a was obtained as a white solid (1.24 g, 76%).
M.p. 69 ˚C
$^1$H NMR ($CDCl_3$, 300 MHz) δ: 10.23 (bs, 1H, OH), 7.13 (bs, 1H, NH), 4.18 (s, 2H, $OCH_2$) , 4.14 (s, 2H, $OCH_2$), 3.29 (m, 2H, $CH_2N$), 1.50 (m, 2H, $CH_2$), 1.34 (m, 2H, $CH_2$), 0.91 (t, $^3J$= 7.33 Hz, 3H, $CH_3$).
$^{13}$C NMR ($CDCl_3$, 75 MHz) δ: 172.4; 170.1 (CO), 70.8; 68.5 ($OCH_2$), 38.9 ($CH_2N$), 31.2; 19. 9 ($CH_2$), 13. 6 ($CH_3$).
MS ($FAB^+$) : m/z 190.2 ($[M+H]^+$, 100 %).

| Elemental analysis | | | |
|---|---|---|---|
| Calculated for $C_5H_{15}NO_4$: | C 50.78; | H 7.99; | N 7.40. |
| Found: | C 50.65; | H 7.81; | N 7.40. |

**2-[(*N*-butyl-*N*-methyl-carbamoyl)methoxy]acetic acid (20b)**

The general process described above was followed, from the diglycolic anhydride (1 g, 8.6 mmol) and N-methylbutylamine (800 mg, 9.2 mmol). After drying the precipitate, acid 20b was obtained as a wax (1.5 g, 86%).
$^1$H NMR ($CDCl_3$, 300 MHz) δ: 4.36 (d, J=7.05 Hz, 2H, $CH_2O$) , 4.14 (s, 2H, $CH_2O$), 3.34 (t, J=7.48 Hz, 1H, $NCH_2$) , 3.09 (t, J=7.48 Hz, 1H, $NCH_2$), 2.91; 2.85 (m, 3H, $NCH_3$), 1.52-1.39 (m, 2H, $CH_2$) , 1.35-1.20 (m, 2H, $CH_2$) , 0.91-0.83 (m, 3H, $CH_3$).
$^{13}$C NMR ($CDCl_3$, 75 MHz) δ: 171.9; 170.7 (CO), 72.0; 70.9; 70.7; 48.3; 48.2 ($CH_2$), 33.6 ($NCH_3$), 29.8; 28.8; 19.7; 19. 6 ($CH_2$), 13.5 ($CH_3$).
MS ($FAB^+$): m/z 204.0 ($[M+H]^+$, 100%).

| Elemental analysis | | | |
|---|---|---|---|
| Calculated for $C_9H_{17}NO_4$.1/2$H_2O$: | C 50.93; | H 8.55; | N 6.60. |
| Found: | C 50.70; | H 8.13; | N 6.51. |

**2-[(N-octyl-*N*-methyl-carbamoyl)methoxy]acetic acid (20c)**

The general process described above was followed, from diglycolic anhydride (4 g, 34 mmol) and N-methyloctylamine (6.8 mL, 38 mmol). After drying the precipitate, acid 20c was obtained as a wax (6.45 g, 72%).
$^1$H NMR ($CDCl_3$, 500MHz) δ: 10.14 (bs, 1H, COOH), 4.44; 4.42 (s, 2H, $OCH_2$); 4.24 (s, 2H, $OCH_2$), 3.44; 3.16 (m, 2H, $CH_2N$), 3.02; 2.94 (s, 3H, $CH_3N$) , 1.58 (m, 2H, $CH_2$), 1.32 (m, 10H, $CH_2$) , 0.93 (m, 3H, $CH_3$).
$^{13}$C NMR ($CDCl_3$, 125MHz) δ: 171.83; 171.78; 170.94; 170.9 (CO), 72. 9; 71.4; 71.1 ($CH_2O$), 48. 8; 48. 0 ($CH_2N$), 33. 9; 33.7 ($CH_3N$) , 31.8; 31.7; 29.3; 29.2; 29.1; 28.0; 26.9; 26.8; 26.7; 26.6; 22.6; 22.5 ($CH_2$), 14.1; 14.0 ($CH_3$) .
MS (ESI+): m/z 540.9 ($[2M+Na]^+$, 100%).

| Elemental analysis | | | |
|---|---|---|---|
| Calculated for $CH_{15}NO_4 \cdot H_2O$: | C 56.30; | H 9.81; | N 5.05. |
| Found: | C 56.74; | H 9.78; | N 5.15. |

**2-[(*N*,*N*-dibutyl-carbamoyl)methoxy]acetic acid (20d)**

It was synthesized according to the method described in Stephan, H.; Gloe, K.; Beger, J.; Muehl, P., Solvent Ext. and Ion Exch. 1991, 9, 435.

**2-[(N,N-dioctyl-carbamoyl)methoxy]acetic acid (20e)**

It was synthesized according to the method described in Stephan, H.; Gloe, K.; Beger, J.; Muehl, P., Solvent Ext. and Ion Exch. 1991, 9, 435, and in Stephan, H.; Gloe, K.; Beger, J.; Muehl, P., Solvent Ext. and Ion Exch. 1991, 9, 459.

**[[N,N-Bis-(3-methylbutyl)carbamoyl]methoxy]acetic** acid (20f)

The general process described above was followed, from diglycolic anhydride (4 g, 34 mmol) and diisoamylamine (6 g, 38 mmol). After drying the precipitate, acid 20f was obtained as an oil (7.53 g, 81%).
$^1$H NMR ($CDCl_3$, 500 MHz) δ: 4.41 (s, 2H, $CH_2O$), 4.23 (s, 2H, $CH_2O$) , 3.39 (t, $^3J$=7.9 Hz, 2H, $NCH_2$) , 3. 12 (t, $^3J$=7.9 Hz, 2H, $NCH_2$) , 1.6 (septuplet, $^3J$=6.7 Hz, 2H, CH), 1.48-1.44 (m, 4H, $CH_2$), 0.97 (d, $^3J$=6.7 Hz, 3H, $CH_3$), 0.95 (d, $^3J$=6.7

Hz, 3H, $CH_3$) .
$^{13}C$ NMR ($CDCl_3$, 125 MHz) δ: 172.0; 170.4 (CO), 72.7; 71.0 ($CH_2O$), 45.3; 45.2 ($NCH_2$), 37.4; 36.2 ($CH_2$), 26.2; 26.1 (CH), 22.4; 22.3 ($CH_3$) .
MS (MALDI-TOF): m/z 274.2 ($[M+H]^+$, 100%).

| Elemental analysis | | | |
|---|---|---|---|
| Calculated for $C_{14}H_{27}NO_4$: | C 61.51; | H 9.96; | N 5.12. |
| Found: | C 60.99; | H 9.87; | N 5.22. |

## General process for the acylation of xylylenediamines

The corresponding xylylenediamine (0.5 eq.) and N-(3-dimethylaminopropyl)-N-ethylcarbodiimide hydrochloride (EDC.HCl) (1 eq.) were added to a 0.4 M solution of the corresponding carboxylic acid in dichloromethane under argon atmosphere. The mixture was stirred for 48 h at room temperature, 1 M HCl was added, the organic phase was extracted with ethyl ether and washed with a saturated NaCl solution and dried ($MgSO_4$) and the solvent is eliminated at reduced pressure.

## Example 1

### Preparation of 2-butylcarbamoylmethoxy-N-[3-[(2-butylcarbamoyl-methoxy-acetylamino)methyl]benzyl] acetamide (compound 1)

It was synthesized from acid 20a (1 g, 5.2 mmol) and m-xylylenediamine (360 mg, 2.6 mmol), according to the general process described above. The crude reaction product was purified by recrystallization in dichloromethane/ hexane to give compound 1 as a white solid (500 mg, 36%).
m.p. 130˚C
$^1H$ NMR ($CDCl_3$, 300 MHz) δ: 7.55 (m, 2H, NHCO), 7.27-7.07 (m, 4H, ArH), 7.02 (m, 2H, NHCO), 4.38 (d, $^3J$= 5.8 Hz, 4H, $CH_2NH$), 3.97 (s, 4H, $OCH_2$), 3.90 (s, 4H, $OCH_2$), 3.17 (c, $^3J$= 7.3 Hz, 4H, $CH_2N$), 1 . 43 (q, $^3J$= 7.3 Hz, 4H, $CH_2$) , 1.29 (q, $^3J$= 7.3 Hz, 4H, $CH_2$), 0. 88 (t, $^3J$= 7.3 Hz, 6H, $CH_3$).
$^{13}C$ NMR ($CDCl_3$, 75 MHz) δ: 168.8; 168.5 (CO), 138.6 (ArC), 129.1; 126.7; 126.5 (ArCH), 70.9 ($OCH_2$), 42.6; 38.8 ($CH_2N$), 31.5; 20. ($CH_2$), 13.7 ($CH_3$).
MS (MALDI-TOF) m/z 479 [$(M+H)^+$, 100%]

| Elemental analysis | | | |
|---|---|---|---|
| Calculated for $C_{24}H_{38}N_4O_6$: | C 60.23; | H 8.00; | N 11.71. |
| Found | C 59.41; | H 8.06; | N 11.58. |

## Example 2

### Preparation of 2-[(butyl-methylcarbamoyl)methoxy]-*N*-[3-[(2-butyl-methylcarbamoyl)methoxyacetylamino] methyl]benzyl]-acetamide (compound 2)

It was synthesized from acid 20b (925 mg, 4.55 mmol) and m-xylylenediamine (0.25 mL, 1.89 mmol), according to the general process described above. The crude reaction product was purified by silica gel column chromatography (dichloromethane/isopropanol 95:5) to give compound 2 as an oil (479 mg, 50%).
$^1H$ NMR ($CDCl_3$, 300 MHz) δ: 8.0 (m, 2H, NHCO), 7.16-7.06 (m, 4H, ArH), 4.33 (d, $^3J$= 5.9 Hz, 4H, $CH_2NH$), 4.13 (s, 4H, $OCH_2$), 3.99 (s, 4H, $OCH_2$), 3.21 (t, $^3J$= 7.3 Hz, 2H, $CH_2N$), 3.0 (t, $^3J$= 7.6 Hz, 2H, $CH_2N$), 2.77 (s, 3H, $CH_3$) , 2.74 (s, 3H, $CH_3$) , 1.44-1.30 (m, 4H, $CH_2$), 1.23-1.12 (m, 4H, $CH_2$), 0.80 (t, $^3J$= 6.7 Hz, 3H, $CH_3$), 0.78 (t, ,$^3J$= 6.7 Hz, 3H, $CH_3$).
$^{13}C$ NMR ($CDCl_3$, 75 MHz) δ: 169.1; 168.0 (CO), 138.2 (ArC), 128.3; 126.6; 126.5 (ArCH), 71.3; 71.2; 69.3; 69.0 ($OCH_2$) , 48.0; 47.3; 42.2 ($CH_2N$) , 33.3; 32.9 ($CH_3N$), 29.8; 28.7; 19.5 ($CH_2$), 13.4 ($CH_3$).
MS (MALDI-TOF) m/z 507.3 [$(M+H)^+$, 100%], 529.3 [$(M+Na)^+$, 42%]

## Example 3

**Preparation of 2-dibutylcarbamoylmethoxy-*N*-[3-[(2-dibutyl-carbamoylmethoxyacetylamino)methyl]benzyl] acetamide (compound 3)**

It was synthesized from acid 20d (744 mg, 3.03 mmol) and m-xylylenediamine (0.2 mL, 1.51 mmol), according to the general process described above. The crude reaction product was purified by silica gel column chromatography (dichloromethane/isopropanol 95:5) to give compound 3 as a solid (485 mg, 54%).
m.p. 83˚C
$^1H$ NMR ($CDCl_3$, 300 MHz) δ: 7.99 (m, 2H, NHCO), 7.26-7.21 (m, 4H, ArH), 4.47 (d, $^3J$= 5.9 Hz, 4H, $CH_2NH$), 4.23 (s, 4H, $OCH_2$), 4.13 (s, 4H, $OCH_2$), 3.28 (t, $^3J$= 7.3 Hz, 4H, $CH_2N$), 3.08 (t, $^3J$= 7.3 Hz, 4H, $CH_2N$), 1.54-1.40 (m, 8H, $CH_2$), 1.34-1.21 (m, 8H, $CH_2$), 0.96-0.87 (m, 12H, $CH_3$).
$^{13}C$ NMR ($CDCl_3$, 75 MHz) δ: 169.4; 168.0 (CO), 138.6 (ArC), 128.8; 127.1; 126.6 (ArCH), 71.8; 69.5 ($OCH_2$), 46.5; 45.8; 42. 6 ($CH_2N$), 30.9; 29. 6; 20.2; 20.0 ($CH_2$), 13.8; 13.7 ($CH_3$).
MS (MALDI-TOF) m/z 591.4 [$(M+H)^+$, 100%]

## Example 4

**Preparation of 2-dioctylcarbamoylmethoxy-*N*-[3-[(2-dioctyl-carbamoylmethoxy-acetylamino)methyl]benzyl] acetamide (compound 4)**

It was synthesized from acid 20e (20 g, 55.9 mmol) and m-xylylenediamine (3.7 mL, 27.9 mmol) according to the general process described above. The crude reaction product was purified by silica gel column chromatography (dichloromethane/methanol 98:2) to give compound 4 as an oil (37.3 g, 82%).
$^1H$ NMR ($CDCl_3$, 500 MHz) δ: 7.96 (m, 2H, NHCO), 7.27-7.19 (m, 4H, ArH), 4.78 (s, 4H, $CH_2NH$), 4.21 (s, 4H, $OCH_2$), 4.11 (s, 4H, $OCH_2$), 3.28 (t, $^3J$= 7.0 Hz, 4H, $CH_2N$), 3.07 (t, $^3J$= 7.0 Hz, 4H, $CH_2N$), 1.50 (m, 8H, $CH_2$), 1.27 (m, 42H, $CH_2$), 0.88 (m, 12H, $CH_3$).
$^{13}C$ NMR ($CDCl_3$, 125 MHz) δ: 169.5; 168.0 (CO), 138.6 (ArC), 128.8; 127.2; 126.6 (ArCH), 71.8; 69.5 ($OCH_2$), 53.4; 46.8; 46.2; 42.7 ($CH_2N$), 31.8; 31.7; 29.4; 29.3; 29.27; 29.24; 28.9; 27.6; 27.0; 26.9; 22.65; 22.62 ($CH_2$), 14.1; 14.0 ($CH_3$).
MS (APCI+) m/z 815.7 ($[M+H]^+$, 100%).

| Elemental analysis | | | |
|---|---|---|---|
| Calculated for $C_{48}H_{86}N_4O_6 \cdot ½$ MeOH: | C 70.08; | H 10.67; | N 6.74. |
| Found | C 70.49; | H 10.71; | N 6.79. |

## Example 5

**Preparation of 2-[(methyloctylcarbamoyl)methoxy]-N-[3-[(2-[(methyloctylcarbamoyl)methoxy]acetylamino] methyl]benzyl]-**acetamide (compound 5)

It was synthesized from acid 20c (1.67 g, 6.46 mmol) and m-xylylenediamine (0.38 mL, 2.94 mmol) according to the general process described above. The crude reaction product was purified by silica gel column chromatography (dichloromethane/methanol 50:1) to give compound 5 as an oil (515 mg, 29%).
$^1H$ NMR ($CDCl_3$, 500 MHz) δ: 7.95 (m, 2H, NH), 7.29-7.25 (m, 4H, ArH), 4.51 (m, 4H, $CH_2N$), 4.28 (s, 4H, $OCH_2$), 4.16 (s, 4H, $OCH_2$) , 3.37 (m, 2H, $CH_2N$) , 3.15 (m, 2H, $CH_2N$), 2.94 (s, 3H, $CH_3N$) , 2.91 (s, 3H, $CH_3N$) , 1.52 (m, 4H, $CH_2$), 1.31 (m, 20H, $CH_2$) , 0.92 (m, 6H, $CH_3$).
$^{13}C$ NMR ($CDCl_3$, 125 MHz) δ: 169.4; 168.4; 168.2 (CO), 138.6 (ArC), 128.8; 127.2; 127.1; 127.0; 126.7 (ArCH), 71.9; 71.8; 69.8; 69.5 ($OCH_2$), 48.8; 48.2; 42.72; 42.69 ($CH_2N$), 33.8; 33.4 ($CH_3N$), 31.8; 31.7; 29.35; 29.28; 29.21; 29.1; 28.3; 27.1; 26.8; 26.7; 22.6 ($CH_2$), 14.09; 14.07 ($CH_3$).
MS (ESI+) m/z 619.4 ($[M+H]^+$, 100%)

| Elemental analysis | | | |
|---|---|---|---|
| Calculated for $C_{34}H_{58}N_4O_6$: | C 65.99; | H 9.45; | N 9.05 |
| Found | C 65.98; | H 9.46; | N 8.95 |

**Example 6**

**Preparation of 2-[[bis(3-methylbutyl)carbamoyl]methoxy]-N-[3-[(2-[[bis-(3-methylbutyl)carbamoyl]methoxy] acetylamino)-methyl]benzyl]acetamide (compound 6)**

It was synthesized from acid 20f (1.76 g, 6.46 mmol) and m-xylylenediamine (0.38 mL, 2.94 mmol) according to the general process described above. The crude reaction product was purified by silica gel column chromatography (dichloromethane/methanol 60:1) to give compound 6 as a white solid (1.14 g, 60%).
m.p. 104˚C
$^1$H NMR ($CDCl_3$, 500 MHz) δ: 7.90 (t, $^3$J= 5.9 Hz, 2H, NHCO), 7.32-7.24 (m, 4H, ArH), 4.52 (d, $^3$J= 5.9 Hz, 4H, $CH_2N$), 4.27 (s, 4H, $OCH_2$) , 4.18 (s, 4H, $OCH_2$) , 3.34 (t, $^3$J= 7.9 Hz, 4H, $CH_2N$), 3.14 (t, $^3$J= 7. 9 Hz, 4H, $CH_2N$), 1.59 (m, 4H, CH), 1.44 (m, 8H, $CH_2$), 0.96 (m, 24H, $CH_3$).
$^{13}$C NMR ($CDCl_3$, 125 MHz) δ: 169.5; 167.9 (CO), 138.6 (ArC), 128.9; 127.2; 126.7 (ArCH), 71.8; 69.6 ($OCH_2$), 45.1; 44.6; 42.7 ($CH_2N$), 37.8; 36.4 ($CH_2$) , 26.18; 26.16 (CH), 22.54; 22. 46 ($CH_3$).
MS (ESI+) m/z 669.5 ($[M+Na]^+$, 100%).

| Elemental analysis | | | |
|---|---|---|---|
| Calculated for $C_{36}H_{62}N_9O_6$: | C 66.84; | H 9.66; | N 8.66 |
| Found | C 66.82; | H 9.71; | N 8.56 |

**Example 7**

**Preparation of N-methyl-N-[3-[(methyl-[2-[(methyl-octyl carbamoyl)methoxy]acetyl]amino)methyl]benzyl]-2-[(methyl-octyl carbamoyl)methoxy]acetamide (compound 7)**

It was synthesized according to the general process described above, from acid 20c (2.28 g, 8.8 mmol) and N-methyl-(3-methylaminomethylbenzyl)amine hydrochloride obtained by the method described in Sander, M.; Burmeister, D., *Berichte* 1962, 95, 964 (520 mL, 2.2 mmol), using triethylamine (0.71 mL, 5 mmol) to release the diamine. The crude reaction product was purified by silica gel column chromatography (dichloromethane/methanol 96:4) to give compound 7 as an oil (740 mg, 53%).
$^1{}_H$ NMR ($CDCl_3$, 500 MHz) δ: 7.32 (m, 1H, ArH), 7.21-7.10 (m, 3H, ArH), 4.60 (s, 4H, $CH_2N$), 4.42-4.31 (s, 8H, $OCH_2$), 3.40 (m, 2H, $CH_2N$), 3.27 (m, 2H, $CH_2N$), 3.00-2.90 (s, 12H, $CH_3N$) , 1.55 (m, 4H, $CH_2$) , 1.30 (s, 20H, $CH_2$) , 0. 90 (m, 6H, $CH_3$) .
$^{13}$C NMR ($CDCl_3$, 125 MHz) δ: 169.0; 168.7; 168.5; 168.3 (CO), 137.7; 137.3; 136.8 (ArC), 129.3; 129.0; 127.9; 127.3; 127.2; 126.6; 125.6 (ArCH), 69.7; 69.6; 69.5; 69.4; 69.1 ($OCH_2$) 52.4; 50.8; 49.0; 48.8; 47.9 ($CH_2N$), 34.2; 33.8; 33.6; 33.1; ($CH_3N$), 31.8; 31.7; 29.4; 29.3; 29.22; 29.19; 28.4; 27.1; 26. 8; 26.7; 22. 6 ($CH_2$), 14.0 ($CH_3$) .
MS (MALDI-TOF) m/z 669.5 ($[M+Na]^+$, 100%).

| Elemental analysis | | | |
|---|---|---|---|
| Calculated for $C_{36}H_{62}N_4O_6$: | C 66.84; | H 9.66; | N 8.66 |
| Found | C 66.82; | H 9.51; | N 8.07 |

**Example 8**

**Preparation of 2-[[4-([2-[dioctylcarbamoyl)methoxy]-acetylamino]-methyl)-benzylcarbamoyl]methoxy]-N,N-dioctyl-**acetamide (Compound 8)

It was synthesized according to the general process described above from acid 20e (2 g, 5.87 mmol) and p-xylylenediamine (400 mg, 2.93 mmol). The crude reaction product was purified by silica gel column chromatography (dichloromethane/methanol 99:1) to give compound 8 as an oil (502 mg, 22%).
$^1$H NMR ($CDCl_3$, 300 MHz) δ: 7.91 (m, 2H, NHCO), 7.21 (s, 4H, ArH), 4.39 (apparent d, 4H, $CH_2NH$), 4.15 (s, 4H, $OCH_2$), 4.06 (s, 4H, $OCH_2$), 3.21 (t, $^3$J= 7 Hz, 4H, $CH_2N$), 3.07 (t, $^3$J= 7 Hz, 4H, $CH_2N$), 1.44 (m, 8H, $CH_2$) , 1.21 (m, 42H, $CH_2$), 0.80 (m, 12H, $CH_3$).
$^{13}$C NMR ($CDCl_3$, 75 MHz) δ: 169.4; 168.0 (CO), 137.3 (ArC), 127.9 (ArCH), 71.8; 69.5 ($OCH_2$), 46.8; 46.1; 42.5 ($CH_2N$), 31.76; 31.69; 29.3; 29.21; 29.18; 29.13; 28.9; 27.5; 26.9; 26.8; 22.60; 22.57 ($CH_2$), 14.0 ($CH_3$).
MS (APCI+) m/z 815.7 ($[M+H]^+$, 100%).

| | | | |
|---|---|---|---|
| Elemental analysis | | | |
| Calculated for $C_{48}H_{86}N_4O_6$: | C 70.72; | H 10.63; | N 6.87 |
| Found | C 70.81; | H 10.96; | N 6.74 |

**Example 9**

**Preparation of 2-di-butylcarbamoylmethoxy-N-[3-[(2-dibutylcarbamoyl-methoxyacetylamino)methyl]-5-hexyloxybenzyl]-acetamide (compound 9).**

**a) Preparation of 1-benzyloxy-3,5-bis(chloromethyl)benzene**

A solution of 1-benzyloxy-3,5-bis(hydroxymethyl)benzene (2 g, 8.18 mmol) obtained according to the method described by Le Stang et al. (Le Stang, S.; Meier, R.; Rocaboy, C.; Gladysz, J. A., J. Fluorine Chem. 2003, 119, 141) in thionyl chloride (30 mL) was stirred at room temperature and under argon atmosphere for 18 hours. The remaining thionyl chloride was eliminated under reduced pressure and the obtained product was vacuum-dried for 1 day. Then, it was ground in ethanol/methanol/dichloromethane to give 1-benzyloxy-3,5-bis(chloromethyl)benzene quantitatively as a white solid.
m.p. 62-63°C
$^1$H NMR ($CDCl_3$, 300 MHz) δ: 7.46-7.34 (m, 5H, ArH), 7.01 (s, 1H, ArH), 6.98 (s, 2H, ArH), 5.08 (s, 2H, $OCH_2$), 4.54 (s, 4H, $CH_2Cl$) -
$^{13}$C NMR ($CDCl_3$, 75 MHz) δ: 159.2; 139.4; 136.4 (ArC), 128.6; 128.1; 127.5; 121.0; 114.9 (ArCH), 70.2 ($OCH_2$), 45.7 ($CH_2Cl$).
MS (APCI$^+$) m/z 282.2 ( $[M+H]^+$, 100%).

**b) Preparation of 3-aminomethyl-5-benzyloxy-benzylamine**

NaI (30 mg, 0.18 mmol) and potassium phthalimide (741 mg, 4 mmol) were added to a solution of 1-benzyloxy-3,5-bis(chloromethyl)benzene obtained in step a) (500 mg, 1.77 mmol) in N,N-dimethylformamide (16 mL). The mixture was heated under argon atmosphere at 90°C for 6 days. 1 M HC1 was added until precipitation, it was filtered and washed with $H_2O$ a beige solid being obtained which was used without additional purification (803 mg, 89%).
m.p. 170-180 °C
$^1$H NMR ($CDCl_3$, 300 MHz) δ: 7.84 (m, 2H, ArH), 7.71 (m, 2H, ArH), 7.49-7.23 (m, 5H, ArH), 7.10 (s, 1H, ArH), 6.90 (s, 2H, ArH), 4.98 (s, 2H, $OCH_2$), 4.79 (s, 4H, $NCH_2$).
$^{13}$C NMR ($CDCl_3$, 75 MHz) δ: 167.9 (CO), 159.3; 138.2; 136.6; 132.1; 127.9; 121.0 (ArC), 133.9; 128.5; 127.6; 123.4; 114.1 (ArCH), 70.1 ($OCH_2$), 41.3 ($CH_2N$).
MS (MALDI-TOF) m/z 525.1 ($[M+Na]^+$, 100%).

| | | | |
|---|---|---|---|
| Elemental analysis | | | |
| Calculated for $C_{31}H_{22}N_2O_5.2H_2O$: | C 69.14; | H 4.87; | N 5.20 |
| Found | C 70.09; | H 4.83; | N 5.11 |

Hydrazine monohydrate (4.6 mL, 59.7 mmol) was added to a previously sonicated suspension of the previous crude reaction product (300 mg, 0.57 mmol) in ethanol (15 mL), and was heated at 80°C for 18 h. Afterwards, the reaction mixture was cooled, diluted with water and extracted with dichloromethane. The organic phase was dried ($Na_2SO_4$) and concentrated to dryness to quantitatively obtain 3-aminomethyl-5-benzyloxy-benzylamine as a white solid.
m.p. 98-100°C
$^1$H NMR ($CDCl_3$, 300 MHz) δ: 7.43-7.28 (m, 5H, ArH), 6.86 (s, 1H, ArH), 6.82 (s, 2H, ArH) , 5.06 (s, 2H, $OCH_2$), 3.81 (s, 4H, $CH_2NH_2$) .
$^{13}$C NMR ($CDCl_3$, 75 MHz) δ: 159.2; 145.1; 136.9 (ArC), 128.5; 127.9; 127.4; 118.3; 111.8 (ArCH), 69.9 ($OCH_2$), 46.4 ($CH_2NH_2$).
MS (FAB$^+$) m/z, 243 ($[M+H]^+$, 100%), 485.3($[2M+H]^+$, 10%).

**c) Preparation of 2-dibutylcarbamoylmethoxy-N-[3-[(2-dibutylcarbamoylmethoxy-acetylamino)methyl]-5-hydroxybenzyl]-acetamide.**

3-aminomethyl-5-benzyloxy-benzylamine obtained in step b) (161 mg, 0.66 mmol) and N-(3-dimethyl-aminopropyl)-N-ethylcarbodiimide hydrochloride (EDC.HCl) (266.5 mg, 1.39 mmol) were added, under argon atmosphere, to a solution of acid 20d (325.7 mg, 1.33 mmol) in dichloromethane (7 mL). The mixture was stirred for 48 h at room temperature, 1 M HCl was added, and the organic phase was successively washed with $H_2O$, a saturated $NaHCO_3$ solution, $H_2O$ and a saturated NaCl solution and it was dried ($MgSO_4$). The solvent was eliminated under reduced pressure and the crude reaction product was purified by silica gel column chromatography (dichloromethane/methanol 25:1) to give an oil (286.1 mg, 62%).
$^1H$ NMR ($CDCl_3$, 500 MHz) δ: 7.97 (s, 2H, NHCO), 7.40-7.28 (m, 5H, ArH), 6.82 (s, 3H, ArH), 5.02 (s, 2H, $ArOCH_2$) , 4.40 (d, $^3j$ = 6. 0 Hz, 4H, $CH_2NH$) , 4.20 (s, 4H, $OCH_2$), 4.09 (s, 4H, $OCH_2$), 3.27 (t, $^3J$= 8 Hz, 4H, $CH_2N$), 3.06 (t, $^3J$= 8 Hz, 4H, $CH_2N$), 1.50 (m, 8H, $CH_2$) , 1.26 (m, 8H, $CH_2$), 0.89 (m, 12H, $CH_3$).
$^{13}C$ NMR ($CDCl_3$, 125 MHz) δ: 169.5; 168.1 (CO), 159.4; 140.1; 136.9 (ArC), 128.5; 127.9; 127.7; 119.6; 112.9 (ArCH), 71.8; 70.0; 69.5 ($OCH_2$), 53.4; 46.5; 45.8; 42.7 ($CH_2N$), 30.9; 29.6; 20.2; 20.0 ($CH_2$), 13.8; 13.7 ($CH_3$).
MS ($FAB^+$) m/z 697.3 ($[M+H]^+$, 100%).

| Elemental analysis | | | |
|---|---|---|---|
| Calculated for $C_{39}H_{60}N_4O_7 \cdot MeOH$: | C 65.91; | H 8.85; | N 7.69 |
| Found | C 65.94; | H 8.76; | N 7.85 |

10% Pd(C) (catalytic) was added to a solution of previously obtained oil (136.2 mg, 0.19 mmol) in ethanol (15 mL) and was stirred under $H_2$ atmosphere for 1 h. It was filtered on Celite and concentrated to dryness, obtaining 2-dibutylcarbamoylmethoxy-N-[3-[(2-dibutylcarbamoylmethoxy-acetylamino)methyl]-5-hydroxybenzyl]acetamide as a transparent oil (111.8 mg, 94%).
$^1H$ NMR ($CDCl_3$, 500 MHz) δ: 7.97 (s, 2H, NHCO), 6.73 (s, 1H, ArH), 6.63 (s, 2H, ArH), 4.36 (d, $^3J$ = 6.1 Hz, 4H, $CH_2NH$) , 4.26 (s, 4H, $OCH_2$), 4.12 (s, 4H, $OCH_2$) , 3.33 (t, $^3J$= 7.6 Hz, 4H, $CH_2N$), 3.12 (t, $^3J$= 7.6 Hz, 4H, $CH_2N$), 1.50 (m, 8H, $CH_2$), 1.32 (m, 8H, $CH_2$), 0.95 (m, 12H, $CH_3$) .
$^{13}C$ NMR ($CDCl_3$, 125 MHz) δ: 169.4; 168.2 (CO), 157.3; 139.8 (ArC), 118.3; 113.9 (ArCH), 71.3; 69.1 ($OCH_2$), 46.6; 45.9; 42.7 ($CH_2N$) , 30.9; 29.6; 20.2; 20.0 ($CH_2$), 13.8; 13.7 ($CH_3$).
MS ($APCI^+$) m/z 607.5 ($[M+H]^+$, 100%).

| Elemental analysis | | | |
|---|---|---|---|
| Calculated for $C_{32}H_{54}N_4O_7$.EtOH: | C 62.55; | H 9.26; | N 8.58 |
| Found | C 62.67; | H 9.36; | N 8.72 |

**General process of alkylation of 2-di-butyl-carbamoylmethoxy-N-[3-[(2-dibutylcarbamoylmethoxy-acetylamino)-methyl]-5-hydroxybenzyl]acetamide obtained in step c)**

$K_2CO_3$ (1.2 eq.) is added to a 0.06 M of 2-dibutyl-carbamoylmethoxy-N-[3-[(2-dibutylcarbamoylmethoxy-acetylamino)-methyl]-5-hydroxybenzyl]acetamide obtained in step c) in N,N-dimethylformamide under argon atmosphere and is heated at 60˚C for 1 h. Afterwards, the corresponding alkyl bromide (1.2 eq.) is added and is heated at 80˚C for 24 h. The mixture is concentrated to dryness, dissolved in dichloromethane and the organic phase is successively washed with 1 M HCl, $H_2O$ and saturated NaCl solution, it is dried ($MgSO_4$) and the solvent is eliminated under reduced pressure.

**d) Preparation of 2-di-butylcarbamoylmethoxy-N-[3-[(2-dibutylcarbamoylmethoxyacetylamino)methyl]-5-hexyloxy-benzyl]-acetamide (compound 9)**

It was synthesized according to the general process described above, from 2-dibutylcarbamoylmethoxy-N-[3-[(2-dibutylcarbamoylmethoxy-acetylamino)methyl]-5-hydroxybenzyl]acetamide obtained in step c) (120 mg, 0.198 mmol) and from 1-bromohexane (33 μL, 0.234 mmol). The crude reaction product was purified by silica gel column chromatography (dichloromethane/methanol 40:1) to give compound 9 as an oil (90 mg, 63%).
$^1H$ NMR ($CDCl_3$, 500 MHz) δ: 7.85 (t, $^3J$= 5.8 Hz, 2H, NHCO), 6.79 (s, 1H, ArH), 6.72 (s, 2H, ArH), 4.42 (d, $^3J$ = 5.8Hz, 4H, $ArCH_2N$) , 4.23 (s, 4H, $OCH_2$), 4.12 (s, 4H, $OCH_2$), 3.92 (t, $^3j$ = 6.6 Hz, 2H, $ArOCH_2$) , 3.29 (m, 4H, $CH_2N$), 3.08 (m, 4H, $CH_2N$), 1.76-1.24 (m, 24H, $CH_2$), 0.95-0.87 (m, 15H, $CH_3$).

$^{13}C$ NMR ($CDCl_3$, 125 MHz) δ: 169.4; 168.1 (CO), 159.7; 139.9 (ArC), 119.2; 112.8 (ArCH), 71.7; 69.5; 68.0 ($OCH_2$), 46.5; 45.8; 42.7 ($CH_2N$), 31.6; 31.0; 29.7; 29.3; 25.8; 22.6; 20.2; 20.1($CH_2$), 14.1; 13.85; 13.78 ($CH_3$).
MS ($ESI^+$) m/z 713.5 ($[M+Na]^+$, 100%).

| Elemental analysis | | | |
|---|---|---|---|
| Calculated for $C_{38}H_{66}N_4O_7 \cdot 1/2MeOH$: | C 65.41; | H 9.69; | N 7.92. |
| Found | C 65.43; | H 9.75; | N 7.79. |

**Example 10**

**Preparation of 2-di-butylcarbamoylmethoxy-N-[3-[(2-dibutyl-carbamoylmethoxyacetylamino)methyl]-5-(1-methyl-hexyloxy)-benzyl]acetamide (compound 10)**

It was synthesized according to the general process described above, from 2-dibutylcarbamoylmethoxy-N-[3-[(2-dibutylcarbamoylmethoxy-acetylamino)methyl]-5-hydroxybenzyl]-acetamide obtained in step c) of the process for the preparation of compound 9 (120 mg, 0.198 mmol) and from 2-bromoheptane (37 μL, 0.234 mmol). The crude reaction product was purified by silica gel column chromatography (dichloromethane/methanol 60:1) to give compound 10 as an oil (64 mg, 44%).
$^1H$ NMR ($CDCl_3$, 500 MHz) δ: 7.88 (t, $^3J$ = 5.7 Hz, 2H, NHCO), 6.80 (s, 1H, ArH), 6.74 (s, 2H, ArH), 4.42 (d, $^3J$ = 5.7 Hz, 4H, $ArCH_2N$), 4.34 (m, 1H, OCH), 4.23 (s, 4H, $OCH_2$) 4.12 (s, 4H, $OCH_2$) , 3.29 (t, $^3J$ = 7.7 Hz, 4H, $CH_2N$), 3.09 (t, $^3J$ = 7.7 Hz, 4H, $CH_2N$), 1.61-1.39 (m, 9H, $CH_2$ and CH), 1.33-1.25 (m, 16H, $CH_2$), 0.95-0.87 (m, 18H, $CH_3$) .
$^{13}C$ NMR ($CDCl_3$, 125 MHz) δ: 169.4; 168.0 (CO), 158.7; 139.9 (ArC), 119.1; 114.0 (ArCH), 73.7 (OCH), 71.7; 69.5 ($OCH_2$), 46.5; 45.8; 42.8 ($CH_2N$), 36.5; 31.9; 31.0; 29.7; 25.2; 22.6; 20.2; 20. 1 ($CH_2$), 19.7 ($CH_3CH$), 14.1; 13.86; 13.78 ($CH_3$).
MS ($ESI^+$) m/z 727.5 ($[M+Na]^+$, 100%).

| Elemental analysis | | | |
|---|---|---|---|
| Calculated for $C_{39}H_{68}N_4O_7$: | C 66.44; | H 9.72; | N 7.95. |
| Found | C 66.19; | H 9.69; | N 7.38 |

**Example 11**

**Preparation of 2-di-butylcarbamoylmethoxy-N-[3-[(2-dibutyl-carbamoylmethoxyacetylamino)methyl]-5-dodecyloxy-benzyl]-**acetamide (compound 11)

It was synthesized according to the general process described above, from 2-dibutylcarbamoylmethoxy-N-[3-[(2-dibutylcarbamoylmethoxy-acetylamino)methyl]-5-hydroxybenzyl]acetamide obtained in step c) of the process for the preparation of compound 9 (120 mg, 0.198 mmol) and from 1-bromododecane (56 μL, 0.234 mmol). The crude reaction product was purified by silica gel column chromatography (dichloromethane/methanol 40:1) to give compound 11 as an oil (121 mg, 76%).
$^1H$ NMR ($CDCl_3$, 500 MHz) δ: 7.88 (t, $^3J$ = 5.7 Hz, 2H, NHCO), 6.80 (s, 1H, ArH), 6.74 (s, 2H, ArH), 4.42 (d, $^3J$ = 5.7 Hz, 4H, $ArCH_2N$), 4.23 (s, 4H, $OCH_2$) , 4.12 (s, 4H, $OCH_2$), 3.91 (t, $^3J$ = 6.5 Hz, 2H, $ArOCH_2$), 3.29 (t, $^3J$ = 7.6 Hz, 4H, $CH_2N$), 3.09 (t, $^3J$ = 7.6 Hz, 4H, $CH_2N$), 1.53-1.44 (m, 10H, $CH_2$), 1.42-1.26 (m, 26H, $CH_2$) , 0. 94-0. 86 (m, 15H, $CH_3$) .
$^{13}C$ NMR ($CDCl_3$, 125 MHz) δ: 169.4; 168.1 (CO), 159.7; 139.9 (ArC), 119.2; 112.8 (ArCH), 71.8; 69.6; 68.0 ($OCH_2$), 46.5; 45.8; 42.7 ($CH_2N$), 31.9; 31.0; 29.70; 29.69; 29.65; 29.63; 29.49; 29.37; 26.1; 22.7; 20.2; 20.1 ($CH_2$), 14.1; 13.9; 13.8 ($CH_3$) .
MS ($ESI^+$) m/z 797.7 ($[M+Na]^+$, 100%).

| Elemental analysis | | | |
|---|---|---|---|
| Calculated for $C_{44}H_{78}N_4O_7 \cdot 1/2MeOH$: | C 67.56; | H 10.19; | N 7.08 |
| Found | C 67.65; | H 10.49; | N 6.94 |

**General process of acylation of alkylidenediamines and bis(aminoalkyl)ethers.**

The corresponding alkylidenediamine (0.5 eq.) or the corresponding bis(aminoalkyl)ether (0.5 eq.) and N-(3-dimethylaminopropyl)-N-ethylcarbodiimide hydrochloride (EDC.HCl) (1 eq.) are added to a 0.4 M solution of the corresponding acid in dichloromethane under argon atmosphere. The mixture is stirred for 48 h at room temperature, 1 M HCl is added, the organic phase is extracted with diethyl ether, it is washed with a saturated NaCl solution, dried ($MgSO_4$) and the solvent is eliminated under reduced pressure.

## Example 12

### Preparation of 2-dibutylcarbamoylmethoxy-*N*-[5-(2-dibutyl-carbamoylmethoxyacetylamino)pentyl]acetamide (compound 12)

It was synthesized from acid 20d (398 mg, 1.62 mmol) and 1,5-pentanediamine (142 mg, 0.81 mmol), according to the general process described above. The crude reaction product was purified by silica gel column chromatography (dichloromethane/methanol 94:6) to give compound 12 as an oil (185 mg, 41%).
$^1$H NMR ($CDCl_3$, 300 MHz) δ: 7.69 (m, 2H, NHCO), 4.18 (s, 4H, $OCH_2$), 4.01 (s, 4H, $OCH_2$), 3.23 (m, 8H, $CH_2N$), 3.05 (t, $^3$J= 7.5 Hz, 4H, $CH_2N$), 1 . 54-1 .20 (m, 24H, $CH_2$), 0.9-0.8 (m, 12H, $CH_3$) .
$^{13}$C NMR ($CDCl_3$, 75 MHz) δ: 168.7; 166.6 (CO), 68.0; 66.9 ($OCH_2$) , 46.9; 40.4; 38.7 ($CH_2N$), 30.1; 29.8; 29.6; 23.9; 20.2 ($CH_2$), 13.8 ($CH_3$).
MS ($FAB^+$) m/z 557.5 ($[M+H]^+$, 100%).

| Elemental analysis | | | |
|---|---|---|---|
| Calculated for $C_{29}H_{56}N_4O_6$-MeOH: | C 61.19; | H 10.27; | N 9.52 |
| Found | C 61.36; | H 10.19; | N 9.65 |

## Example 13

### Preparation of 2-dioctylcarbamoylmethoxy-*N*-[5-(2-dioctyl-carbamoylmethoxyacetylamino)pentyl]acetamide (compound 13)

It was synthesized from acid 20e (2 g, 5 mmol) and 1,5-pentanediamine (437 mg, 2.5 mmol), according to the general process described above. The crude reaction product was purified by silica gel column chromatography (dichloromethane/methanol 98:2) to give compound 13 as an oil (640 mg, 33%).
$^1$H NMR ($CDCl_3$, 500 MHz) δ: 7.71 (m, 2H, NHCO), 4.23 (s, 4H, $OCH_2$), 4.06 (s, 4H, $OCH_2$), 3.29 (m, 8H, $CH_2N$), 3.09 (t, $^3$j = 7.7 Hz, 4H, $CH_2N$) , 1.55 (m, 12H, $CH_2$), 1.29 (broad s, 40H, $CH_2$) , 0.9-0.8 (m, 12H, $CH_3$).
$^{13}$C NMR ($CDCl_3$, 125 MHz) δ: 169.4; 168.1 (CO), 71.9; 69.6 ($OCH_2$) , 46.8; 46.2; 38.7 ($CH_2N$) , 31.8; 31.7; 29.3; 29.24; 29.20; 29.1; 28.9; 27.6; 27.0; 26.8; 24.2; 22.61; 22.58 ($CH_2$), 14.1; 14.0 ($CH_3$).
MS ($ESI^+$) m/z 803.7 ($[M+Na]^+$, 100%).

| Elemental analysis | | | |
|---|---|---|---|
| Calculated for $C_{45}H_{88}N_4O_6$: | C 69.19; | H 11.35; | N 7.17 |
| Found | C 68.96; | H 11.64; | N 7.33 |

## Example 14

### Preparation of 2-dibutylcarbamoylmethoxy-N-[6-(2-dibutyl-carbamoylmethoxyacetylamino)hexyl]acetamide (compound 14)

It was synthesized from acid 20d (3 g, 12.2 mmol) and 1,6-hexanediamine (674 mg, 5.8 mmol), according to the general process described above. The crude reaction product was purified by silica gel column chromatography (dichloromethane/methanol 99:1) to give compound 14 as an oil (2.4 g, 72%).
$^1$H NMR ($CDCl_3$, 300 MHz) δ: 7.66 (m, 2H, NHCO), 4.20 (s, 4H, $OCH_2$), 4.02 (s, 4H, $OCH_2$), 3.25 (m, 8H, $CH_2N$), 3.06 (t, $^3$J = 7.3 Hz, 4H, $CH_2N$), 1.68-1.42 (m, 12H, $CH_2$), 1.33-1.20 (m, 12H, $CH_2$), 0. 91 (t, $^3$J = 7.3 Hz, 6H, $CH_3$) , 0. 88 (t, $^3$j = 7.3 Hz, 6H, $CH_3$) .
$^{13}$C NMR ($CDCl_3$, 125 MHz) δ: 169.3; 168.0 (CO), 71.8; 69.5 ($OCH_2$), 46.4; 45.7; 38.7 ($CH_2N$), 30.9; 29.5; 29.2; 26.4; 20.1; 19. 9 ($CH_2$) , 13.7; 13. 6 ($CH_3$).
MS (Maldi-TOF) m/z 571.4 ($[M+H]^+$, 100%), 593.4 ($[M+Na]^+$, 50%)

**Example 15**

**Preparation of 2-dioctylcarbamoylmethoxy-*N*-[6-(2-dioctyl-carbamoylmethoxyacetylamino)hexyl]acetamide (compound 15)**

It was synthesized from acid 20e (539 mg, 1.5 mmol) and 1,6-hexanediamine (87.7 mg, 0.75 mmol), according to the general process described above. The crude reaction product was purified by silica gel column chromatography (dichloromethane/isopropanol 95:5) to give compound 15 as an oil (249 mg, 42%).
$^1H$ NMR ($CDCl_3$, 500 MHz) δ: 7.71 (m, 2H, NHCO), 4.25 (s, 4H, $OCH_2$), 4.08 (s, 4H, $OCH_2$), 3.32 (m, 8H, $CH_2N$), 3. 11 (t, $^3J$ = 7.7 Hz, 4H, $CH_2N$) , 1.55 (broad s, 12H, $CH_2$), 1.31 (broad s, 44H, $CH_2$), 0.92-0.89 (m, 12H, $CH_3$).
$^{13}C$ NMR ($CDCl_3$, 125 MHz) δ: 169.3; 168.0 (CO), 71.8; 69.5 ($OCH_2$), 46.7; 46.1; 38.7 ($CH_2N$), 31.7; 31.6; 29.35; 29.28; 29.18; 29.14; 29.10; 28.8; 27.5; 26.9; 26.8; 26.5; 22.55; 22.52 ($CH_2$), 13.9 ($CH_3$).
MS (APCI$^+$) m/z 795.8 ($[M+H]^+$, 100%).

| Elemental analysis | | | |
|---|---|---|---|
| Calculated for $C_{46}H_{90}N_4O_6 \cdot$i-PrOH: | C 68.81; | H 11.55; | N 6.55 |
| Found | C 68.73; | H 11.30; | N 6.87 |

**Example 16**

**Preparation of 2-dioctylcarbamoylmethoxy-*N*-[7-(2-dioctyl-carbamoylmethoxyacetylamino)heptyl]acetamide (compound 16)**

It was synthesized from acid 20e (6 g, 15 mmol) and 1,7-heptanediamine (475 mg, 7.5 mmol), according to the general process described above. The crude reaction product was purified by silica gel column chromatography (dichloromethane/methanol 98:2) to give compound 16 as an oil (4.3 g, 71%).
$^1H$ NMR ($CDCl_3$, 500 MHz) δ: 7.67 (m, 2H, NHCO), 4.26 (s, 4H, $OCH_2$) , 4.09 (s, 4H, $OCH_2$), 3.31-3.13 (m, 12H, $CH_2N$), 1.56 (broad s, 12H, $CH_2$) , 1.31 (m, 46H, $CH_2$) ; 0.92 (m, 12H, $CH_3$).
$^{13}C$ NMR ($CDCl_3$, 125 MHz) δ: 169.3; 168.1 (CO), 71.9; 69.6 ($OCH_2$), 46.8; 46.2; 38. 9 ($CH_2N$), 31.8; 31.7; 29.5; 29.4; 29.3; 29.2; 29.18; 28.9; 27.6; 27.0; 26.9; 22.6 ($CH_2$), 14.1 ($CH_3$).
MS (ESI$^+$) m/z 831.7 ($[M+Na]^+$, 37%), 810.0 ($[M+H]^+$, 50%).

| Elemental analysis | | | |
|---|---|---|---|
| Calculated for $C_{47}H_{92}N_4O_6$: | C 69.76; | H 11.46; | N 6.92 |
| Found | C 69.06; | H 11.55; | N 6.99 |

**Example 17**

**Preparation of 2-dioctylcarbamoylmethoxy-N-[2-[2-(2-dioctyl-carbamoylmethoxyacetylamino)ethoxy]ethyl] acetamide (compound 17)**

It was synthesized from acid 20e (4.64 g, 13 mmol) and bis-(2-aminoethyl)ether (690.8 mg, 6.5 mmol), according to the general process described above. The crude reaction product was purified by silica gel column chromatography (dichloromethane/methanol 96:4) to give compound 17 as an oil (4.3 g, 85%).
$^1H$ NMR ($CDCl_3$, 500 MHz) δ: 7.87 (m, 2H, NHCO), 4.21 (s, 4H, $OCH_2$), 4.05 (s, 4H, $OCH_2$), 3.53 (m, 4H, $OCH_2$), 3.44 (m, 4H, $CH_2N$), 3.26 (m, 4H, $CH_2N$), 3.06 (m, 4H, $CH_2N$), 1.48 (broad s, 8H, $CH_2$), 1.23 (broad s, 40H, $CH_2$); 0.83 (m, 12H, $CH_3$).
$^{13}C$ NMR ($CDCl_3$, 125 MHz) δ: 169.7; 168.2 (CO), 71.5; 69.5; 69.4 ($OCH_2$), 47.8; 46.8; 46.1; 38.7 ($CH_2N$), 31.7; 31.68; 29.3; 29.2; 29.16; 29.11; 29.10; 29.0; 28.8; 27.5; 26.9; 26.8; 26.82; 26.1; 22.6; 22.5 ($CH_2$), 14.0; 13.9 ($CH_3$).
MS (ESI$^+$) m/z 805.5 ($[M+Na]^+$, 100%).

| Elemental analysis | | | |
|---|---|---|---|
| Calculated for $C_{44}H_{86}N_4O_7 \cdot$1/2MeOH: | C 66.75; | H 11.01; | N 7.08 |
| Found | C 66.87; | H 11.22; | N 7.18 |

**Example 18**

**Preparation of 2-dioctylcarbamoylmethoxy-N-[3-[3-(2-dioctyl-carbamoylmethoxyacetylamino)propoxy]propyl]acetamide (compound 18)**

It was synthesized from acid 20e (20 g, 55.9 mmol) and from bis-(3-aminopropil)ether (3.7 g, 27.9 mmol), according to the general process described above. The crude reaction product was purified by silica gel column chromatography (dichloromethane/methanol 96:4) to give compound 18 as an oil (39 g, 85%).
$^1$H NMR ($CDCl_3$, 500 MHz) δ: 7.79 (m, 2H, NHCO), 4.26 (s, 4H, $OCH_2$), 4.09 (s, 4H, $OCH_2$), 3.52 (t, $^3J$ = 6.1 Hz, 4H, $OCH_2$), 3.43 (m, 4H, $CH_2N$), 3.33 (m, 4H, $CH_2N$), 3.13 (m, 4H, $CH_2N$), 1.84 (q, $^3J$= 6.4 Hz, 4H, $CH_2$), 1.58-1.31 (m, 48H, $CH_2$); 0.91 (m, 12H, $CH_3$).
$^{13}$C NMR ($CDCl_3$, 125 MHz) δ: 169.5; 168.0 (CO), 71.7; 69.6; 68. 9 ($OCH_2$), 46.8; 46. 1 ($CH_2N$), 36. 6; 31.8; 31.7; 29.5; 29.4; 29. 3; 29.24; 29.20; 29.0; 27.6; 27.0; 26.9; 22.65; 22.62 ($CH_2$), 14.1 ($CH_3$).
MS (ESI$^+$) m/z 1643.5 ([2M+Na]$^+$, 100%), 833.8 ([M+Na]$^+$, 89%), 811.7 ([M+H]$^+$, 73%).

| Elemental analysis | | | |
|---|---|---|---|
| Calculated for $C_{46}H_{90}N_4O_7 \cdot 1/2MeOH$: | C 67.51; | H 11.21; | N 6.77 |
| Found | C 67.58; | H 11.51; | N 6.93 |

**Example 19**

**Preparation of 2,7-di-tert-butyl-9,9-dimethyl-9H-xanthen-4,5-dicarboxylic acid bis-[[3-(2-dibutylcarbamoylmethoxy-acetylamino) propyl] amide] (compound 19)**

**a) Preparation of 2,7-di-tert-butyl-N4,N5-bis(3-aminopropyl)-9,9-dimethyl-9H-xanthen-4,5-dicarboxamide**

Triethylamine (0.79 mL, 5.70 mmol) and PyBOP (1.5 g, 2.85 mmol) were added to a solution of 2,7-di-tert-butyl-9,9-dimethyl-9H-xanthen-4,5-dicarboxylic acid (300 mg, 1.42 mmol) in dichloromethane (15 mL). The mixture was stirred for 5 min at room temperature and tert-butyl 3-aminopropyl-carbamate (0.5 mL, 2.85 mmol) was added to it. After 48 h, 10% citric acid was added, it was extracted with dichloromethane and the organic phase was successively washed with saturated $NaHCO_3$, water and brine, and it was dried ($MgSO_4$). The solvent was eliminated under reduced pressure and the obtained residue was purified by silica gel column chromatography (dichloromethane/methanol, 99:1) to give 2,7-di-tert-butyl-N4,N5-bis(3-aminopropyl)-9,9-dimethyl-9H-xanthen-4,5-dicarboxamide dicarbamate, as a white solid (361.3 mg, 35%). m.p. 100-110˚C
$^1$H NMR ($CDCl_3$, 300 MHz) δ: 8.32 (broad s, 2H, NHCO), 7.85 (s, 2H, ArH), 7.54 (s, 2H, ArH), 5.46 (broad s, 2H, NHCO), 3.57 (m, 4H, $CH_2NH$), 3.25 (m, 4H, $CH_2NH$), 1.81 (m, 4H, $CH_2$), 1.65 (s, 6H, $CH_3$), 1.43 [s, 18H, $C(CH_3)_3$], 1.35 [s, 18H, C $(CH_3)_3$] .
$^{13}$C NMR ($CDCl_3$, 75 MHz) δ: 166.5; 156.7 (CO), 145.8; 145.7; 129.8; 121.7 (ArC), 125.7 (ArCH), 79.0; 77.1 [$OC(CH_3)_3$], 37.0; 36. 2 ($CH_2N$), 34.5; 34.4 [$C(CH_3)$], 32.6; 31.3[$C(CH_3)_3$] , 29. 9 ($CH_2$), 28.4 ($CH_3$).
MS (APCI$^+$) m/z 723.5 ([M+H]$^+$, 100%).

| Elemental analysis | | | |
|---|---|---|---|
| Calculated for $C_{41}H_{62}N_4O_7 \cdot 1/2MeOH$: | C 67.45; | H 8.73; | N 7.58 |
| Found | C 67.44; | H 8.77; | N 7.82 |

A solution of 2,7-di-tert-butyl-N4,N5-bis(3-aminopropyl)-9,9-dimethyl-9H-xanthen-4,5-dicarboxamide dicarbamate obtained in the previous reaction (701 mg, 0.97 mmol) in trifluoroacetic acid (5 mL) was stirred for 20 minutes at room temperature. The solvent was eliminated under reduced pressure, the obtained residue was dissolved in ethyl acetate and the organic phase was successively washed with 1 M NaOH and with water to neutral pH, it was dried ($MgSO_4$) and concentrated to dryness to give 2,7-di-tert-butyl-N4,N5-bis(3-aminopropyl)-9,9-dimethyl-9H-xanthen-4,5-dicarboxamide (445.4 mg, 870) .
m.p. 110-120 ˚C
$^1$H NMR ($CDCl_3$, 300 MHz) δ: 8.83 (m, 2H, NHCO) , 7.75 (d, $^3J$ = 2.4 Hz, 2H, ArH), 7.51 (d, $^3J$ = 2.4 Hz, 2H, ArH), 3.62 (m, 4H, $CH_2NH$), 2.88 (broad s, 4H, $CH_2NH_2$), 2.14 (broad s, 4H, $NH_2$), 1.82 (m, 4H, $CH_2$), 1.64 (s, 6H, $CH_3$), 1.33 [s, 18H, $C(CH_3)_3$].
$^{13}$C NMR ($CDCl_3$, 75 MHz) δ: 166.5 (CO), 145.8; 145.6; 129.8; 122.0 (ArC), 125.5; 125.4 (ArCH), 39.8; 38.2 ($CH_2N$),

34.5; 34.4 [$C(CH_3)$], 32.5 ($CH_3$), 31.3[$C(CH_3)_3$], 29.6 ($CH_2$).
MS ($APCI^+$) m/z 523.3 ($[M+H]^+$, 100%).

| Elemental analysis | | | |
|---|---|---|---|
| Calculated for $C_{31}H_{46}N_4O_3.2H_2O$-1AcOEt: | C 64.99; | H 9.04; | N 8.66 |
| Found | C 64.89; | H 8.45; | N 9.01 |

**b) Preparation of 2,7-di-tert-butyl-9,9-dimethyl-9H-xanthen-4,5-dicarboxylic acid bis-[[3-(2-dibutylcarbamoyl-methoxy-acetylamino)propyl]-amide] (compound 19)**

2,7-Di-tert-butyl-N4,N5-bis(3-aminopropyl)-9,9-dimethyl-9H-xanthen-4,5-dicarboxamide obtained in step a) (209.8 mg, 0.4 mmol) and N-(3-dimethylaminopropyl)-N-ethyl-carbodiimide hydrochloride (EDC.HCl) (161 mg, 0.84 mmol) were added, under argon atmosphere, to a solution of acid 20d (196.2 mg, 0.8 mmol) in dichloromethane (7 mL). The mixture was stirred for 48 h at room temperature, 1 M HCl was added and the aqueous phase was extracted with ethyl acetate. The organic phases were combined and washed with $H_2O$ and with a saturated NaCl solution and dried ($MgSO_4$). The solvent was eliminated under reduced pressure and the crude reaction product was purified by silica gel column chromatography (dichloromethane/methanol 94:6) to give compound 19 as an oil (179.7 mg, 46%).
$^1H$ NMR ($CDCl_3$, 500 MHz) δ: 8.29 (m, 2H, NHCO), 8.04 (s, 2H, NHCO), 7.82 (d, $^3J$ = 2.2 Hz, 2H, ArH), 7.52 (d, $^3J$ = 2.2 Hz, 2H, ArH), 4.26 (s, 4H, $OCH_2$), 4.10 (s, 2H, $CH_2O$), 3.54 (m, 4H, $CH_2NH$), 3.44 (m, 4H, $CH_2NH$), 3. 30 (t, $^3J$ = 8.0 Hz, 4H, $CH_2N$), 3.14 (t, $^3J$ = 8.0 Hz, 4H, $CH_2N$), 1.87 (m, 4H, $CH_2$) , 1.64 (s, 6H, $CH_3$) , 1.50 (m, 8H, $CH_2$), 1.35 [s, 18H, C $(CH_3)_3$], 0.91 (m, 12H, $CH_3$).
$^{13}C$ NMR ($CDCl_3$, 125 MHz) δ: 170.1; 168.3; 166.7 (CO), 145.8; 145.7; 129.8; 122.0 (ArC), 125.7; 125.6 (ArCH), 71.6; 69.7 ($OCH_2$), 46.6; 45.8; 38.7; 36.6; 36.0 ($CH_2N$), 34.6; 34.5 [$C(CH_3)$], 31.0; 29.7; 29.6 [$C(CH_3)$], 32.6; 31.4; 30.3; 20.2; 20.0 ($CH_2$), 13.9; 13.8 ($CH_3$).
MS ($APCI^+$) m/z 977.7 ($[M+H]^+$, 100%).

| Elemental analysis | | | |
|---|---|---|---|
| Calculated for $C_{55}H_{88}N_6O_9$: | C 67.59; | H 9.08; | N 8.60 |
| Found | C 67.99; | H 9.39; | N 7.84 |

**EXTRACTION TESTS**

**Evaluation of the extraction capability of the compounds of the invention from aqueous solutions of Am(III) and Eu(III) in 3 mol/L nitric acid**

**A) Preparation of the organic phase**

The organic solutions of the extractant compounds to be tested were prepared dissolving an amount of the extractant compound in the solvent considered in each case to reach the ligand concentration of 0.1 mol/L. The solvents considered were 1-octanol, n-heptane, n-dodecane, the industrial solvent "TPH" (hydrogenated tetrapropylene) used in the "La Hague" reprocessing plant of the COGEMA company (Compagnie générale des matières nucléaires) and different mixtures "TPH/*1-octanol* or n-dodecane/1-octanol".

**B) Organic phase equilibration**

Once said solution was obtained, its equilibration was carried out. To that end, 800 μL of the organic solution of the compound to be studied were contacted with 800 μL of nitric acid of 3 mol/L concentration and it was stirred vigorously in an oscillating shaker for 5 minutes. The phases were separated by centrifugation at 5000 rpm. 800 μL of the said organic phase were again contacted with 800 μL of nitric acid of 3 mol/L concentration for another 5 minutes of vigorous shaking. The phases were separated by centrifugation, the organic phase being considered to be equilibrated.

**C) General extraction process**

600 μL of the organic phase of the compound to be studied, previously equilibrated, were contacted with 600 μL of aqueous phase consisting of a 3 mol/L nitric acid solution spiked with 600 Bq of $^{152}Eu$ and 600 Bq of $^{241}Am$. It was stirred vigorously by means of an oscillating shaker for 30 minutes and the phases were separated by centrifugation

at 5000 rpm. 400 μL were taken from each of the phases for determining the concentration of the radionuclides $^{152}Eu$ and $^{241}Am$. This determination was carried out by means of the high energy gamma spectrometry technique with a high-purity Ge detector (Canberra Inc.), using the 122 keV line for the determination of $^{152}Eu$ concentration and the 59.5 keV line for the determination of $^{241}Am$ concentration. The distribution coefficient was calculated by relating the concentration of each of the radionuclides in the organic phase and in the aqueous phase, according to the expression:

$$D_M = \frac{[M]_{org}}{[M]_{aq}}$$

The Eu/Am selectivity factor was calculated by relating the corresponding distribution coefficients of Eu with those of Am in the same experimental conditions:

$$SF_{Eu/Am} = D_{Eu}/D_{Am}$$

Example 20

**Evaluation of the extraction capability from aqueous solutions of Am(III) and Eu(III) in 3 M nitric acid of compounds 3, 4, 5, 6, 8, 9, 10, 11, 14, 15, 16, 18 and 19**

Compounds 3, 4, 5, 6, 8, 9, 10, 11, 14, 16, 18 and 19 were dissolved at 0.1 mol/L in the "TPH/1-octanol (50:50)$_{\%vol}$" mixture and the compound 15 was dissolved at 0.1 mol/L in 1-octanol. The distribution coefficients and the selectivity factor of each of them were determined as indicated in sections A) to C).

Table 2 shows the distribution coefficients ($D_M$) for Am(III) and Eu(III) obtained after the extraction process from a 3 mol/L nitric acid solution spiked with $^{152}Eu$ and $^{241}Am$, when compounds 3, 4, 5, 6, 8, 9, 10, 11, 14, 16, 18 and 19 dissolved at 0.1 mol/L in the "TPH/1-octanol (50:50) $_{\%vol}$" mixture and compound 15 dissolved at 0.1 mol/L in 1-octanol are used. Considering that in the "TPH/1-octanol(50:50)$_{\%vol}$" mixture or in 1-octanol, the values of the distribution coefficients are lower than those obtained in the "TPH/1-octanol" mixtures with proportions of 1-octanol between 5% and 10% (v/v), as obtained in the extraction studies with compounds 4, 16 and 18 (figures 8, 9 and 10), that can be extrapolated to the remaining studied bis-diglycolamides, it is considered that the distribution coefficients of the compounds shown in table 2 are high enough for the quantitative extraction of Am(III) and Eu(III) from the aqueous solution. Furthermore, these new compounds have a greater Eu(III)/Am(III) selectivity than TBDGA and DMDODGA (extractants of the state of the art), especially compounds 4 ($SF_{Eu/Am}$ = 4.3), 10 ($SF_{Eu/Am}$ = 6.7), 16 ($SF_{Eu/Am}$ = 5.3), 18 ($Sf_{Eulam}$ = 6.0) and 19 ($SF_{Eu/Am}$ = 4.9).

**TABLE 2: Extraction properties of compounds 3, 4, 5, 6, 8, 9, 10, 11, 14, 15, 16, 18 and 19**

| Compound | Number or Acronym | $D_{Am}$ | $D_{Eu}$ | $SF_{Eu/Am}$ |
|---|---|---|---|---|
| 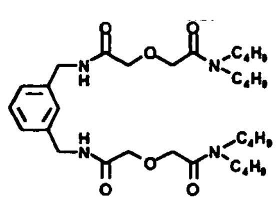 | 3 | 99 | 183 | 1.9 |
| 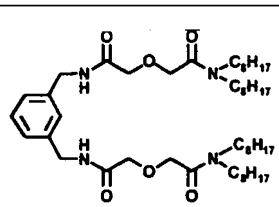 | 4 | 33 | 141 | 4.3 |

(continued)

| Compound | Number or Acronym | $D_{Am}$ | $D_{Eu}$ | $SF_{Eu/Am}$ |
|---|---|---|---|---|
| 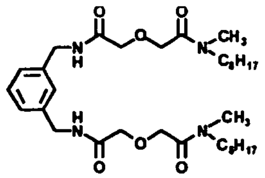 | 5 | 83 | 309 | 3.7 |
| 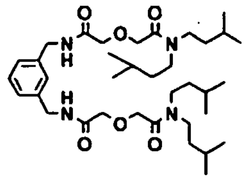 | 6 | 82 | 251 | 3.1 |
| 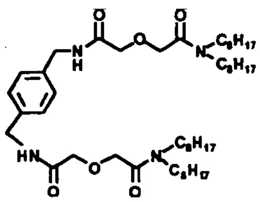 | 8 | 67 | 276 | 4.1 |
| 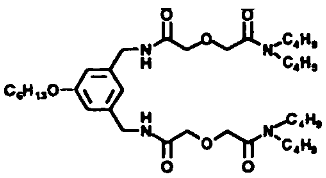 | 9 | 104 | 330 | 3.2 |
| 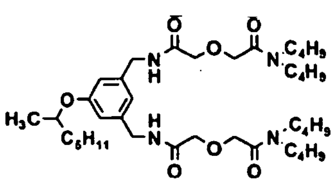 | 10 | 41 | 276 | 6.7 |
| 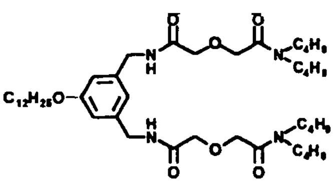 | 11 | 88 | 335 | 3.8 |
| 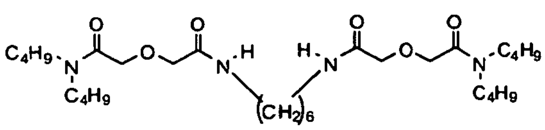 | 14 | 119 | 215 | 1.8 |
| 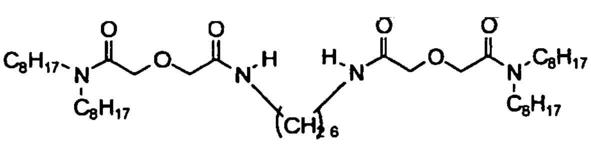 | 15(*) | 25 | 98 | 3.9 |

(continued)

| Compound | Number or Acronym | $D_{Am}$ | $D_{Eu}$ | $SF_{Eu/Am}$ |
|---|---|---|---|---|
| 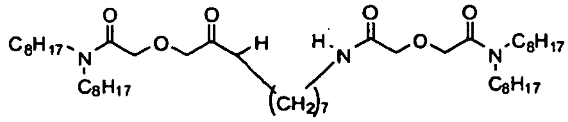 | 16 | 29 | 156 | 5.3 |
| 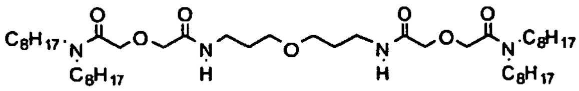 | 18 | 29 | 177 | 6.0 |
| 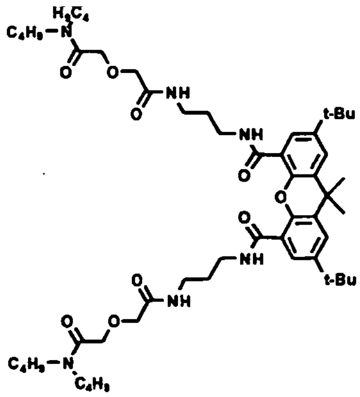 | 19 | 65 | 318 | 4.9 |
| 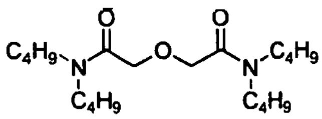 | TBDGA | 476 | 680 | 1.4 |
| 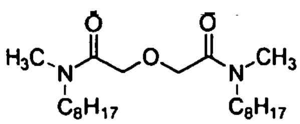 | DMDODGA | 477 | 789 | 1.7 |

Organic solution: [3, 4, 5, 6, 8, 9, 10, 11, 14, 16, 18 and 19, TBDGA and DMDODGA] = 0.1 mol/L in "TPH/1-octanol (50:50)%vol" mixture and [15] = 0.1 mol/L in 1-octanol.
Aqueous solution: $[HNO_{3]}$ 3 mol/L, spiked with $^{152}$ Eu(III) and $^{241}$Am(III).

**Determination of the extraction kinetics**

Solutions of the compounds to be studied were prepared and equilibrated according to the process detailed in the previous sections A) and B).

600 μL of the equilibrated organic phase were put into five vials with an approximate volume of 2 mL and were contacted with 600 μL of the aqueous phase formed by a 3 mol/L nitric acid solution spiked with 600 Bq of $^{152}$Eu and with 600 Bq of $^{241}$Am. They were stirred vigorously in an oscillating shaker for different time periods comprised between 5 minutes and 100 minutes. The separation of the phases was carried out by centrifugation at 5000 rpm. 400 μL of each of the phases was taken for the determination of the concentration of $^{152}$Eu and $^{241}$Am, as in the previous section C).

**Example 21**

**Determination of the extraction kinetics of compounds 3, 4, 6, 9, 10, 11, 14, 15, 16, 18 and 19**

The distribution coefficients of Am(III) and Eu(III) were determined using 0.1 mol/L solutions of compounds 3, 4, 6, 9, 10, 11, 14, 15, 16, 18 and 19 in different solvents such as in 1-octanol, n-heptane and the "TPH/1-octanol (50:50) $_{\%vol}$" and "TPH/1-octanol (75:25) $_{\%vol}$" mixtures, as indicated in the description of Figures 1 to 7.

One of the main characteristics that these new compounds must fulfill for their industrial application is that the

contact time period between the aqueous and organic phases necessary to establish equilibrium must be as minimal as possible. As observed in Figures 1 to 7, the equilibrium is reached within the first five minutes of the mixing time in all the tested compounds, therefore, the extraction kinetics are fast enough in all the cases for their industrial application in processes with centrifugal contactors.

**Determination of the extraction capability of the compounds of the invention at different nitric acid concentrations in the aqueous phase.**

The organic solutions were prepared as indicated previously in section A). The aqueous solutions consisted of nitric acid solutions at different concentrations (0.06 mol/L, 0.11 mol/L, 0.65 mol/L, 1.0 mol/L, 3.1 mol/L and 6.7 mol/L) each of them spiked with 600 Bq of $^{152}Eu$ and 600 Bq of $^{241}Am$. The nitric acid concentration of these solutions was determined by a potentiometric titration before and after the extraction equilibrium.

The organic solutions of the compound to be studied were equilibrated with the corresponding aqueous solution, according to the different nitric acid concentrations considered, as indicated previously in section B).

600 μL of equilibrated organic phase were contacted with 600 μL of the aqueous phase of the corresponding nitric acid concentration. It was stirred vigorously by means of an oscillating shaker for 15 minutes. The phases were separated by centrifugation at 5000 rpm. 400 μL of each of the phases were taken for the determination of the concentration of $^{152}Eu$ and $^{241}Am$, as indicated previously in section C).

**Example 22**

**Determination of the extraction capability of compound 4 at different nitric acid concentrations in the aqueous phase**

The influence of the concentration of aqueous nitric acid of the aqueous phase, from 0.05 mol/L to 7 mol/L, on the extraction of Am/Eu with compound 4 was studied in different TPH/1-octanol mixtures as solvent, and also in 100% 1-octanol. To that end, the distribution coefficients of Am(III) and Eu(III) were determined for compound 4 dissolved at 0.1 mol/L in 1-octanol and in the following TPH/1-octanol mixtures: 50:50, 75:25 and 90:10 by volume, for the following nitric acid concentrations of the solution containing Am(III)/Eu(III): 0.06 mol/L; 0.11 mol/L; 0.65 mol/L; 1.0 mol/L; 3.1 mol/L and 6.7 mol/L.

The results are shown in Figure 8. As it can be observed, the extraction capability of compound 4 increases as the nitric acid concentration increases with all the organic solvents tested. The extraction capability of compound 4 also increases as the concentration of 1-octanol in the organic solvent decreases. The highest extraction coefficients are obtained for values of nitric acid concentration equal to or greater than 3 mol/L, corresponding to the typical values of nitric acid concentration of HLLW from the PUREX process (3 mol/L < $HNO_3$ < 6 mol/L). It is important to point out that at diluted nitric acid concentrations the extraction capability of compound 4 is very low, facilitating the subsequent step of re-extraction of Am/Eu to regenerate the organic phase with a view to its re-use.

**Example 23**

**Determination of the extraction capability of compound 16 at different nitric acid concentrations in the aqueous phase**

The variation of the extraction coefficients of Am(III) and Eu(III) with the nitric acid concentration in the aqueous phase was studied for compound 16 dissolved at 0.1 mol/L in the following mixtures: "TPH/1-octanol $(50 : 50)_{\%vol}$" and "TPH/1-octanol $(95 : 5)_{\%vol}$".

As shown in Figure 9, the distribution coefficients increase with the nitric acid concentration, being maximum at nitric acid concentrations equal to or greater than 3 mol/L which, as indicated in example 22, is the nitric acid concentration present in the HLLW from the PUREX process. The extraction capability of compound 16 is low at diluted nitric acid concentrations; therefore the subsequent re-extraction step is favored.

**Example 24**

**Determination of the extraction capability of compound 18 at different nitric acid concentrations in the aqueous phase**

The variation of the extraction coefficients of Am(III) and Eu(III) with the nitric acid concentration was studied for compound 18 dissolved at 0.1 mol/L in the "TPH/1-octanol $(50:50)_{\%vol}$" mixture and in the "TPH/1-octanol $(95:5)_{\%vol}$"

mixture.

As it is shown in Figure 10, the distribution coefficients increase with the nitric acid concentration. The same conclusions can be established as in examples 22 and 23.

**Study of the loading capacity of the compounds of the invention**

The organic solutions were prepared and equilibrated with 3 mol/L nitric acid as detailed in the previously described sections A) and B.

Aqueous solutions of 3 mol/L nitric acid, spiked with 600 Bq of $^{152}Eu$ and 600 Bq of $^{241}Am$, containing different amounts of stable Eu(III) (0.004 mol/L, 0.008 mol/L, 0.016 mol/L, 0.032 mol/L, 0.064 mol/L and 0.128 mol/L), obtained dissolving the corresponding amount of Eu $(NO_3)_3 \cdot 6H_2O$ salt in 3 mol/L nitric acid, were prepared.

600 μL of equilibrated organic phase were contacted with 600 μL of aqueous phase of the corresponding concentration of Eu(III). It was stirred vigorously by means of an oscillating shaker for 15 minutes. The phases were separated by centrifugation at 5000 rpm and 400 μL of each of the phases were taken for the determination of the concentration of $^{152}Eu$ and $^{241}Am$, as described previously in section C).

The loading capacity was determined according to the limiting organic concentration or LOC, which is the highest concentration of metal admitted by the organic phase before the third phase appears, as a result of the organic phase separating into two phases: a light or less heavy phase formed mainly by the organic solvent and a heavy phase formed by the complex formed by the extractant and the metal. Said LOC is obtained by plotting the concentration of extracted metal in the organic phase *versus* the concentration of metal in the aqueous phase after equilibrium, and calculating the slope change in the line, in the intersection of which the maximum concentration of metal that can be extracted by the organic phase is determined.

**Example 25**

**Determination of the loading capacity of compounds 4, 16 and 18**

The limiting organic concentration of Eu(III) ($LOC_{Eu}$) was determined for compounds 4, 16 and 18 in 3 M nitric acid with a 0.2 mol/L concentration of each ligand in the "TPH/1-octanol $(95:5)_{\%vol}$" concentration. To that end, aqueous Eu(III) solutions of concentrations of 0.004 mol/L to 0.1 mol/L were prepared, dissolving the corresponding amount of Eu $(NO_3)_3.6H_2O$ in 3 mol/L nitric acid spiked with $^{152}Eu$. The concentration of Eu(III) in the organic solution was represented *versus* the concentration of Eu(III) in the aqueous solution after the equilibrium for each ligand (compound 4 in Figure 11 and compounds 16 and 18 in Figure 12, respectively).

The presence of a third phase was not observed with any of the three tested compounds and the slope change in the lines of Figures 11 and 12, in the intersection of which the limiting organic concentration ($LOC_{EU}$) is calculated, is due to the saturation of the metal in the organic phase. Although the concentration of metal in the aqueous phase increases, the extracted amount in the organic phase remains constant from the intersection point.

Table 3 shows the limiting organic concentration of Eu(III) ($LOC_{Eu}$) obtained with the three ligands from Figures 11 and 12.

**Table 3: Limiting organic concentration of compounds 4, 16 and 18**

| Compound | 4 | 16 | 18 |
|---|---|---|---|
| $LOC_{Eu}$ | ~ 0.03 mol/L | ~ 0.03 mol/L | ~ 0.06 mol/L |

The loading capacity obtained with compounds 4, 16 and 18 is suitable for their industrial application in the extraction of An(III) and Ln(III) in HLLW from the PUREX process, because the total concentration of An(III) and Ln(III) present in HLLW from the PUREX process applied to a UOX2 type fuel, with a burn-up of 33 GWd/t and a cooling period of 4 years, is approximately 2.62 g/l (- 0.017 mol/L).

**Evaluation of the extraction capability of the compounds of the invention from a simulated high-level liquid waste from the PUREX process.**

The industrial solvent "TPH" was used as a solvent to carry out this study. The other chemical reagents and compounds are of analytical grade.

The feed solution used for the extraction tests is a simulated solution of an HLLW from the PUREX process, the composition of which is shown in Table 4.

**Table 4: Composition of the simulated HLLW-PUREX solution used for the extraction tests**

| $[HNO_3]$ 3.16 mol/L | | $[HNO_3]$ 3.16 mol/L | |
|---|---|---|---|
| Element | mg/l | Element | mg/l |
| Ag | 12.6 | Ni | 47 |
| Al | 2 | Pd | 123 |
| Ba | 264 | Rb | 65 |
| Cd | 17 | Pr | 219 |
| Ce | 518 | Rh | 80 |
| Cr | 91 | Ru | 388 |
| Cu | 19 | Sb | 4.4 |
| Cs | 556 | Se | 9 |
| Eu | 33 | Sm | 146 |
| Fe | 1900 | Sn | 11 |
| Gd | 35 | Sr | 167 |
| La | 218 | Te | 481 |
| Mo | 672 | Y | 102 |
| Na | 2034 | Zr | 1165 |
| Nd | 718 | | |

The composition of the HLLW corresponds to a burn-up of 33 $GWd/t_{HM}$, the generated volume of HLLW being 5000 1/t of fuel. The feed was spiked with $^{241}Am$, $^{244}Cm$, $^{252}Cf$ and $^{152}Eu$. Oxalic acid and N-(2-hydroxyethyl)ethylenediaminotriacetate (HEDTA) were also added before the extraction tests.

**Batch extraction process and analysis**

500 μL of the aqueous and organic phases were spiked with 10 μL of a tracer solution containing $241_{Am}$, $244_{Cm}$, $252_{Cf}$ and $^{152}{}_{Eu}$, and were equilibrated for 10 minutes while they were stirred vigorously in a vortex stirrer. After the separation of the phases by centrifugation, 200 μL aliquots of each phase were taken for the determination of the concentration of $^{241}Am$ and $^{152}Eu$ using a high-purity germanium spectrometer system (EG&G Ortec). The nuclides $^{241}Am$, $^{244}Cm$ and $^{252}Cf$ were measured by alpha spectrometry with an α-Analyst device of the company Canberra-Packard GmbH, Germany.

The concentrations of the stable elements were determined by mass spectrometry with an inductively coupled plasma source (ICP-MS) in a DRC Elan 6100 device of Perkin Elmer Sciex. The aqueous phase was measured directly after the appropriate dilution. The organic phase was analyzed after having mineralized the organic matter by microwave acid digestion and subsequent dilution.

The distribution coefficients were calculated for each element according to section C).

**Example 26**

**Evaluation of the extraction capability of compound 4 from a simulated HLLW from the PUREX process**

A 0.1 mol/L solution of compound 4 in the "TPH/*1-octanol* $(90:10)_{\%vol}$" mixture was prepared. Then, extractions were carried out with the simulated HLLW solution, the composition of which is shown in Table 4. Extraction experiments were carried out with said simulated HLLW in a pure form and also adding oxalic acid and HEDTA to it. These complexing agents have shown to be effective in the DIAMEX process for the complexing of Mo, Zr ($H_2C_2O_4$) and Pd (HEDTA).

The results of Table 5 and Figure 13 show that compound 4 (0.1 mol/L) has extraction properties similar to those of TODGA (0.2 mol /L). The actinides Am (III), Cm (III) and Cf (III) as well as trivalent lanthanides were extracted

with high distribution coefficients from HLLW with a 3.2 mol/L nitric acid concentration. Without oxalic acid and without HEDTA, Zr and Pd are co-extracted like in the process with TODGA, a process in which however, a third phase was observed in these conditions. As expected, Zr and Pd are not co-extracted when complexing agents are added to HLLW.

A great difference between the compounds TODGA and 4 is that a large amount of strontium is extracted with TODGA with high distribution coefficients, which makes it necessary to add a complementary step of re-extraction of this cation to regenerate the organic phase. With compound 4, the distribution coefficient ($D_{sr}$ < 0.13) is lower by a factor of 10 to that obtained with TODGA for this element, such that the re-extraction step is not necessary in the design of the industrial process, which entails a great advantage.

**Table 5: Extraction of actinides (III) and elements from HLLW with a 0.1 M solution of compound 4 in a "TPH/ 1-octanol (90:10)$_{\%vol}$" mixture from a simulated HLLW solution**

| Oxalic acid mol/L | No | No | 0.2 | 0.2 | 0.3 | 0.3 |
|---|---|---|---|---|---|---|
| HEDTA mol/L | No | No | 0.05 | 0.05 | 0.05 | 0.05 |
| TBP mol/L | No | 0.5 | No | 0.5 | No | 0.5 |
| **Element** | **Distribution coefficients** | | | | | |
| Am-241, $\gamma$ | 99 | 70 | 131 | 63 | 141 | 82 |
| Am-241, $\alpha$ | 112 | 58 | 158 | 52 | 164 | 83 |
| Cm-244 | 191 | 95 | 262 | 83 | 286 | 133 |
| Cf-252 | 2079 | 692 | 2057 | 702 | 1816 | 1403 |
| Eu-152 | 293 | 294 | 379 | 430 | 553 | 348 |
| Eu | 685 | 327 | 764 | 362 | 513 | 446 |
| Ce | 23 | 16 | 36 | 16 | 32 | 21 |
| Gd | 166 | 122 | 239 | 91 | 258 | 190 |
| La | 13 | 10 | 21 | 10 | 18 | 13.7 |
| Nd | 94 | 55 | 142 | 52 | 120 | 68 |
| Pr | 39 | 26 | 59 | 26 | 53 | 32 |
| Sm | 615 | 233 | 690 | 253 | 451 | 378 |
| Tb | 123 | 57 | 153 | 54 | 107 | 77 |
| Dy | 141 | 64 | 171 | 86 | 195 | 184 |
| Mo | 0.069 | 0.066 | 0.087 | 0.065 | 0.115 | 0.094 |
| Pd | 2.5 | 2.6 | 0.073 | 0.081 | 0.043 | 0.041 |
| Rh | 0.00066 | 0.00069 | 0.00086 | 0.00068 | 0.00069 | 0.00067 |
| Ru | 0.25 | 0.27 | 0.29 | 0.29 | 0.17 | 0.24 |
| Sr | 0.071 | 0.061 | 0.110 | 0.071 | 0.132 | 0.099 |
| Y | 1753 | 1773 | 1766 | 1698 | 1708 | 1658 |
| Zr | 133 | 295 | 0.096 | 0.062 | 0.099 | 0.022 |
| Ag | 37 | 39 | 0.013 | 0.013 | 0.013 | 0.012 |
| Ba | 0.00019 | 0.00019 | 0.00019 | 0.00020 | 0.00020 | 0.00020 |
| Cd | 0.034 | 0.033 | 0.034 | 0.035 | 0.037 | 0.036 |
| Cs | 0.000084 | 0.000082 | 0.000087 | 0.000087 | 0.000604 | 0.000087 |
| Rb | 0.0078 | 0.0080 | 0.0093 | 0.0082 | 0.0082 | 0.0081 |
| Sb | 0.012 | 0.011 | 0.011 | 0.012 | 0.012 | 0.011 |

(continued)

| Element | Distribution coefficients | | | | | |
|---|---|---|---|---|---|---|
| Sn | 0.0073 | 0.0089 | 0.0076 | 0.0087 | 0.0092 | 0.0072 |
| Te | 0.0011 | 0.0012 | 0.0014 | 0.0012 | 0.0012 | 0.0011 |

Organic phase: 0.1 mol/L of compound 4 or 0.1 mol/L of compound 4 + 0.5 mol/L of TBP in a "TPH/1-octanol $(90:10)_{\%vol}$" mixture (not pre-equilibrated with $HNO_3$.).
Aqueous phase: simulated HLLW, with or without + 0.2, 0.3 mol/L of oxalic acid + 0.05 mol/L of HEDTA, trace amounts of $^{241}Am$, $^{244}Cm$, $^{252}Cf$ and $^{152}Eu$.

**Example 27**

**Evaluation of the extraction capability of compound 18 from a simulated HLLW from the PUREX process**

A 0.1 mol/L solution of compound 18 in the "TPH/*1-octanol* $(90:10)_{\%vol}$" mixture was prepared. Then, extractions were carried out with the simulated HLLW, the composition of which is shown in Table 4. Extraction experiments were carried out with said simulated HLLW in a pure form and also adding oxalic acid and HEDTA to it.

The results are shown in Table 6. The influence of the complexing agents (oxalic acid and HEDTA) and the addition of TBP to the extracting solution were evaluated. No significant differences were observed between compounds 4 and 18. Therefore, this compound has the same advantages against TODGA as compound 4.

**Table 6: Extraction of actinides (III) and elements from HLLW with a 0.1 M solution of compound 18 in a "TPH/*1-octanol* $(90:10)_{vol}$" mixture from a simulated HLLW solution**

| Oxalic acid mol/L | No | No | 0.2 | 0.2 | 0.3 | 0.3 |
|---|---|---|---|---|---|---|
| HEDTA mol/L | No | No | 0.05 | 0.05 | 0.05 | 0.05 |
| TBP mol/L | No | 0.5 | No | 0.5 | No | 0.5 |
| **Element** | **Distribution coefficients** | | | | | |
| Am-241, γ | 96 | 65 | 100 | 70 | 97 | 61 |
| Am-241, α | 96 | 56 | 46 | 55 | 87 | 90 |
| Cm-244 | 165 | 102 | 85 | 95 | 163 | 164 |
| Cf-252 | 1352 | 605 | 511 | 666 | 1451 | 1353 |
| Eu-152 | 499 | 393 | 523 | 282 | 436 | 330 |
| Eu | 267 | 299 | 341 | 309 | 275 | 335 |
| Oxalic acid mol/L | No | No | 0.2 | 0.2 | 0.3 | 0.3 |
| HEDTA mol/L | No | No | 0.05 | 0.05 | 0.05 | 0.05 |
| TBP mol/L | No | 0.5 | No | 0.5 | No | 0.5 |
| **Element** | **Distribution coefficients** | | | | | |
| Ce | 17 | 14 | 24 | 20 | 24 | 22 |
| Gd | 160 | 126 | 197 | 140 | 196 | 146 |
| La | 10 | 8.5 | 12 | 12 | 12 | 12 |
| Nd | 74 | 49 | 101 | 69 | 97 | 78 |

(continued)

| Element | Distribution coefficients | | | | | |
|---|---|---|---|---|---|---|
| Pr | 32 | 24 | 44 | 32 | 43 | 35 |
| Sm | 266 | 234 | 432 | 284 | 331 | 363 |
| Y | 574 | 1119 | 618 | 631 | 455 | 811 |
| Zr | 9.1 | 16.5 | 0.51 | 0.18 | 0.13 | 0.06 |
| Ag | 5.8 | 6.8 | 0.5 | 0.19 | 0.14 | 0.08 |
| Pd | 1.7 | 2.2 | 0.10 | 0.05 | 0.04 | 0.02 |
| Ru | 0.24 | 0.28 | 0.21 | 0.22 | 0.18 | 0.23 |
| Mo | 0.09 | 0.06 | 0.21 | 0.15 | 0.19 | 0.19 |
| Sr | 0.033 | 0.031 | 0.043 | 0.045 | 0.041 | 0.047 |
| Cu | 0.064 | 0.067 | 0.075 | 0.085 | 0.083 | 0.093 |
| Cd | 0.021 | 0.015 | 0.032 | 0.022 | 0.034 | 0.024 |
| Ni | 0.014 | 0.014 | 0.014 | 0.015 | 0.014 | 0.015 |
| Cr | 0.006 | 0.006 | 0.006 | 0.006 | 0.006 | 0.006 |
| Fe | 0.001 | 0.001 | 0.001 | 0.001 | 0.001 | 0.009 |
| Rb | 0.008 | 0.008 | 0.008 | 0.008 | 0.008 | 0.008 |
| Rh | 0.004 | 0.003 | 0.004 | 0.003 | 0.003 | 0.003 |
| Ba | 0.0008 | 0.0008 | 0.0008 | 0.0008 | 0.0008 | 0.0008 |
| Cs | 0.0004 | 0.0004 | 0.0004 | 0.0005 | 0.0006 | 0.0004 |

Organic phase: 0.1 mol/L of compound 18 or 0.1 mol/L of compound 18 + 0.5 mol/L of TBP in a "TPH/1-octanol (90:10)$_{\%vol}$" mixture (not pre-equilibrated with $HNO_3$.).
Aqueous phase: simulated HLLW, with or without + 0.2; 0.3 mol/L of oxalic acid + 0.05 mol/L of HEDTA, trace amounts of $^{241}$Am, $^{244}$Cm, $^{252}$Cf and $^{152}$Eu.

**Determination of the re-extraction capability of Am(III) and** Eu(III) with diluted nitric acid solutions from organic solutions containing the compounds of the invention.

**Example 28**

**Re-extraction tests of An(III) and Ln(III) with compounds 4 and 18**

From the extraction results of compound 4 shown in Figure 8, it is deduced that the re-extraction of Am(III) and Eu(III) with diluted nitric acid solutions is possible, to regenerate the organic phase with a view to its re-use in a continuous process.

0.1 mol/L samples of compound 4 in a "TPH/1-octanol (95:5)$_{\%vol}$" mixture were prepared. Extractions of Am (III) and Eu(III) were carried out from 3 mol/L nitric acid solutions containing only the radionuclides $^{152}$Eu and $^{241}$Am and 3 mol/L nitric acid solutions containing the radionuclides $^{152}$Eu and $^{241}$Am and 0.016 mol/L of stable Eu(III), simulating the total concentration of Ln(III) in a HLLW from the PUREX process. Once the extraction process was carried out, a washing step with a 1 mol/L nitric acid solution was carried out. Then, different re-extraction steps were carried out with 0.01 mol/L nitric acid solutions, simulating a continuous process. The evolution of the concentration of Am(III) and Eu (III) in the organic phase in the different extraction, scrubbing and re-extraction steps is shown in Figure 14.

The obtained results indicate that when the tests are carried out with tracers ($^{152}$Eu and $^{241}$Am), more than 99.9% of Am(III) and Eu(III) is recovered from the organic phase after three re-extraction steps, whereas when there is 0.016 mol/L of stable Eu(III), an additional re-extraction step is necessary. In both cases, it is possible to regenerate the organic solution for its re-use in subsequent extraction cycles in the continuous process.

Figure 15 shows the evolution of the nitric acid concentration in the aqueous phase in the different extraction,

scrubbing and re-extraction steps.

The same experiment was carried out with compound 18 and the evolution of the concentration of Am(III) and Eu(III) in the organic phase is shown in Figure 16. When only the radionuclides $^{152}Eu$ and $^{241}Am$ are present, the complete re-extraction of both elements to the aqueous phase is achieved after four re-extraction steps. However, when 0.016 mol/L of Eu(III) are present, six re-extraction steps are necessary. Nevertheless, it is possible to regenerate the organic solutions of compound 18 for subsequent extraction cycles in an industrial continuous process.

Figure 17 shows the evolution of the nitric acid concentration in the aqueous phase in the different extraction, scrubbing and re-extraction steps.

The regeneration of the organic solutions for subsequent extraction cycles is very important in the industrial process, considering the production costs of the ligands and the generation of organic radioactive wastes that are difficult to manage.

**Determination of the hydrolytic stability of the compounds of the invention**

The organic solutions of the compounds to be tested were prepared and equilibrated with 3 mol/L nitric acid, as has been indicated in sections A) and B).

Then, 700 μL of the equilibrated organic phase were put in vials of an approximate volume of 2 mL and were contacted with 700 μL of 3 mol/L nitric acid. They were stirred vigorously in an oscillating shaker for periods of 7, 15, 24, 36, 42, 50 and 66 days. After each permanent contact period with the nitric acid aqueous phase, the extraction capability of the organic solution was evaluated, taking 600 μL of the corresponding organic phase and contacting it with 600 μL of aqueous phase formed by a 3 mol/L nitric acid solution spiked with 600 Bq of $^{152}Eu$ and with 600 Bq of $^{241}Am$. It was stirred vigorously by means of an oscillating shaker for 15 minutes. The separation of the phases was carried out by centrifugation at 5000 rpm. 400 μL of each of the phases were taken for the determination of the concentration of $^{152}Eu$ and $^{241}Am$, as in section C).

**Example 29**

**Determination of the hydrolytic stability of compounds 4 and 18**

The stability of compounds 4 and 18 against hydrolysis was studied by contacting a 0.1 mol/L organic solution of compound 4 or a 0,1 mol/L organic solution of compound 18 in the "n-dodecane/1-octanol $(95:5)_{\%vol}$" mixture, previously equilibrated with 3 mol/L nitric acid, with a 3 mol/L nitric acid solution for periods of 7, 15, 24, 36, 42, 50 and 66 days in the case of compound 4 and periods of 7, 15, 24, 36 and 42 days in the case of compound 18. After the permanent contact periods with the 3 mol/L nitric acid solution, the extraction capability of the organic solutions of both compounds was evaluated with 3 mol/L nitric acid solutions containing 600 Bq of $^{152}$ Eu and 600 Bq of $^{241}Am$. The evolution of the distribution coefficients of Am(III) and Eu(III) during the study of the hydrolytic stability for compounds 4 and 18 is shown in Figure 18.

As observed in Figure 18, both compounds are very stable against hydrolysis, because the extraction capability of Eu (III) ($D_{Eu}$) remains constant during the different contact time periods considered, and although the extraction capability of Am(III) ($D_{Am}$) decreases slightly in both compounds, after 66 days of permanent contact, the value for compound 4 is 145 and after 42 days of permanent contact is 280 for compound 18. In both cases, the extraction capability is still very high, allowing a quantitative extraction of An(III) and Ln(III) from the aqueous solution of HLLW.

**Determination of the radiolytic stability of the compounds of the invention**

The tests of stability against irradiation were carried out in the NAYADE facility at CIEMAT site, with external $^{60}Co$ sources with an approximate activity of 5000 Ci, with a homogeneous irradiation flux. The dose rate applied in all the experiments was 4 kGy/h and integrated doses of 250 kGy, 500 kGy, 750 kGy and 1000 kGy were considered. The irradiation device consists of a metal cylinder which is introduced in a rack wherein the $^{60}CO$ sources are placed radially, such that the irradiation is homogeneous. The irradiation process is carried out submersing the device in a pool of water with a depth of 4 meters.

Irradiation tests of the solid compound (in the absence of solvent) and of the compound dissolved in the "n-dodecane/1-octanol $(95:5)_{\%vol}$" mixture, equilibrated with 3 mol/L nitric acid, were carried out. The organic solutions were prepared and equilibrated as detailed in the previous sections A) and B).

Once each programmed integrated dose was reached, the extraction capability of the organic samples was evaluated as detailed in section C).

**Example 30**

**Determination of the radiolytic stability of compound 4**

The irradiation of compound 4 in solid form (in the absence of solvent) and dissolved at a 0.1 mol/L concentration in the "n-dodecane/1-octanol 1 $(95:5)_{\%vol}$" mixture, equilibrated with 3 mol/L nitric acid, was carried out. The applied dose rate was 4 kGy/h and the considered integrated doses were 250 kGy, 500 kGy, 750 kGy and 1000 kGy. After each integrated dose, the extraction capability of Am(III) and Eu(III) was evaluated as described in section C. The results are shown in Figure 19.

The compound is very stable against radiolysis when it is irradiated in the absence of solvent, high values of the distribution coefficients being obtained after 1000 kGy: $D_{Am}$ = 540 and $D_{Eu}$ = 1000. When it is irradiated dissolved in the "n-dodecane/1-octanol $(95:5)_{\%vol}$" mixture and equilibrated with 3 mol/L nitric acid, the extraction capability of Eu (III) remains virtually constant and that of Am(III) slightly decreases, although the value after 1000 kGy is sufficiently high: $D_{Am}$ = 180 and $D_{Eu}$ = 780.

**Example 31**

**Determination of the radiolytic stability of compound 18**

The gamma irradiation of compound 18 was carried out in the same facility and in same experimental conditions as with compound 4 (example 30). The considered integrated doses in this experiment were 500 kGy and 1000 kGy at a dose rate of 4 kGy/h. This compound is very stable against radiolysis up to 1000 kGy and the loss of the extraction capability is not observed when the sample is irradiated in the absence of solvent or when it is irradiated dissolved in the "n-dodecane/1-octanol $(95:5)_{\%vol}$" mixture, equilibrated with 3 mol/L nitric acid ($D_n$ = 531 and $D_{EU}$ = 964). The values of the distribution coefficients are shown in Figure 20.

The results obtained with the solutions of compounds 4 and 18 in n-dodecane/1-octanol (95:5)%vol at an integrated dose of 1000 KGy are better than those obtained with TODGA in the same experimental conditions ($D_{Am}$= 121 and $D_{Eu}$= 458).

**Claims**

1. A compound having general formula (I)

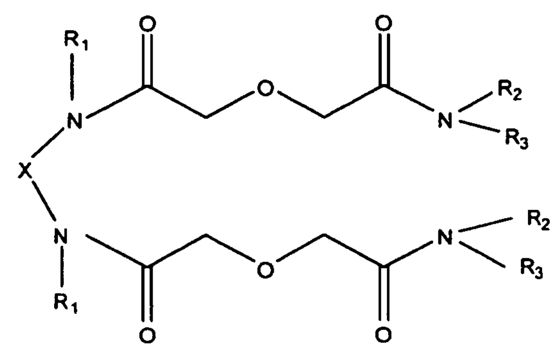

(I)

wherein
X represents a spacer selected from:

a) a xylylene radical non-substituted or optionally substituted with an $R_4O$ alcoxy group, wherein $R_4$ is a linear or branched $C_6$-$C_{12}$ alkyl group,
b) a $C_5$-$C_7$ alkylidene radical,
c) a $R_5$-O-$R_5$ group, wherein $R_5$ is a $C_2$-$C_3$ alkylidene radical, and

d) a xanthene-derived group having formula (II)

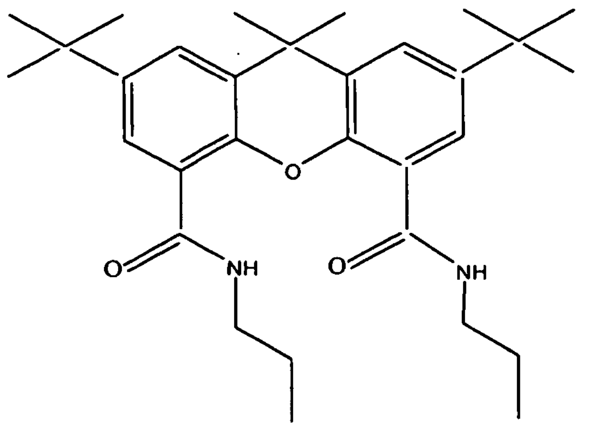

(II)

$R_1$ is an H atom or a $C_1$-$C_3$ alkyl radical,
$R_2$ is an H atom or a linear or branched $C_1$-$C_8$ alkyl radical,
and
$R_3$ is a linear or branched $C_4$-$C_8$ alkyl radical

**2.** A compound having general formula (I) according to claim 1 wherein X is a non-substituted xylylene radical, $R_1$ is an H atom or a methyl radical, $R_2$ is an H atom or an alkyl radical selected from methyl, butyl, isopentyl and octyl, and $R_3$ is an alkyl radical selected from butyl, isopentyl and octyl.

**3.** A compound having general formula (I) according to claim 1 wherein X is a xylylene radical substituted with an $R_4O$ alcoxy group, wherein $R_4$ is an alkyl radical selected from hexyl, sec-heptyl and dodecyl, $R_1$ is an H atom, and $R_2$=$R_3$ is butyl.

**4.** A compound having general formula (I) according to claim 1 wherein X is a $C_5$-$C_7$ alkylidene radical, $R_1$ is an H atom, and $R_2$=$R_3$ is an alkyl radical selected from butyl and octyl.

**5.** A compound having general formula (I) according to claim 1 wherein X is a $R_5$-O-$R_5$ group, wherein $R_5$ is a $C_2$-$C_3$ alkylidene radical, $R_1$ is an H atom, and $R_2$=$R_3$ is octyl.

**6.** A compound having general formula (I) according to claim 1 wherein X is a xanthene-derived group having formula (II), $R_1$ is an H atom, and $R_2$=$R_3$ is butyl.

**7.** A compound having general formula (I) according to claim 1, selected from the following group:

[1] 2-Butylcarbamoylmethoxy-N-[3-[(2-butylcarbamoylmethoxyacetylamino)methyl]benzyl]acetamide,
[2] 2-[(Butyl-methylcarbamoyl)methoxy]-*N*-[3-[(2-butyl-methylcarbamoyl)methoxyacetylamino]methyl)benzyl] acetamide,
[3] 2-Dibutylcarbamoylmethoxy-N-[3-[(2-dibutylcarbamoylmethoxy-acetylamino)methyl]benzyl]acetamide,
[4] 2-Dioctylcarbamoylmethoxy-N-[3-[(2-dioctylcarbamoylmethoxy-acetylamino)methyl]benzyl]acetamide,
[5] 2-[(Methyloctylcarbamoyl)methoxy]-N-[3-[(2-[(methyloctylcarbamoyl)methoxy]acetylamino]methyl)benzyl] acetamide,
[6] 2-[[Bis(3-methylbutyl)carbamoyl]methoxy]-N-[3-[(2-[[bis-(3-methylbutyl)carbamoyl]methoxy]acetylamino) methyl]-benzyl]-acetamide,
[7] N-Methyl-N-[3-[(methyl-[2-[(methyloctylcarbamoyl)methoxy]-acetyl]amino)methyl]benzyl]-2-[(methyloctyl-carbamoyl)methoxy]-acetamide,
[8] 2-[[4-([2-[Dioctylcarbamoyl)methoxy]acetylamino]methyl)-benzylcarbamoyl]methoxy]-N,N-dioctylacetamide,

[9] 2-Di-butylcarbamoylmethoxy-N-[3-[(2-dibutylcarbamoylmethoxyacetylamino)methyl]-5-hexyloxybenzyl] acetamide,
[10] 2-Di-butylcarbamoylmethoxy-N-[3-[(2-dibutyl-carbamoylmethoxyacetylamino)methyl]-5-(1-methylhexyloxy)benzyl]-acetamide,
[11] 2-Di-butylcarbamoylmethoxy-N-[3-[(2-dibutyl-carbamoylmethoxyacetylamino)methyl]-5-dodecyloxybenzyl]acetamide,
[12] 2-Dibutylcarbamoylmethoxy-N-[5-(2-dibutylcarbamoylmethoxyacetylamino)pentyl]acetamide,
[13] 2-Dioctylcarbamoylmethoxy-N-[5-(2-dioctylcarbamoylmethoxyacetylamino)pentyl]acetamide,
[14] 2-Dibutylcarbamoylmethoxy-N-[6-(2-dibutylcarbamoylmethoxyacetylamino)hexyl]acetamide,
[15] 2-Dioctylcarbamoylmethoxy-N-[6-(2-dioctylcarbamoylmethoxyacetylamino)hexyl]acetamide,
[16] 2-Dioctylcarbamoylmethoxy-N-[7-(2-dioctylcarbamoylmethoxyacetylamino)heptyl]acetamide,
[17] 2-Dioctylcarbamoylmethoxy-N-[2-[2-(2-dioctylcarbamoylmethoxyacetylamino)ethoxy]ethyl]acetamide,
[18] 2-Dioctylcarbamoylmethoxy-N-[3-[3-(2-dioctylcarbamoylmethoxyacetylamino)propoxy]propyl]acetamide, and
[19] 2,7-di-tert-butyl-9,9-dimethyl-9H-xanthen-4,5-dicarboxylic acid bis-[[3-(2-dibutylcarbamoylmethoxyacetylamino)propyl]amide].

**8.** A process for obtaining a compound having general formula (I) wherein X is a non-substituted xylylene radical, comprising reacting a compound having formula (III),

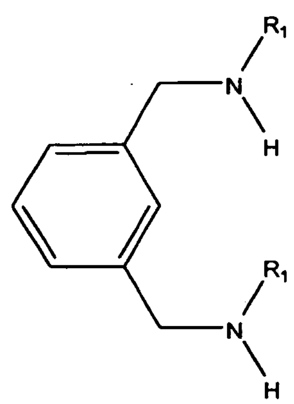

(III)

with a compound having formula (IV)

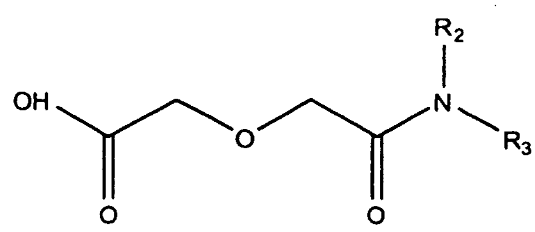

(IV)

in an inert solvent and in the presence of an activating agent, $R_1$, $R_2$ and $R_3$ having the meaning mentioned in claim 1.

**9.** A process for obtaining a compound having general formula (I) wherein X is a xylylene radical substituted with an

$R_4O$ alcoxy group, comprising reacting a compound having formula (VIII),

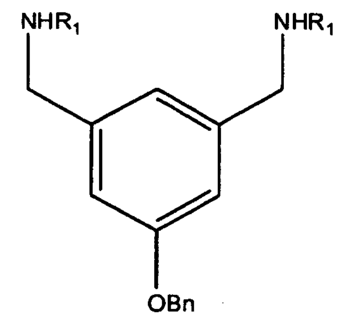

(VIII)

with a compound having formula (IV) in an inert solvent and in the presence of an activating agent, followed by a hydrogenation reaction, in an inert solvent and in the presence of a hydrogenation catalyst, giving rise to a compound having formula (IX),

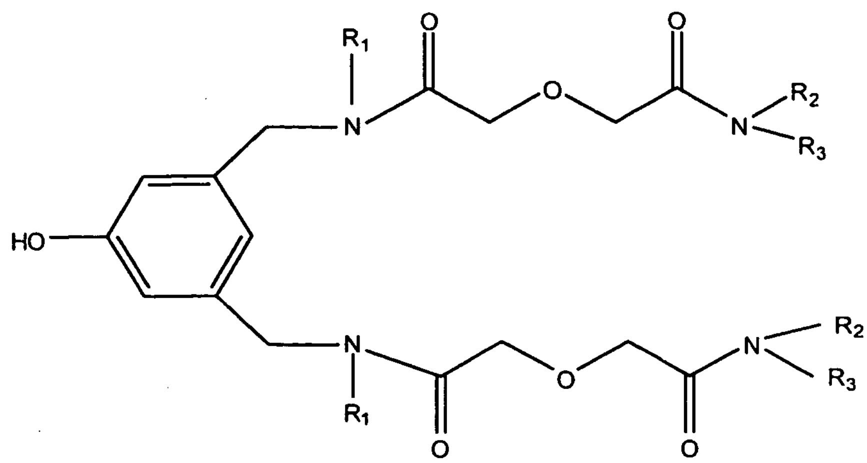

(IX)

followed by an O-alkylation reaction with $BrR_4$, in an inert solvent and in the presence of a base, $R_1$, $R_2$, $R_3$ and $R_4$ having the meaning mentioned in claim 1.

**10.** A process according to claim 9, wherein the compound having formula (VIII) is obtained by the O-benzylation with BrBn of a compound having formula (V),

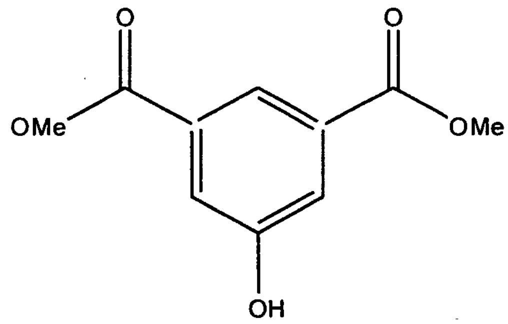

(V)

in an inert solvent and in the presence of a base, followed by a reduction reaction with $LiAlH_4$ in an inert solvent, and by the reaction of the formed dialcohol having formula (VI)

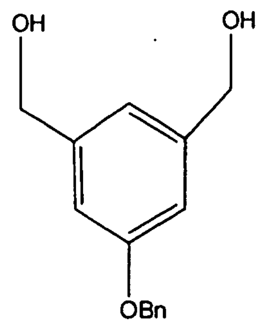

(VI)

with $SOCl_2$, giving rise to a compound having formula (VII)

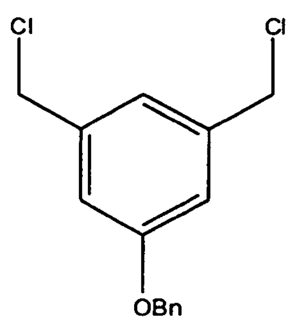

(VII)

which is subjected to a Gabriel synthesis reaction with potassium phthalimide, in an inert solvent and in the presence of sodium iodide, followed by treatment with hydrazine in an inert solvent, to obtain the compound having formula (VIII)

**11.** A process for obtaining a compound having general formula (I) wherein X is a $C_5$-$C_7$ alkylidene radical, comprising reacting a compound having formula (X)

$HNR_1$-$(CH_2)_n$-$NHR_1$ (X)

with a compound having formula (IV) in an inert solvent and in the presence of an activating agent, $R_1$, $R_2$ and $R_3$ having the meaning mentioned in claim 1 and wherein n is 5, 6 or 7.

**12.** A process for obtaining a compound having general formula (I) wherein X is a $R_5$-O-$R_5$ group, wherein $R_5$ is a $C_2$-$C_3$ alkylidene group, comprising reacting a compound having formula (XI)

$HNR_1$-$(CH_2)$n-O-$(CH_2)$n-$NHR_1$ (XI)

with a compound having formula (IV), in an inert solvent and in the presence of an activating agent, $R_1$, $R_2$ and $R_3$ having the meaning mentioned in claim 1 and wherein n is 2 or 3.

**13.** A process for obtaining a compound having general formula (I) wherein X is a xanthene-derived group having formula (II), comprising reacting a compound having formula (XIV)

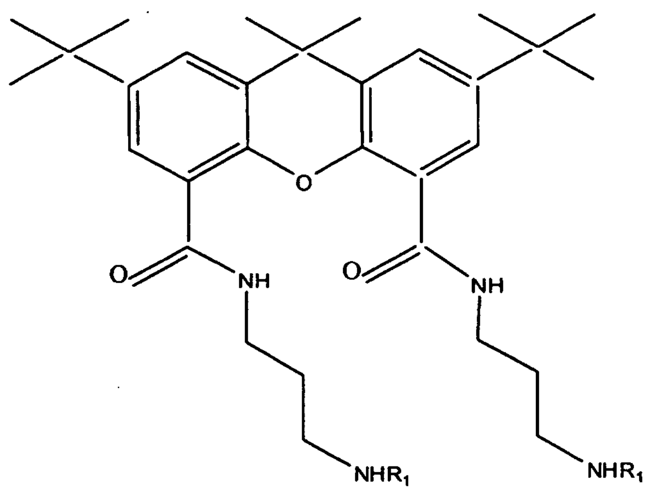

(XIV)

with a compound having formula (IV), in an inert solvent and in the presence of an activating agent, $R_1$, $R_2$ and $R_3$ having the meaning mentioned in claim 1.

**14.** A process according to claim 13, wherein the compound having formula (XIV) is obtained by reacting a compound having formula (XII)

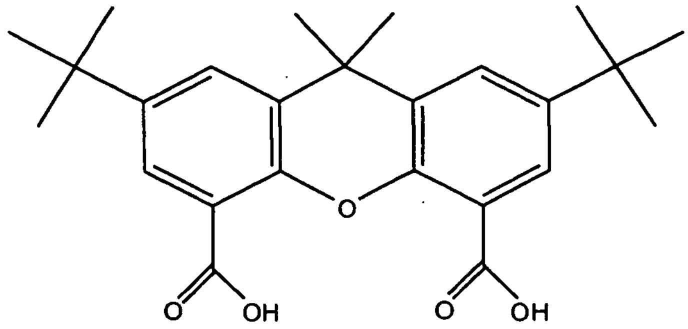

(XII)

with a compound having formula (XIII)

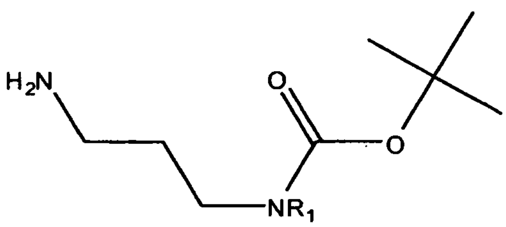

(XIII)

$R_1$ having the meaning mentioned in claim 1, in an inert solvent and in the presence of an activating agent and of a base, followed by treatment with acid.

**15.** The use of a compound having general formula (I), according to any one of claims 1-7, for the extraction of lanthanides [Ln(III)] and actinides [An(III)] present in aqueous nitric solutions.

**16.** The use according to claim 15, for the extraction of lanthanides [Ln(III)] and actinides [An(III)] present in high-level radioactive liquid wastes.

**17.** A method for the extraction of lanthanides [Ln(III)] and actinides [An(III)] from an aqueous nitric solution comprising:

a) contacting the aqueous nitric solution containing lanthanides [Ln(III)] and actinides [An(III)] with an organic solution comprising an extractant having general formula (I) and an organic solvent, and
b) extracting the actinides [An(III)] and the lanthanides [Ln(III)] from the acid solution, after a contact time period.

**18.** A method according to claim 17, **characterized in that** the contact time period in step b) is 1 to 5 minutes.

**19.** A method according to claim 17, **characterized in that** the organic solvent of the organic solution is selected from tetrachloroethane, n-heptane, 1-octanol, TPH, n-dodecane and mixtures thereof.

**20.** A method according to claim 19, **characterized in that** the solvent of the organic solution is selected from a mixture of TPH and 5-10% (v/v) of 1-octanol and a mixture of n-dodecane and 5-10% (v/v) of 1-octanol.

**21.** A method according to claim 20, **characterized in that** the solvent of the organic solution is a mixture of TPH and 5% (v/v) of 1-octanol.

**22.** An extracting solution for the extraction of lanthanides [Ln(III)] and actinides [An(III)] from aqueous nitric solutions and preferably from high-level radioactive liquid wastes, comprising an extractant having general formula (I) and a solvent selected from tetrachloroethane, n-heptane, 1-octanol, TPH, n-dodecane and mixtures thereof.

**23.** An extracting solution according to claim 22, **characterized in that** the solvent is selected from a mixture of TPH and 5-10% (v/v) of 1-octanol and a mixture of n-dodecane and 5-10% (v/v) of 1-octanol.

**24.** An extracting solution according to claim 23, **characterized in that** the solvent is a mixture of TPH and 5% (v/v) of 1-octanol.

## Patentansprüche

**1.** Verbindung mit der allgemeinen Formel (I)

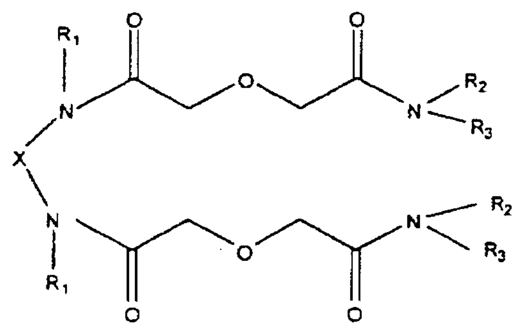

(I)

wobei
X für einen Spacer steht, der ausgewählt ist aus:

a) einem Xylylenrest, der nicht substituiert ist oder gegebenenfalls mit einer $R_4$O-Alkoxygruppe substituiert ist, wobei $R_4$ eine lineare oder verzweigte $C_6$-$C_{12}$-Alkylgruppe ist;
b) einem $C_5$-$C_7$-Alkylidenrest;
c) einer $R_5$-O-$R_5$-Gruppe, wobei $R_5$ ein $C_2$-$C_3$-Alkylidenrest ist; und
d) einer von Xanthen abgeleiteten Gruppe mit der Formel (II)

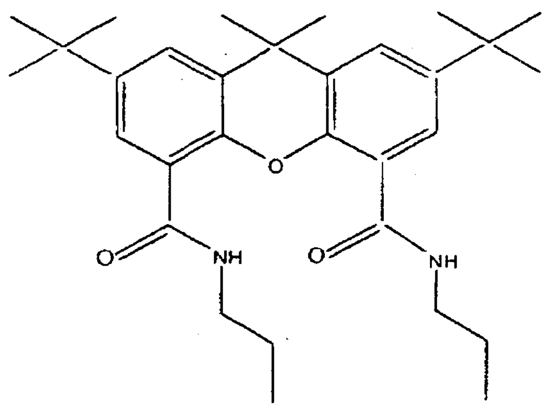

(II)

$R_1$ ein H-Atom oder ein $C_1$-$C_3$-Alkylrest ist;
$R_2$ ein H-Atom oder ein linearer oder verzweigter $C_1$-$C_8$-Alkylrest ist; und
$R_3$ ein linearer oder verzweigter $C_4$-$C_8$-Alkylrest ist.

2. Verbindung mit der allgemeinen Formel (I) gemäß Anspruch 1, wobei X ein nicht substituierter Xylylenrest ist, $R_1$ ein H-Atom oder ein Methylrest ist, $R_2$ ein H-Atom oder ein aus Methyl, Butyl, Isopentyl und Octyl ausgewählter Alkylrest ist und $R_3$ ein aus Butyl, Isopentyl und Octyl ausgewählter Alkylrest ist.

3. Verbindung mit der allgemeinen Formel (I) gemäß Anspruch 1, wobei X ein Xylylenrest ist, der mit einer $R_4$O-Alkoxygruppe substituiert ist, wobei $R_4$ ein aus Hexyl, sek-Heptyl und Dodecyl ausgewählter Alkylrest ist, $R_1$ ein H-Atom ist und $R_2$ = $R_3$ = Butyl ist.

4. Verbindung mit der allgemeinen Formel (I) gemäß Anspruch 1, wobei X ein $C_5$-$C_7$-Alkylidenrest ist, $R_1$ ein H-Atom ist und $R_2$ = $R_3$ ein aus Butyl und Octyl ausgewählter Alkylrest ist.

5. Verbindung mit der allgemeinen Formel (I) gemäß Anspruch 1, wobei X eine $R_5$-O-$R_5$-Gruppe ist, wobei $R_5$ ein $C_2$-$C_3$-Alkylidenrest ist, $R_1$ ein H-Atom ist und $R_2$ = $R_3$ = Octyl ist.

6. Verbindung mit der allgemeinen Formel (I) gemäß Anspruch 1, wobei X eine von Xanthen abgeleitete Gruppe mit der Formel (II) ist, $R_1$ ein H-Atom ist und $R_2$ = $R_3$ = Butyl ist.

7. Verbindung mit der allgemeinen Formel (I) gemäß Anspruch 1, die aus der folgenden Gruppe ausgewählt ist:

[1] 2-Butylcarbamoylmethoxy-N-[3-[(2-butylcarbamoylmethoxyacetylamino)methyl]benzyl]acetamid,
[2] 2-[(Butylmethylcarbamoyl)methoxy]-N-[3-[(2-butylmethylcarbamoyl)-methoxyacetylamino]methyl]benzyl] acetamid,
[3] 2-Dibutylcarbamoylmethoxy-N-[3-[(2-dibutylcarbamoylmethoxyacetylamino)methyl]benzyl]acetamid,
[4] 2-Dioctylcarbamoylmethoxy-N-[3-[(2-dioctylcarbamoylmethoxyacetylamino)methyl]benzyl]acetamid,
[5] 2-[(Methyloctylcarbamoyl)methoxy]-N-[3-[(2-[(methyloctylcarbamoyl)methoxy]acetylamino]methyl]benzyl] acetamid,
[6] 2-[[Bis(3-methylbutyl)carbamoyl]methoxy]-N-[3-[(2-[[bis-(3-methylbutyl)carbamoyl]methoxy]acetylamino) methyl]benzyl]acetamid,
[7] N-Methyl-N-[3-[(methyl-[2-[(methyloctylcarbamoyl)methoxy]acetyl]-amino)methyl]benzyl]-2-[(methyloctylcarbamoyl)methoxy]acetamid,
[8] 2-[[4-([2-[Dioctylcarbamoyl)methoxy]acetylamino]methyl)benzylcarbamoyl]methoxy]-N,N-dioctylacetamid,
[9] 2-Dibutylcarbamoylmethoxy-N-[3-[(2-dibutylcarbamoylmethoxyacetylamino)methyl]-5-hexyloxybenzyl]acetamid,
[10] 2-Dibutylcarbamoylmethoxy-N-[3-[(2-dibutylcarbamoylmethoxyacetylamino)methyl]-5-(1-methylhexyloxy) benzyl]acetamid,
[11] 2-Dibutylcarbamoylmethoxy-N-[3-[(2-dibutylcarbamoylmethoxyacetylamino)methyl]-5-dodecyloxybenzyl]acetamid,
[12] 2-Dibutylcarbamoylmethoxy-N-[5-(2-dibutylcarbamoylmethoxyacetylamino)pentyl]acetamid,
[13] 2-Dioctylcarbamoylmethoxy-N-[5-(2-dioctylcarbamoylmethoxyacetylamino)pentyl]acetamid,
[14] 2-Dibutylcarbamoylmethoxy-N-[6-(2-dibutylcarbamoylmethoxyacetylamino)hexyl]acetamid,
[15] 2-Dioctylcarbamoylmethoxy-N-[6-(2-dioctylcarbamoylmethoxyacetylamino)hexyl]acetamid,
[16] 2-Dioctylcarbamoylmethoxy-N-[7-(2-dioctylcarbamoylmethoxyacetylamino)heptyl]acetamid,
[17] 2-Dioctylcarbamoylmethoxy-N-[2-[2-(2-dioctylcarbamoylmethoxyacetylamino)ethoxy]ethyl]acetamid,
[18] 2-Dioctylcarbamoylmethoxy-N-[3-[3-(2-dioctylcarbamoylmethoxyacetylamino)propoxy]propyl]acetamid, und
[19] 2,7-Di-tert-butyl-9,9-dimethyl-9H-xanthen-4,5-dicarbonsäurebis[[3-(2-dibutylcarbamoylmethoxyacetylamino)propyl]amid].

**8.** Verfahren zur Herstellung einer Verbindung mit der allgemeinen Formel (I), wobei X ein nicht substituierter Xylylenrest ist, umfassend das Umsetzen einer Verbindung, die die Formel (III)

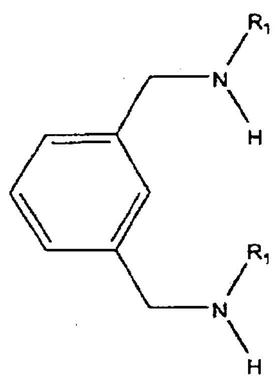

(III)

aufweist, mit einer Verbindung, die die Formel (IV)

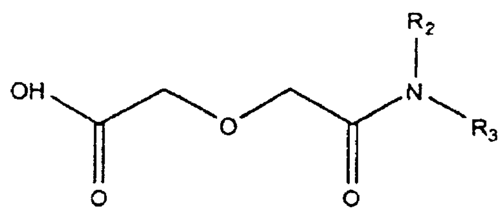

(IV)

aufweist, in einem inerten Lösungsmittel und in Gegenwart eines Aktivierungsmittels, wobei $R_1$, $R_2$ und $R_3$ die in Anspruch 1 genannte Bedeutung haben.

**9.** Verfahren zur Herstellung einer Verbindung mit der allgemeinen Formel (I), wobei X ein mit einer $R_4O$-Alkoxygruppe substituierter Xylylenrest ist, umfassend das Umsetzen einer Verbindung, die die Formel (VIII)

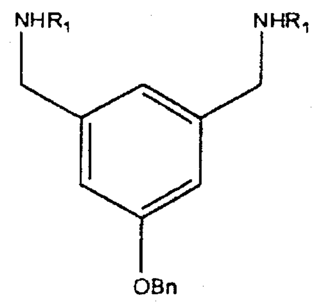

(VIII)

aufweist, mit einer Verbindung, die die Formel (IV) aufweist, in einem inerten Lösungsmittel und in Gegenwart eines Aktivierungsmittels, gefolgt von einer Hydrierungsreaktion in einem inerten Lösungsmittel und in Gegenwart eines Hydrierungskatalysators, was eine Verbindung mit der Formel (IX)

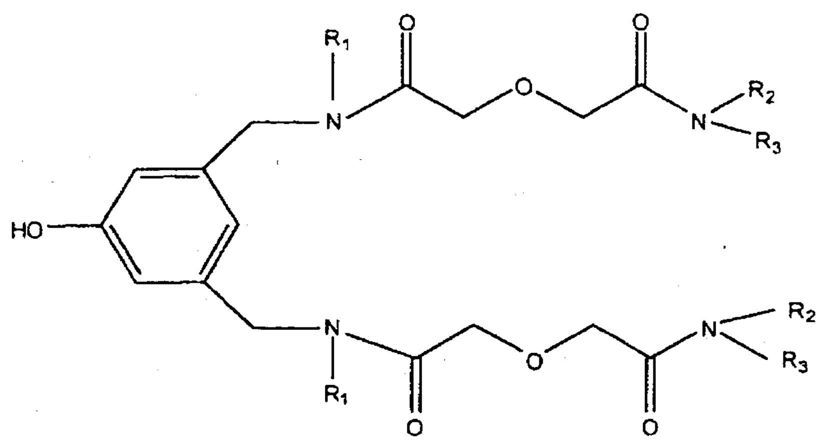

(IX)

ergibt, gefolgt von einer O-Alkylierungsreaktion mit $BrR_4$ in einem inerten Lösungsmittel und in Gegenwart einer Base, wobei $R_1$, $R_2$, $R_3$ und $R_4$ die in Anspruch 1 genannte Bedeutung haben.

**10.** Verfahren gemäß Anspruch 9, wobei die Verbindung mit der Formel (VIII) erhalten wird durch die O-Benzylierung einer Verbindung mit der Formel (V)

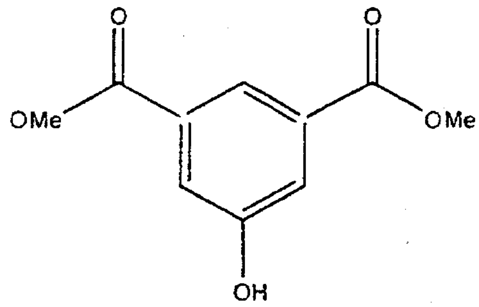

(V)

mit BrBn in einem inerten Lösungsmittel und in Gegenwart einer Base, gefolgt von einer Reduktionsreaktion mit $LiAlH_4$ in einem inerten Lösungsmittel und von der Reaktion des gebildeten Dialkohols der Formel (VI)

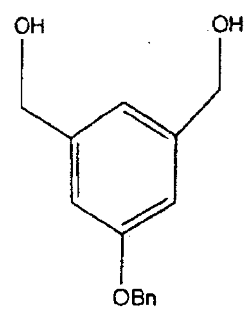

(VI)

mit $SOCl_2$, was eine Verbindung mit der Formel (VII)

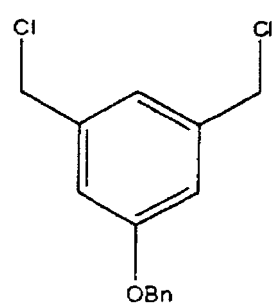

(VII)

ergibt, die einer Gabriel-Synthesereaktion mit Kaliumphthalimid in einem inerten Lösungsmittel und in Gegenwart von Natriumiodid unterzogen wird, gefolgt von einer Behandlung mit Hydrazin in einem inerten Lösungsmittel, wobei man die Verbindung mit der Formel (VIII) erhält.

**11.** Verfahren zur Herstellung einer Verbindung mit der allgemeinen Formel (I), wobei X ein $C_5$-$C_7$-Alkylidenrest ist, umfassend das Umsetzen einer Verbindung, die die Formel (X)

$HNR_1$-$(CH_2)n$-$NHR_1$ (X)

aufweist, mit einer Verbindung, die die Formel (IV) aufweist, in einem inerten Lösungsmittel und in Gegenwart eines Aktivierungsmittels, wobei $R_1$, $R_2$ und $R_3$ die in Anspruch 1 genannte Bedeutung haben und wobei n = 5, 6 oder 7 ist.

**12.** Verfahren zur Herstellung einer Verbindung mit der allgemeinen Formel (I), wobei X eine $R_5$-O-$R_5$-Gruppe ist, wobei $R_5$ eine $C_2$-$C_3$-Alkylidengruppe ist, umfassend das Umsetzen einer Verbindung, die die Formel (XI)

$HNR_1$-$(CH_2)n$-O-$(CH_2)n$-$NHR_1$ (XI)

aufweist, mit einer Verbindung, die die Formel (IV) aufweist, in einem inerten Lösungsmittel und in Gegenwart eines Aktivierungsmittels, wobei $R_1$, $R_2$ und $R_3$ die in Anspruch 1 genannte Bedeutung haben und wobei n = 2 oder 3 ist.

**13.** Verfahren zur Herstellung einer Verbindung mit der allgemeinen Formel (I), wobei X eine von Xanthen abgeleitete Gruppe mit der Formel (II) ist, umfassend das Umsetzen einer Verbindung, die die Formel (XIV)

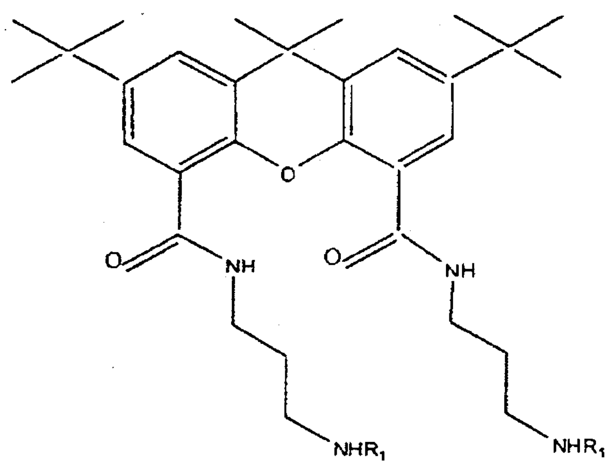

(XIV)

aufweist, mit einer Verbindung, die die Formel (IV) aufweist, in einem inerten Lösungsmittel und in Gegenwart eines Aktivierungsmittels, wobei $R_1$, $R_2$ und $R_3$ die in Anspruch 1 genannte Bedeutung haben.

**14.** Verfahren gemäß Anspruch 13, wobei die Verbindung mit der Formel (XIV) erhalten wird durch Umsetzen einer Verbindung, die die Formel (XII)

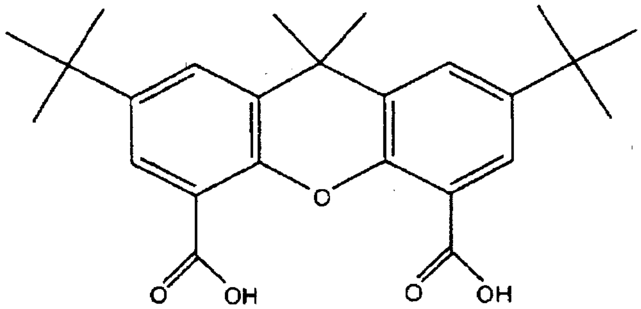

(XII)

aufweist, mit einer Verbindung, die die Formel (XIII)

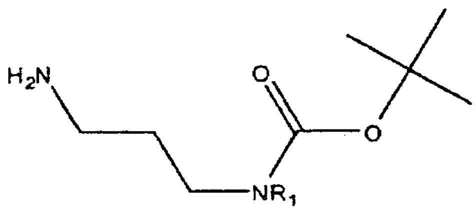

(XIII)

aufweist, wobei $R_1$ die in Anspruch 1 genannte Bedeutung hat, in einem inerten Lösungsmittel und in Gegenwart eines Aktivierungsmittels und einer Base, gefolgt von einer Behandlung mit Säure.

**15.** Verwendung einer Verbindung mit der allgemeinen Formel (I) gemäß einem der Ansprüche 1 bis 7 zur Extraktion von Lanthaniden [Ln(III)] und Actiniden [Ac(III)], die in wässrigen salpetersauren Lösungen vorhanden sind.

**16.** Verwendung gemäß Anspruch 15 zur Extraktion von Lanthaniden [Ln(III)] und Actiniden [Ac(III)], die in hochradioaktiven flüssigen Abfällen vorhanden sind.

**17.** Verfahren zur Extraktion von Lanthaniden [Ln(III)] und Actiniden [Ac(III)] aus einer wässrigen salpetersauren Lösung, umfassend:

a) das In-Kontakt-Bringen der wässrigen salpetersauren Lösung, die Lanthanide [Ln(III)] und Actinide [Ac(III)] enthält, mit einer organischen Lösung, die ein Extraktionsmittel der allgemeinen Formel (I) und ein organisches Lösungsmittel umfasst; und
b) Extrahieren der Actiniden [Ac(III)] und der Lanthaniden [Ln(III)] aus der Säurelösung nach Ablauf einer Kontaktzeit.

**18.** Verfahren gemäß Anspruch 17, **dadurch gekennzeichnet, dass** die Kontaktzeit in Schritt b) 1 bis 5 Minuten dauert.

**19.** Verfahren gemäß Anspruch 17, **dadurch gekennzeichnet, dass** das organische Lösungsmittel der organischen Lösung aus Tetrachlorethan, n-Heptan, 1-Octanol, TPH, n-Dodecan und Gemischen davon ausgewählt ist.

**20.** Verfahren gemäß Anspruch 19, **dadurch gekennzeichnet, dass** das Lösungsmittel der organischen Lösung aus einem Gemisch von TPH und 5 bis 10 Vol.-% 1-Octanol und einem Gemisch von n-Dodecan und 5 bis 10 Vol.-%

1-Octanol ausgewählt ist.

**21.** Verfahren gemäß Anspruch 20, **dadurch gekennzeichnet, dass** das Lösungsmittel der organischen Lösung ein Gemisch von TPH und 5 Vol.-% 1-Octanol ist.

**22.** Extraktionslösung zur Extraktion von Lanthaniden [Ln(III)] und Actiniden [Ac(III)] aus wässrigen salpetersauren Lösungen und vorzugsweise aus hochradioaktiven flüssigen Abfällen, umfassend ein Extraktionsmittel der allgemeinen Formel (I) und ein Lösungsmittel, das aus Tetrachlorethan, n-Heptan, 1-Octanol, TPH, n-Dodecan und Gemischen davon ausgewählt ist.

**23.** Extraktionslösung gemäß Anspruch 22, **dadurch gekennzeichnet, dass** das Lösungsmittel aus einem Gemisch von TPH und 5 bis 10 Vol.-% 1-Octanol und einem Gemisch von n-Dodecan und 5 bis 10 Vol.-% 1-Octanol ausgewählt ist.

**24.** Extraktionslösung gemäß Anspruch 23, **dadurch gekennzeichnet, dass** das Lösungsmittel ein Gemisch von TPH und 5 Vol.-% 1-Octanol ist.

## Revendications

**1.** Composé ayant la formule générale (1)

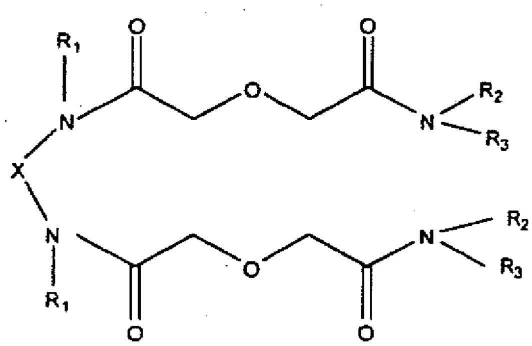

(I)

où
X représente un espaceur sélectionné parmi:

a) un radical xylylène non-substitué ou optionnellement substitué par un groupe alcoxy $R_4O$, où $R_4$ est un groupe alkyle $C_6$-$C_{12}$ linéaire ou ramifié,
b) un radical alkylidène $C_5$-$C_7$,
c) un groupe $R_5$-O-$R_5$, où $R_5$ est un radical alkylidène $C_2$-$C_3$ et
d) un groupe dérivé de xanthène de la formule (II)

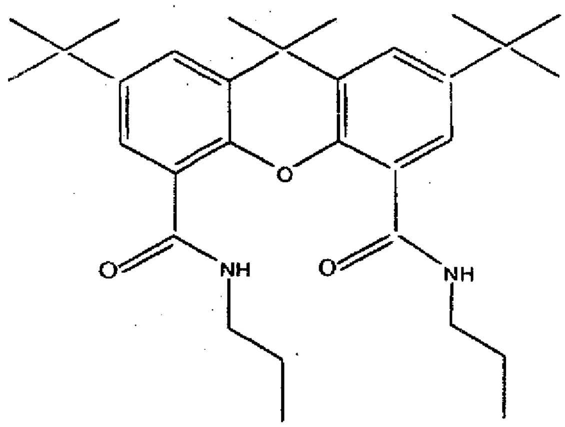

(II)

$R_1$ est un atome H ou un radical alkyle $C_1$-$C_3$,
$R_2$ est un atome H ou un radical alkyle $C_1$-$C_8$ linéaire ou ramifié,
et
$R_3$ est un radical alkyle $C_4$-$C_8$ linéaire ou ramifié.

**2.** Composé ayant la formule générale (I) selon la revendication 1, où X est un radical xylylène non-substitué, $R_1$ est un atome H ou un radical méthyle, $R_2$ est un atome H ou un radical alkyle sélectionné parmi méthyle, butyle, isopentyle et octyle, et $R_3$ est un radical alkyle sélectionné parmi butyle, isopentyle et octyle.

**3.** Composé ayant la formule générale (I) selon la revendication 1, où X est un radical xylylène substitué par un groupe alcoxy $R_4O$, où $R_4$ est un radical alkyle sélectionné parmi hexyle, sec-heptyle et dodécyle, $R_1$ est un atome H, et $R_2$=$R_3$ est butyle.

**4.** Composé ayant la formule générale (I) selon la revendication 1, dans lequel X est un radical alkylidène$C_5$-$C_7$, $R_1$ est un atome H, et $R_2$=$R_3$ est un radical alkyle sélectionné parmi butyle et octyle.

**5.** Composé ayant la formule générale (I) selon la revendication 1, dans lequel X est un groupe $R_5$-O-$R_5$, où $R_5$ est un radical alkylidène $C_2$-$C_3$, $R_1$ est un atome H, et $R_2$=$R_3$ est octyle.

**6.** Composé ayant la formule générale (I) selon la revendication 1, dans lequel X est un groupe dérivé de xanthène ayant la formule (II), $R_1$ est un atome H et $R_2$=$R_3$ est butyle.

**7.** Composé ayant la formule générale (I) selon la revendication 1, sélectionné dans le groupe suivant:

[1] 2-Butylcarbamoylméthoxy-N-[3-[(2-butylcarbamoylméthoxy-acétylamino)méthyl]benzyl]acétamide,
[2] 2-[(Butyl-méthylcarbamoyl)méthoxy]-N-[3-[(2-butylméthylcarbamoyl)méthoxyacétylamino]méthyl)benzyl] acétamide,
[3] 2-Dibutylcarbamoylméthoxy-N-[3-[(2-dibutylcarbamoylméthoxy-acétylamino)méthylibenzyllacétamide,
[4] 2-Dioctylcarbamoylméthoxy-N-(3-[(2-dioctylcarbamoylméthoxy-acétylamino)méthyl]benzyljacétamide,
[5] 2-[(Méthyloctylcarbamoyl)méthoxy]-N-[3-[(2-[(méthyloctylcarbamoyl)méthoxy]acétylamino]méthyl)benzyl) acetamide,
[6] 2-[[Bis(3-méthylbutyl)carbamoyl]méthoxy]-N-[3-[(2-[[bis-(3-méthylbutyl)carbamoyl]méthoxy]acétylamino) methyl]-benzyl]-acetamide,
[7]N-Méthyl-N-[3-[(méthyl-(2-[(méthyloctylcarbamoyl)méthoxy]acétyl]amino)méthyl]benzyl]-2-[(méthyloctylcarbamoyl)méthoxy]-acétamide,
[8] 2-[[4-([2-(Dioctylcarbamoyl)méthoxy]acétylamino) méthyl)-benzylcarbamoyl]méthoxy)-N,N-dioctylacétamide,
[9] 2-Di-butylcarbamoylméthoxy-N-[3-[(2-dibutylcarbamoylméthoxyacétylamino)méthyl]-5-hexyloxybenzyl] acétamide,
[10] 2-Di-butylcarhamoylméthoxy-N-[3-((2-dibutyl-carbamoylméthoxyacétylamino)méthyl]-5-(1-méthylhexyloxy)benzyl]-acétamide,
[11] 2-Di-butylcarbamoylméthoxy-N-[3-[(2-dibutyl-carbamoylméthoxyacétylamino)méthyl]-5-dodécyloxybenzyl]acétamide,
(12] 2-Dibutylcarbamoylméthoxy-N-[5-(2-dibutylcarbamoylméthoxyacétylamino)pentyl]acétamide,
[13] 2-Dioctylcarbamoylméthoxy-N-[5-(2-dioctylcarbamoylméthoxyacétylamino)pentyl]acétamide,
[14] 2-Dibutylcarbamoylméthoxy-N-[6-(2-dibutylcarbamoylméthoxyacétylamino)hexyl]acétamide,
[15] 2-Dioctylcarbamoylméthoxy-N-[6-(2-dioctylcarbamoylméthoxyacétylamino)hexyl]acétamide,
[16] 2-Dioctylcarbamoylmethoxy-N-[7-(2-dioctylcarbamoylméthoxyacétylamino)heptyl]acétamide,
[17] 2-Dioctylcarbamoylméthoxy-N-[2-[2-(2-dioctylcarbamoylméthoxyacétylamino)éthoxy]éthyl]acétamide,
[18] 2-Dioctylcarbamoylméthoxy-N-[3-[3-(2-dioctylcarbamoylméthoxyacétylamino)propoxy]propyl]acétamide, et
[19] Acide 2,7-di-tert-butyl-9,9-diméthyl-9H-xanthen-4,,5-dicarboxylique bis-[[3-(2-dibutylcarbamoylméthoxyacétylamino)propyl]amide].

**8.** Procédé pour obtenir un composé ayant la formule générale (I), où X est un radical xylylène non substitué, comprenant la réaction d'un composé ayant la formule (III),

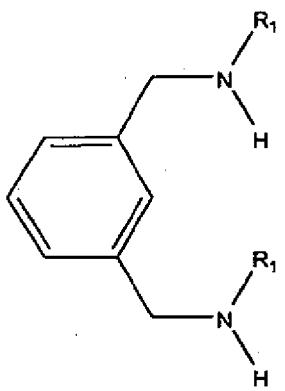

(III)

avec un composé ayant la formule (IV)

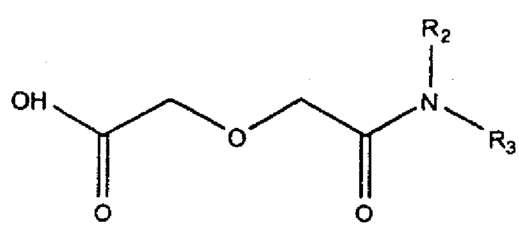

(IV)

dans un solvant inerte et en présence d'un agent d'activation, $R_1$, $R_2$ et $R_3$ ayant la signification mentionnée dans la revendication 1.

**9.** Procédé d'obtention d'un composé ayant la formule générale (I), où X est un radical xylylène substitué par un groupe alcoxy $R_4O$, comprenant la réaction d'un composé ayant la formule (VIII),

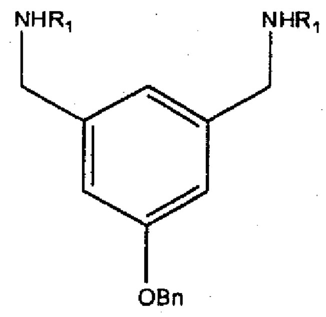

(VIII)

avec un composé ayant la formule (IV) dans un solvant inerte et en présence d'un agent d'activation, suivi d'une réaction d'hydrogénation, dans un solvant inerte et en présence d'un catalyseur d'hydrogénation, donnant lieu à un composé ayant la formule (IX),

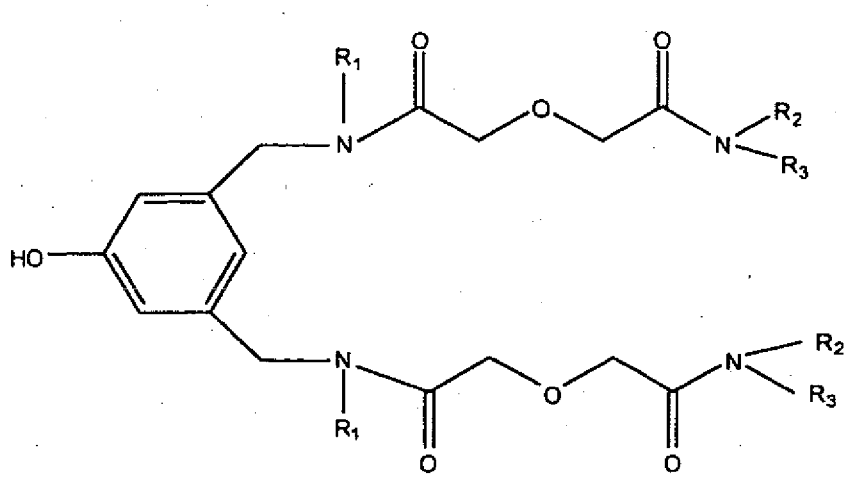

(IX)

suivi d'une réaction de O-alkylation avec $BrR_4$, dans un solvant inerte et en présence d'une base, $R_1$, $R_2$, $R_3$ et $R_4$ ayant la signification mentionnée dans la revendication 1.

**10.** Procédé selon la revendication 9, dans lequel le composé ayant la formule (VIII) est obtenu par la 0-benzylation avec BrBn d'un composé ayant la formule (V),

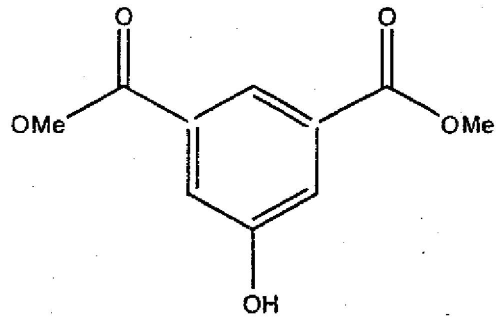

(V)

dans un solvant inerte et en présence d'une base, suivi d'une réaction de réduction avec $LiAlH_4$ dans un solvant inerte, et par la réaction du dialcool formé ayant la formule (VI)

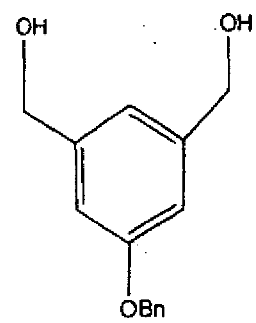

(VI)

avec $SOCl_2$, donnant lieu à un composé ayant la formule (VII)

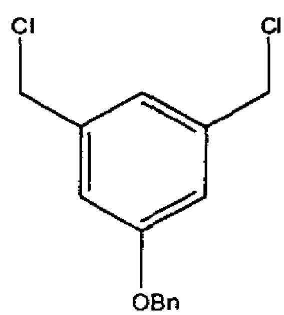

(VII)

qui est soumis à une réaction de synthèse de Gabriel avec du potassium phtalimide, dans un solvant inerte et en présence de sodium iodure, suivi du traitement avec de l'hydrazine dans un solvant inerte pour obtenir le composé ayant la formule (VIII).

**11.** Procédé d'obtention d'un composé ayant la formule générale (I) dans lequel X est un radical alkylidène $C_5$-$C_7$, comprenant la réaction d'un composé ayant la formule (X)

$$HNR_1\text{-}(CH_2)n\text{-}NHR_1 \qquad (X)$$

avec un composé ayant la formule (IV) dans un solvant inerte et en présence d'un agent d'activation, $R_1$, $R_2$ et $R_3$ ayant la signification mentionnée dans la revendication 1, et où n est 5, 6 ou 7.

**12.** Procédé d'obtention d'un composé ayant la formule générale (I), où X est un groupe $R_5$-O-$R_5$, où $R_5$ est un groupe alkylidène $C_2$-$C_3$, comprenant la réaction d'un composé ayant la formule (XI)

$$HNR_1\text{-}(CH_2)n\text{-}O\text{-}(CH_2)n\text{-}NHR_1 \qquad (XI)$$

avec un composé ayant la formule (IV), dans un solvant inerte et en présence d'un agent d'activation, $R_1$, $R_2$ et $R_3$ ayant la signification mentionnée dans la revendication 1 et où n est 2 ou 3.

**13.** Procédé d'obtention d'un composé ayant la formule générale (I), où X est un groupe dérivé de xanthène ayant la formule (II), comprenant la réaction d'un composé ayant la formule (XIV)

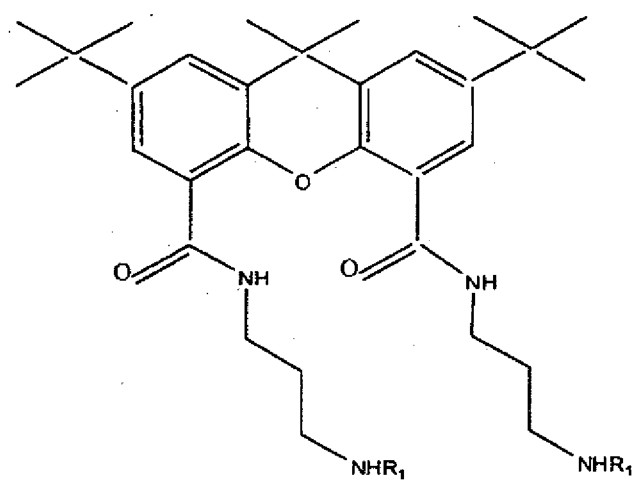

(XIV)

avec un composé ayant la formule (IV), dans un solvant inerte et en présence d'un agent d'activation, $R_1$, $R_2$ et $R_3$ ayant la signification mentionnée dans la revendication 1.

**14.** Procédé selon la revendication 13, dans lequel le composé ayant la formule (XIV) est obtenu par la réaction d'un composé ayant la formule (XII)

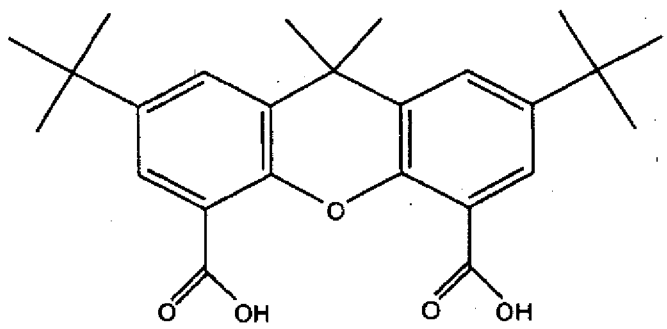

(XII)

avec un composé ayant la formule (XIII)

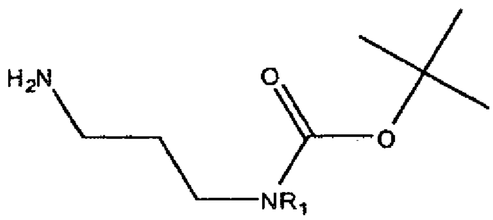

(XIII)

$R_1$ ayant la signification mentionnée dans la revendication 1, dans un solvant inerte et en présence d'un agent d'activation et d'une base, suivi du traitement avec de l'acide.

**15.** Utilisation d'un composé ayant la formule générale (I) selon l'une quelconque des revendications 1 à 7, pour l'extraction de lanthanides [Ln(III)] et actinides [An(III)] présents dans des solutions nitriques aqueuses.

**16.** Utilisation selon la revendication 15, pour l'extraction de lanthanides [Ln(III)] et actinides [An(III)] présents dans des déchets liquides radioactifs à activité élevée.

**17.** Méthode d'extraction de lanthanides [Ln(III)] et d'actinides [An(III)] d'une solution nitrique aqueuse comprenant:

a) mettre en contact la solution nitrique aqueuse contenant des lanthanides [Ln(III)] et des actinides [An(III)] avec une solution organique comprenant un solvant d'extraction ayant la formule générale (I) et un solvant organique, et
b) extraire les actinides [An(III)] et les lanthanides [Ln(III)] de la solution acide, après une période de temps de contact.

**18.** Méthode selon la revendication 17, **caractérisée en ce que** la période de temps de contact à l'étape b) est de 1 à 5 minutes.

**19.** Méthode selon la revendication 17, **caractérisée en ce que** le solvant organique de la solution organique est sélectionné parmi tétrachloroéthane, n-heptane, 1-octanol, TPH, n-dodécane et leurs mélanges.

**20.** Méthode selon la revendication 19, **caractérisée en ce que** le solvant de la solution organique est sélectionné parmi un mélange de TPH et de 5-10% (v/v) de 1-octanol et un mélange de n-dodécane et de 5-10% (v/v) de 1-octanol.

**21.** Méthode selon la revendication 20, **caractérisée en ce que** le solvant de la solution organique est un mélange de TPH et de 5% (v/v) de 1-octanol.

**22.** Solution d'extraction pour l'extraction de lanthanides [Ln(III)] et d'actinides [An(III)] de solutions nitriques aqueuses et de préférence de déchets liquides radioactifs à activité élevée, comprenant un solvant d'extraction ayant la formule générale (I) et un solvant sélectionné parmi tétrachloroéthane, n-heptane, 1-octanol, TPH, n-dodécane et leurs mélanges.

**23.** Solution d'extraction selon la revendication 22, **caractérisée en ce que** le solvant est sélectionné parmi un mélange de TPH et de 5-10% (v/v) de 1-octanol et d'un mélange de n-dodécane et de 5-10% (v/v) de 1-octanol.

**24.** Solution d'extraction selon la revendication 23, **caractérisée en ce que** le solvant est un mélange de TPH et de 5% (v/v) de 1-octanol.

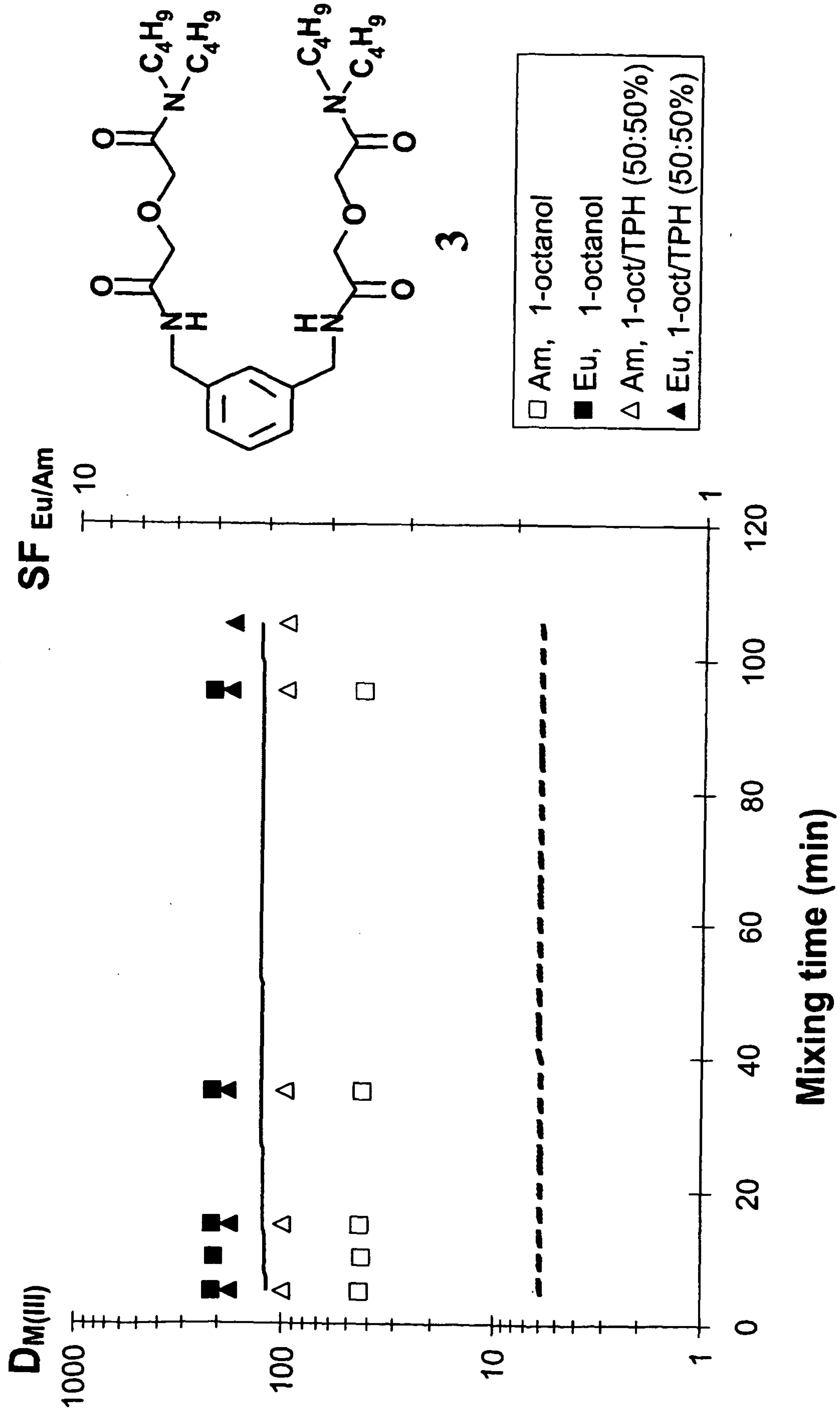

$D_{M(III)}$
1000
100
10
1
$SF_{Eu/Am}$
10
1
0
20
40
60
80
100
120
Mixing time (min)
□ Am, 1-octanol
■ Eu, 1-octanol
△ Am, 1-oct/TPH (50:50%)
▲ Eu, 1-oct/TPH (50:50%)


3

FIG. 1

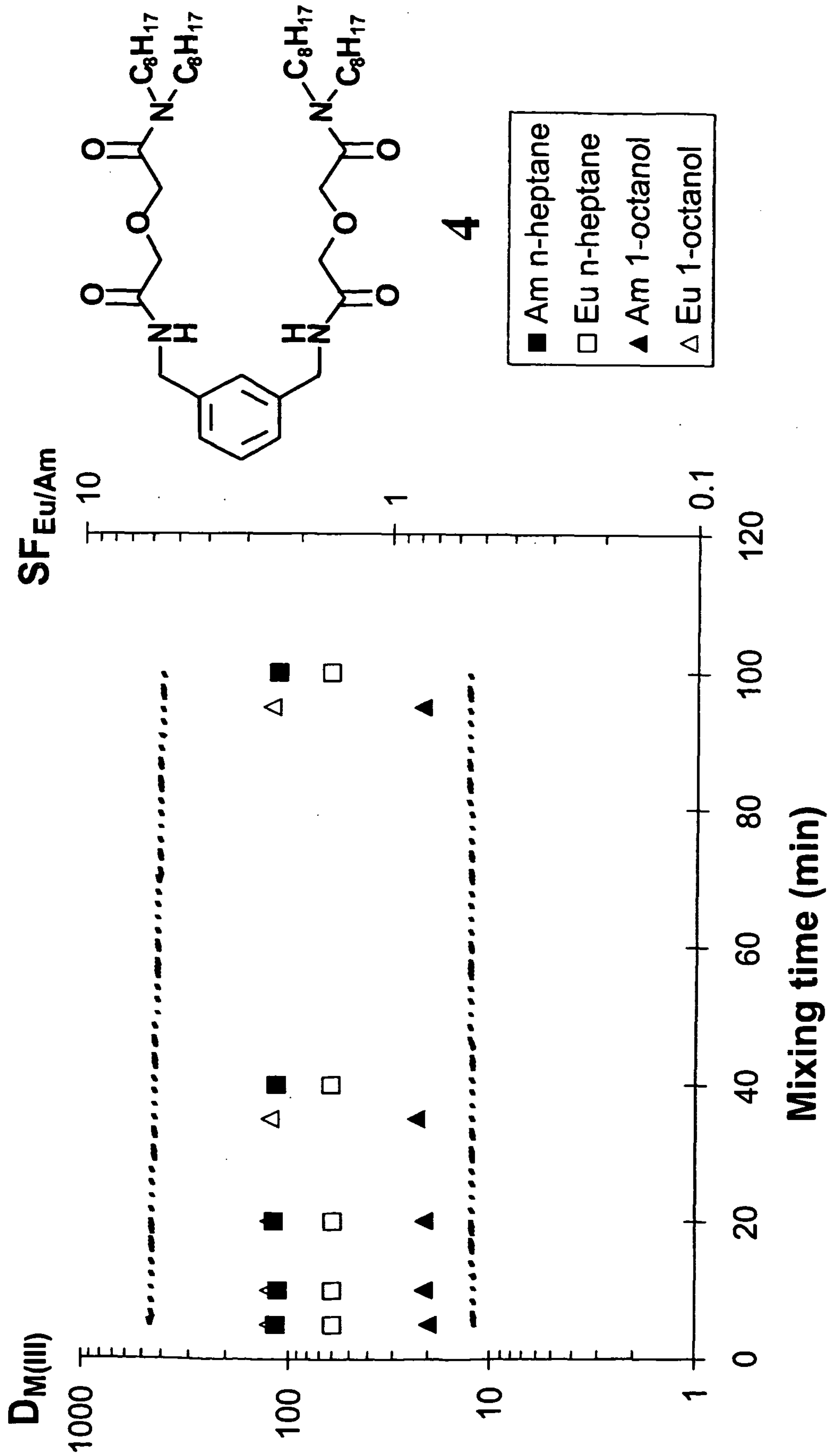

$D_{M(III)}$
1000
100
10
1
0
20
40
60
80
100
120
Mixing time (min)
$SF_{Eu/Am}$
10
1
0.1
■ Am n-heptane
□ Eu n-heptane
▲ Am 1-octanol
△ Eu 1-octanol


4

FIG. 2

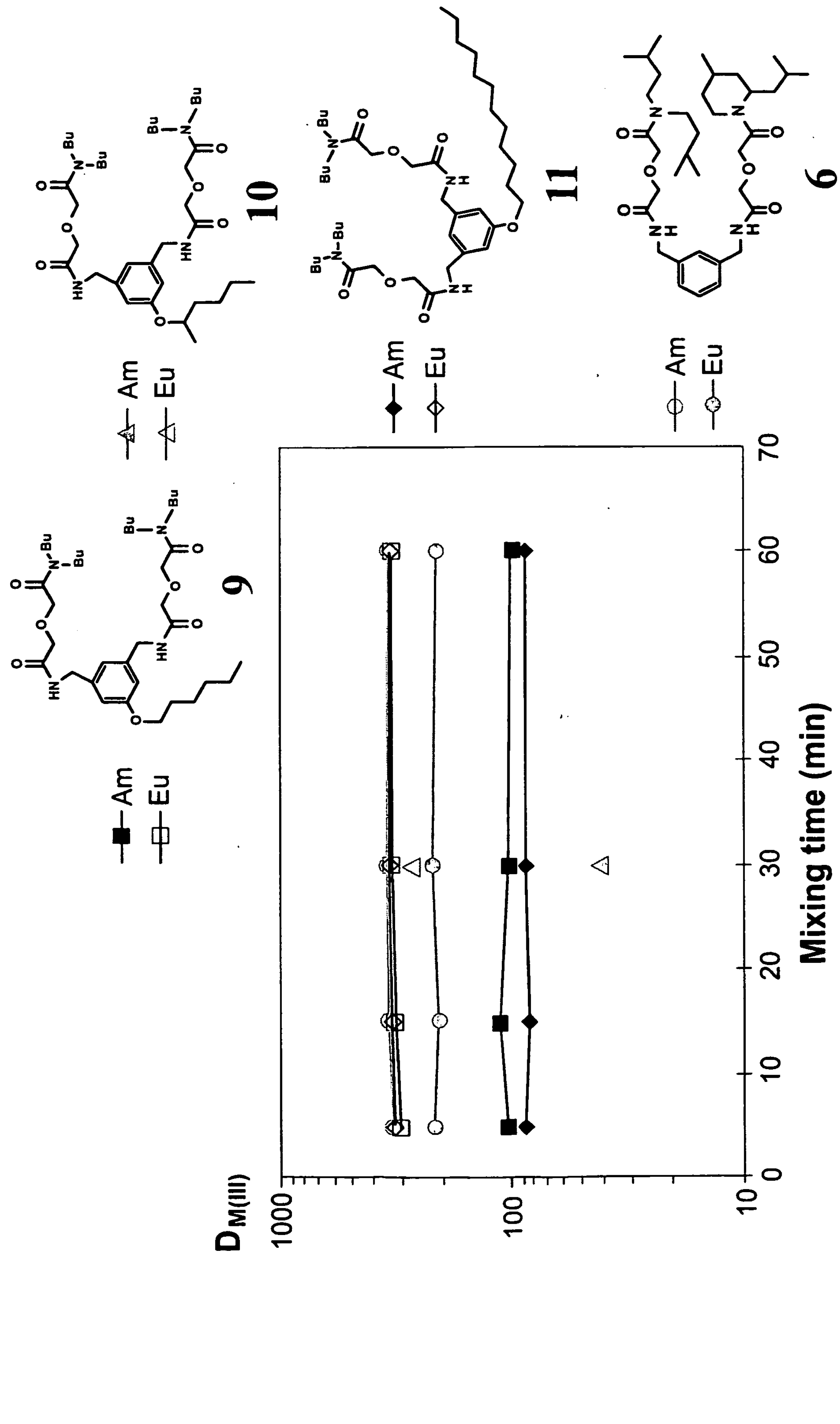
$D_{M(III)}$
1000
100
10
0
10
20
30
40
50
60
70
Mixing time (min)
Am
Eu
9
Am
Eu
10
Am
Eu
11
Am
Eu
6

FIG. 3

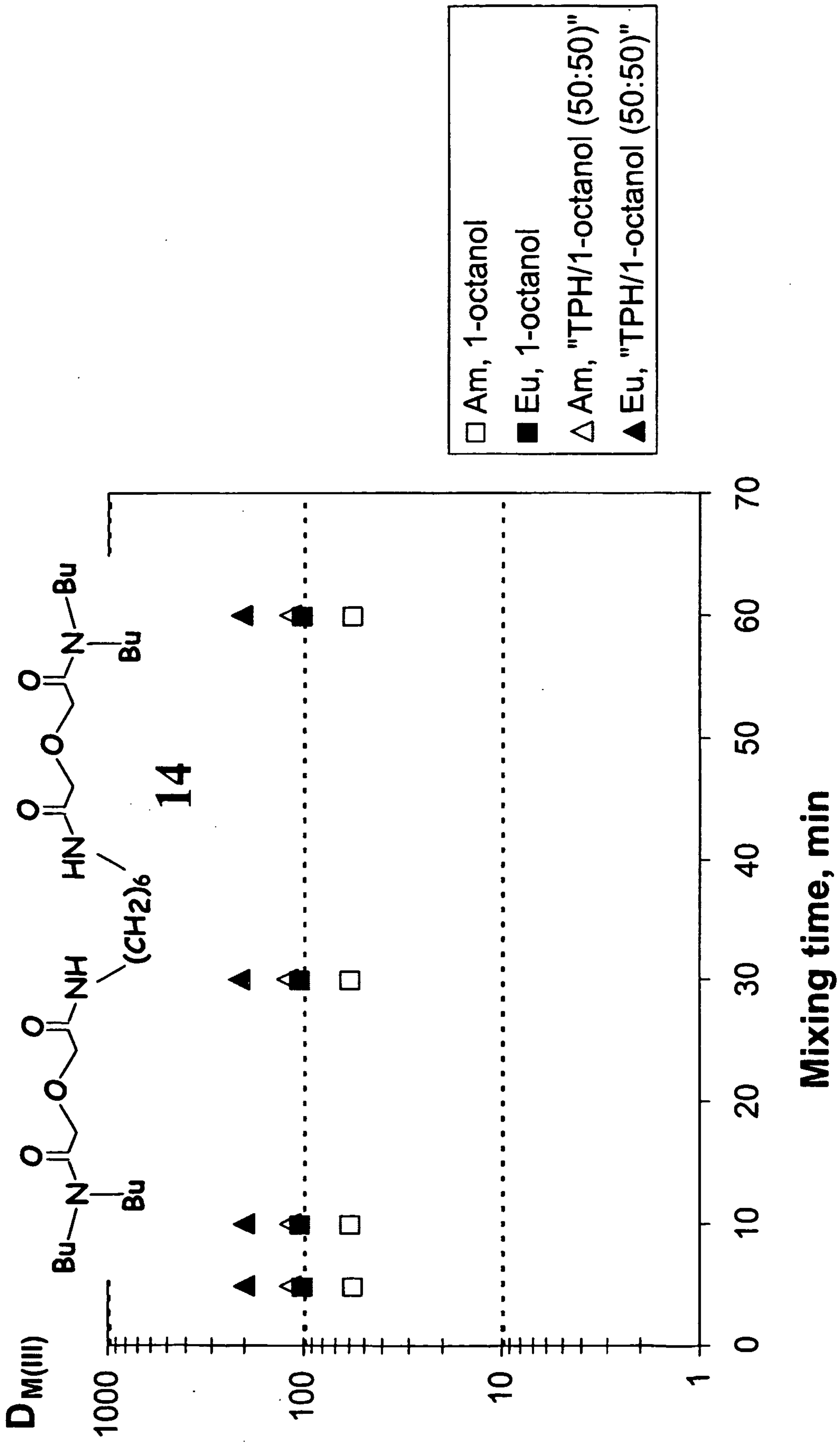
D M(III)
1000
100
10
1
0
10
20
30
40
50
60
70
Mixing time, min
□ Am, 1-octanol
■ Eu, 1-octanol
△ Am, "TPH/1-octanol (50:50)"
▲ Eu, "TPH/1-octanol (50:50)"

14

FIG. 4

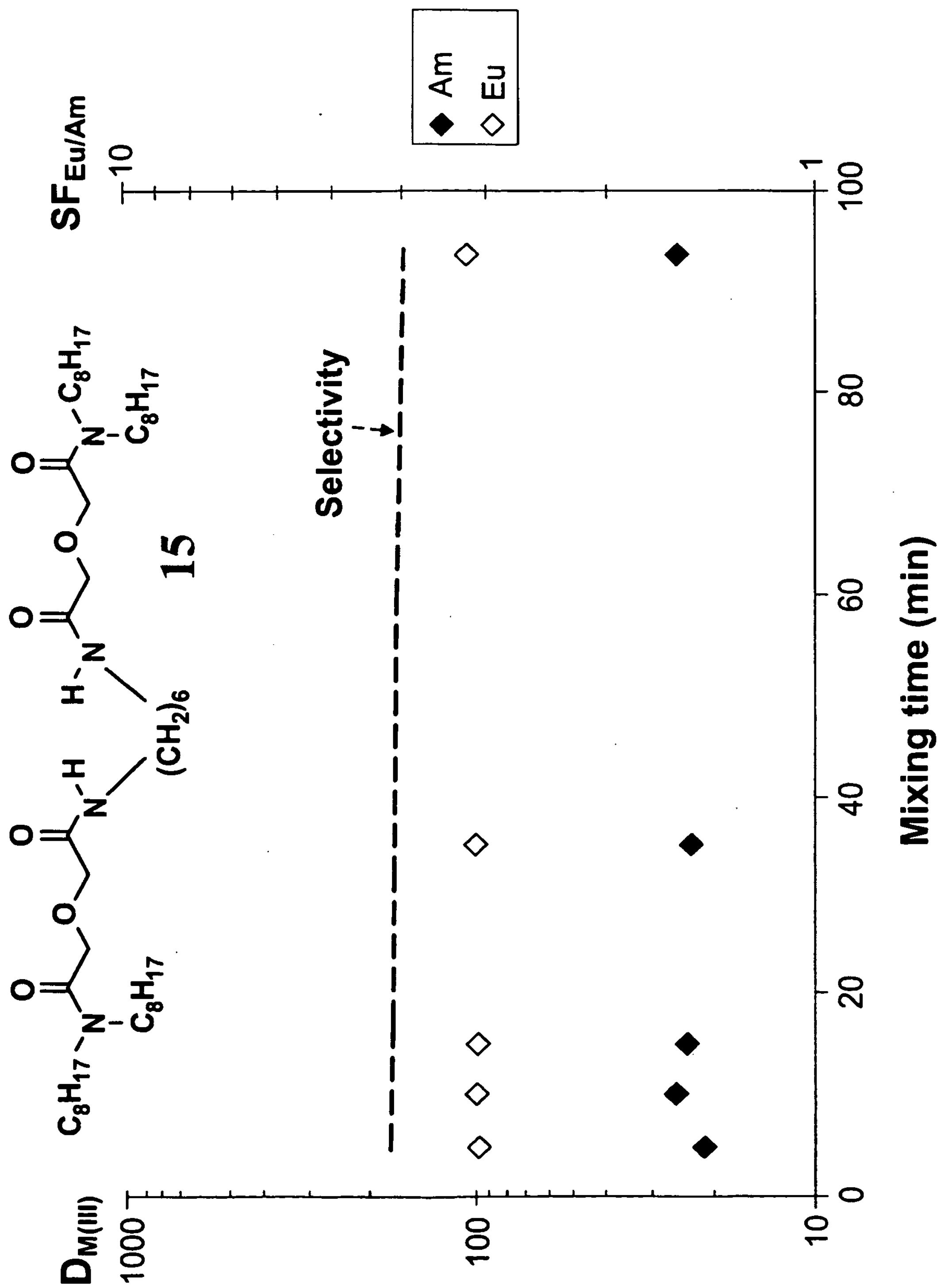
15
Selectivity
D_M(III)
SF_Eu/Am
1000
100
10
1
Am
Eu
0
20
40
60
80
100
Mixing time (min)

FIG. 5

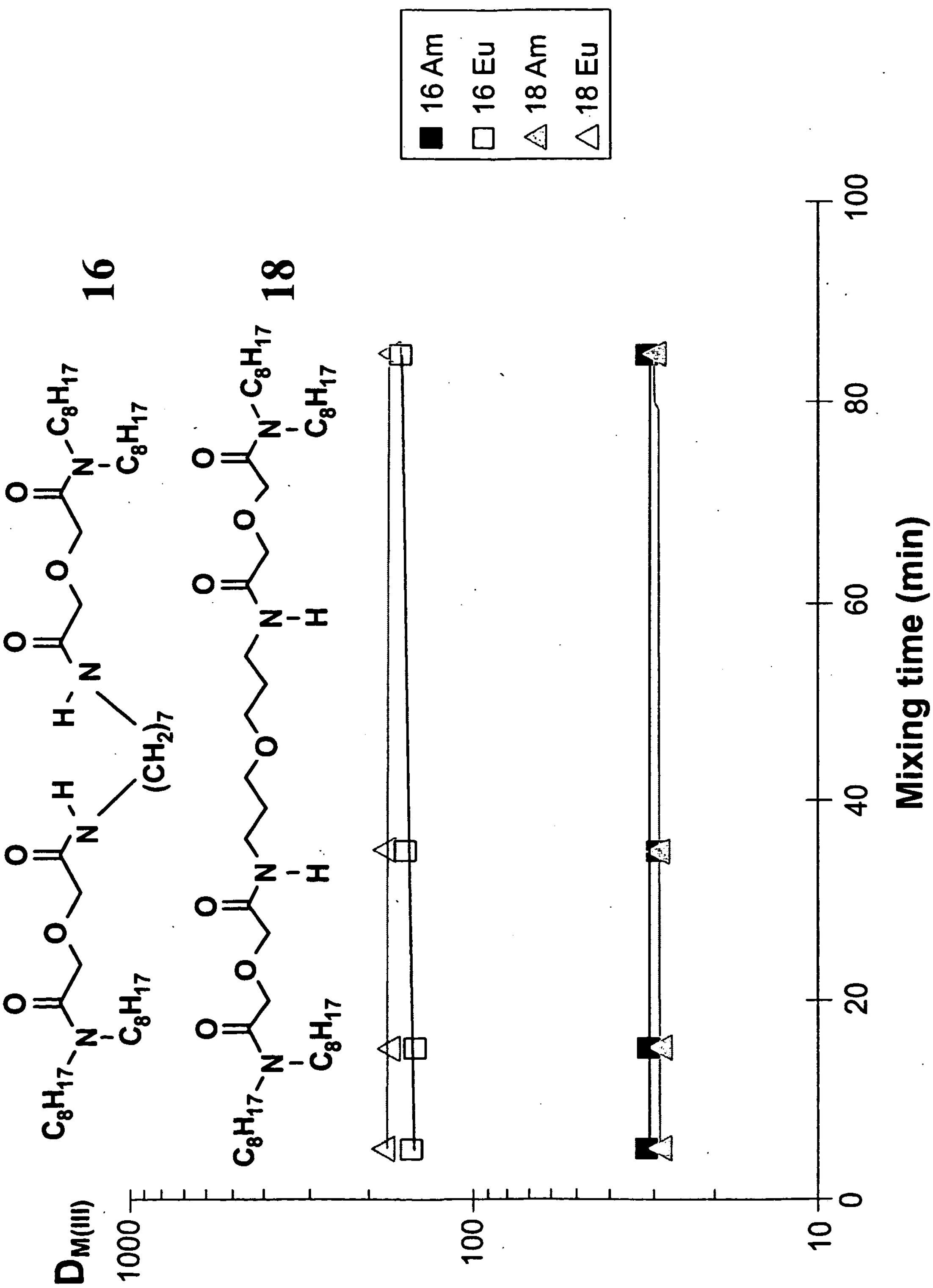
D_M(III)
1000
100
10
0
20
40
60
80
100
Mixing time (min)
16 Am
16 Eu
18 Am
18 Eu

16

18

FIG. 6

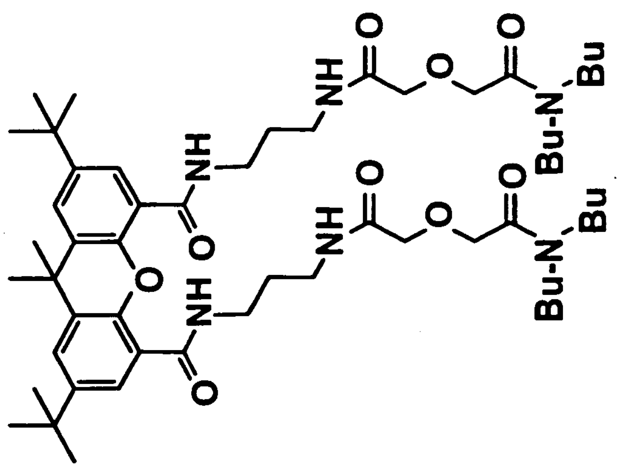

19

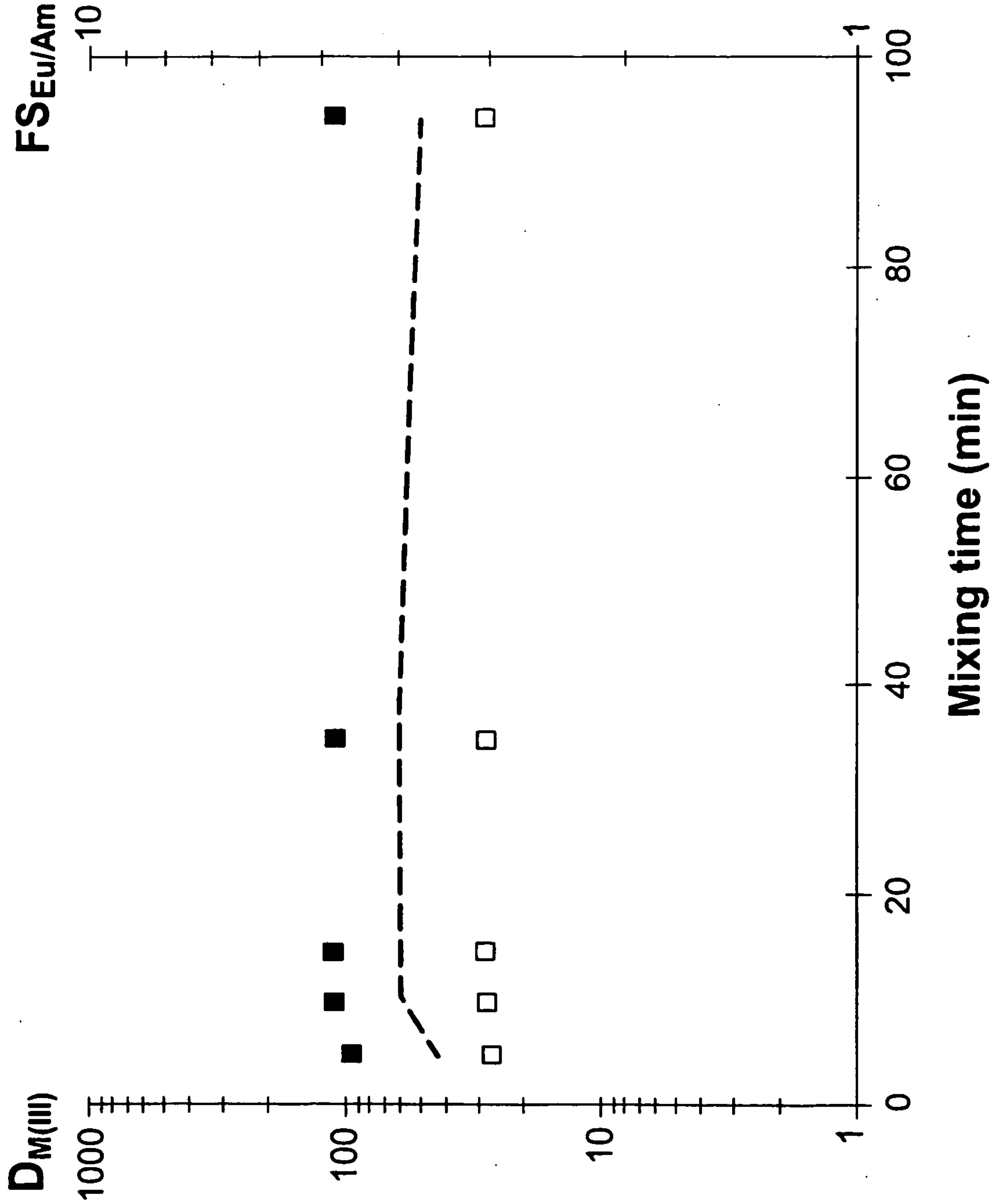
$D_{M(III)}$
1000
100
10
1
$FS_{Eu/Am}$
10
1
0
20
40
60
80
100
Mixing time (min)
Am
Eu

FIG. 7

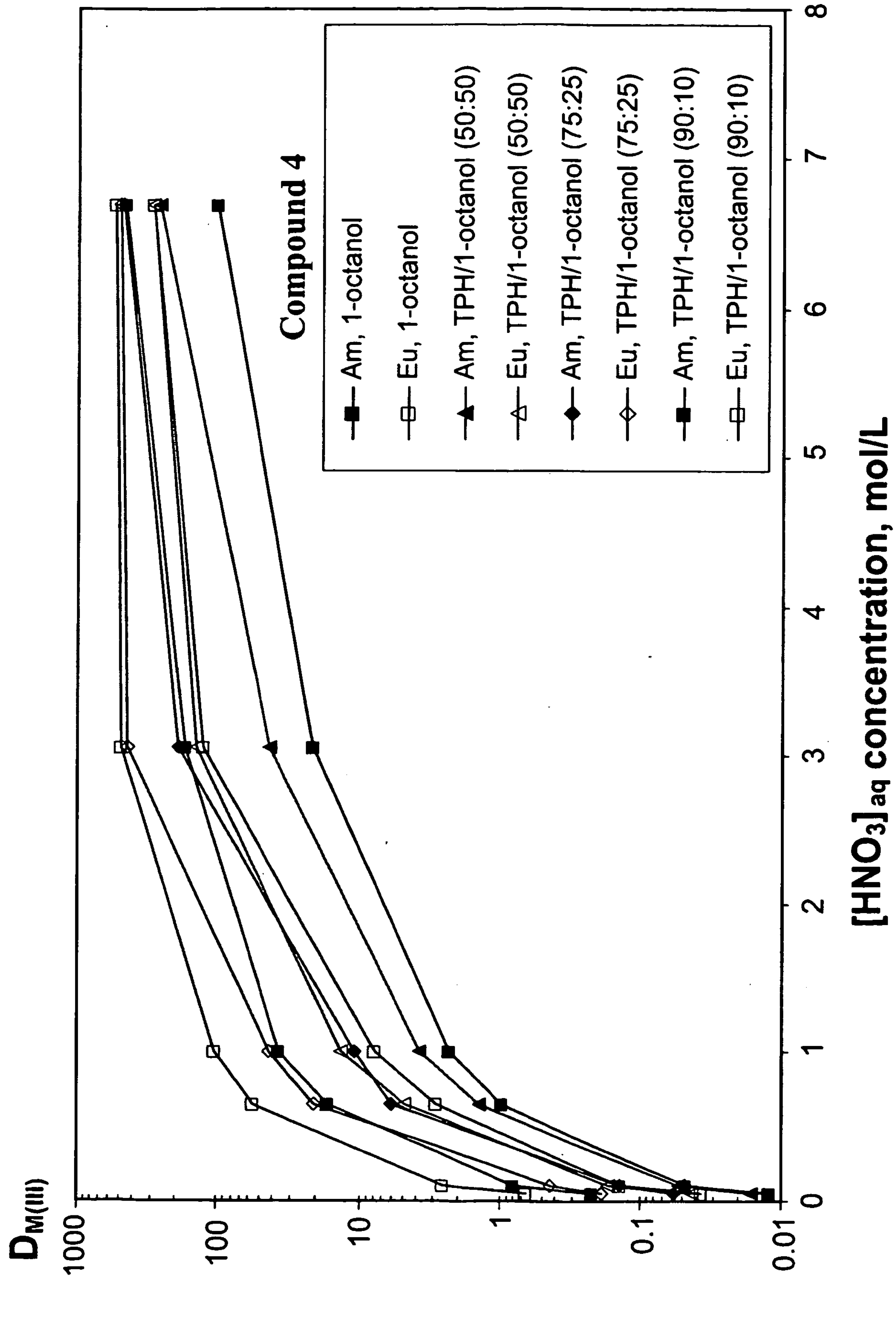
$D_{M(III)}$
1000
100
10
1
0.1
0.01
0
1
2
3
4
5
6
7
8
Compound 4
Am, 1-octanol
Eu, 1-octanol
Am, TPH/1-octanol (50:50)
Eu, TPH/1-octanol (50:50)
Am, TPH/1-octanol (75:25)
Eu, TPH/1-octanol (75:25)
Am, TPH/1-octanol (90:10)
Eu, TPH/1-octanol (90:10)
$[HNO_3]_{aq}$ concentration, mol/L

FIG. 8

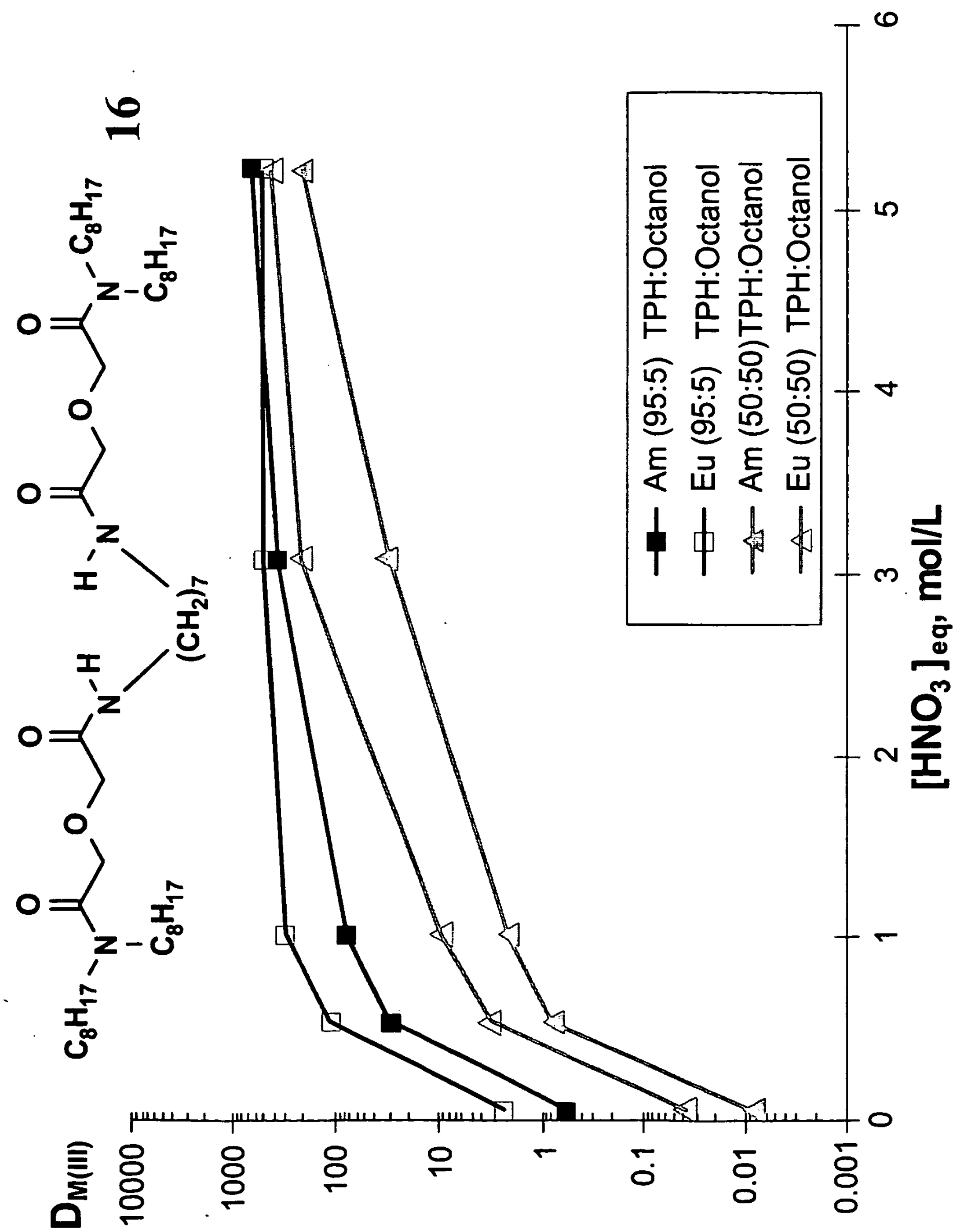

$D_{M(III)}$
10000
1000
100
10
1
0.1
0.01
0.001
0
1
2
3
4
5
6
$[HNO_3]_{eq}$, mol/L
Am (95:5) TPH:Octanol
Eu (95:5) TPH:Octanol
Am (50:50) TPH:Octanol
Eu (50:50) TPH:Octanol


16

FIG. 9

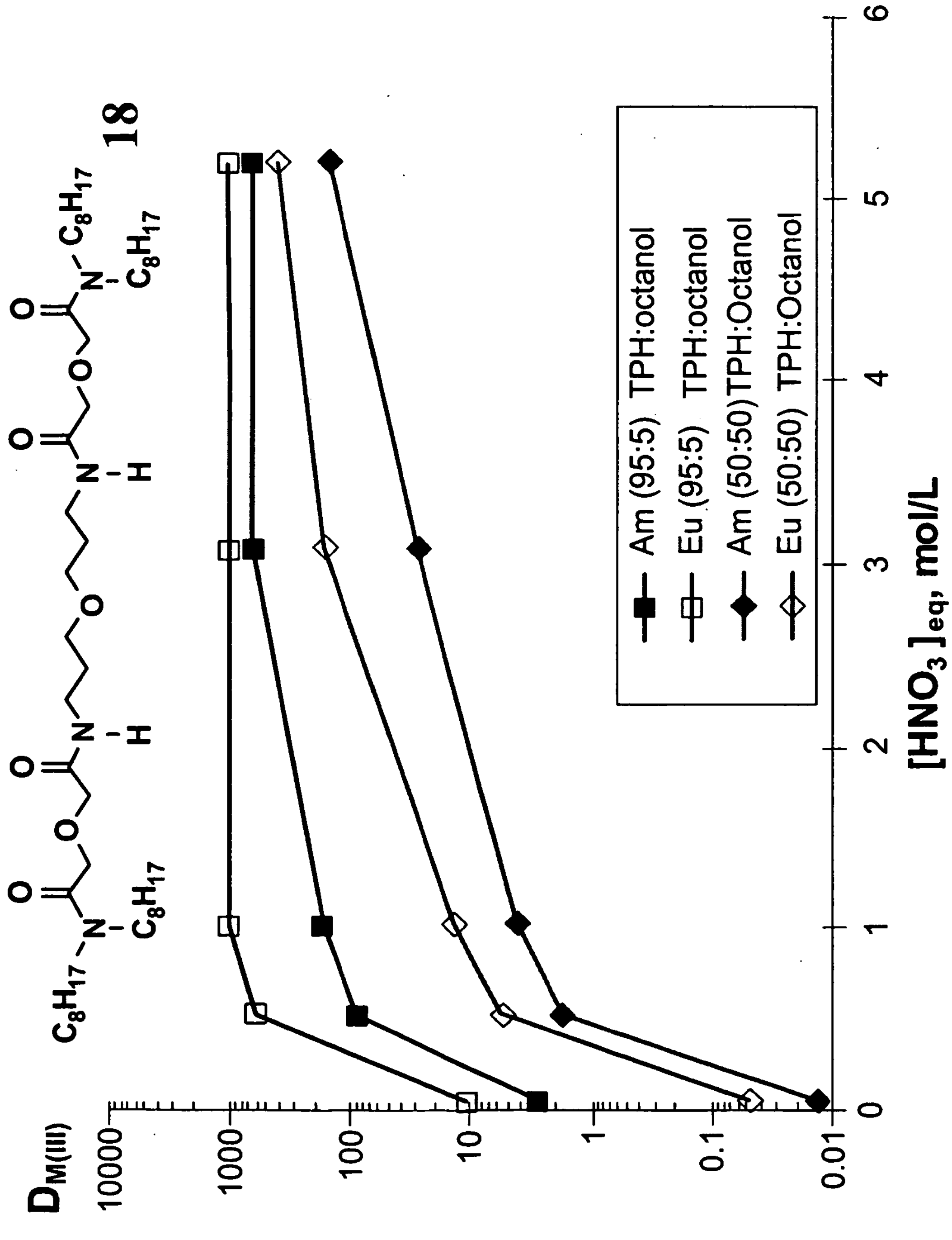

$D_{M(III)}$
10000
1000
100
10
1
0.1
0.01
0
1
2
3
4
5
6
Am (95:5) TPH:octanol
Eu (95:5) TPH:octanol
Am (50:50)TPH:Octanol
Eu (50:50) TPH:Octanol
$[HNO_3]_{eq}$, mol/L


18

FIG. 10

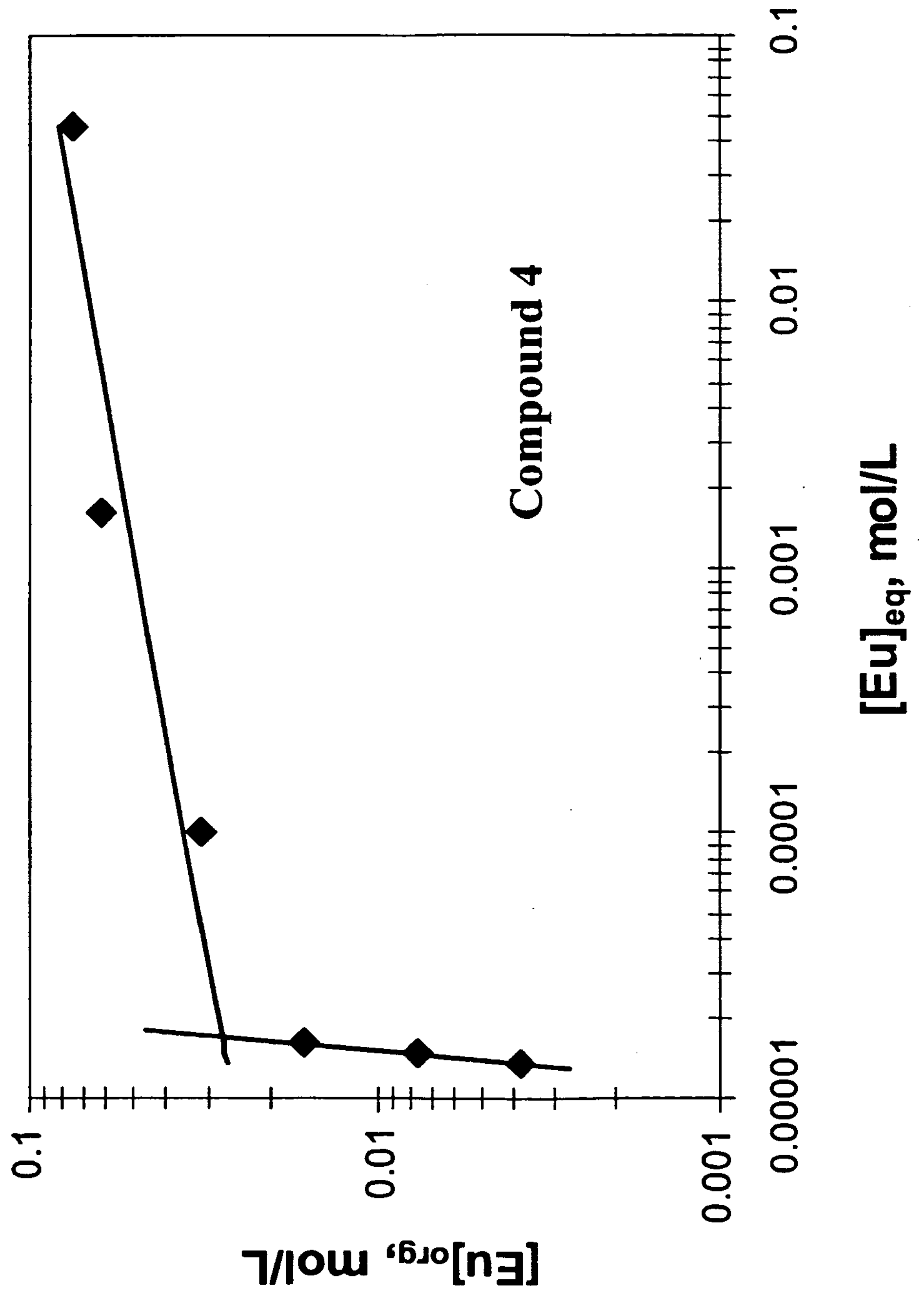

Compound 4
[Eu]org, mol/L
0.1
0.01
0.001
[Eu]eq, mol/L
0.000001
0.00001
0.0001
0.001
0.01
0.1


FIG. 11

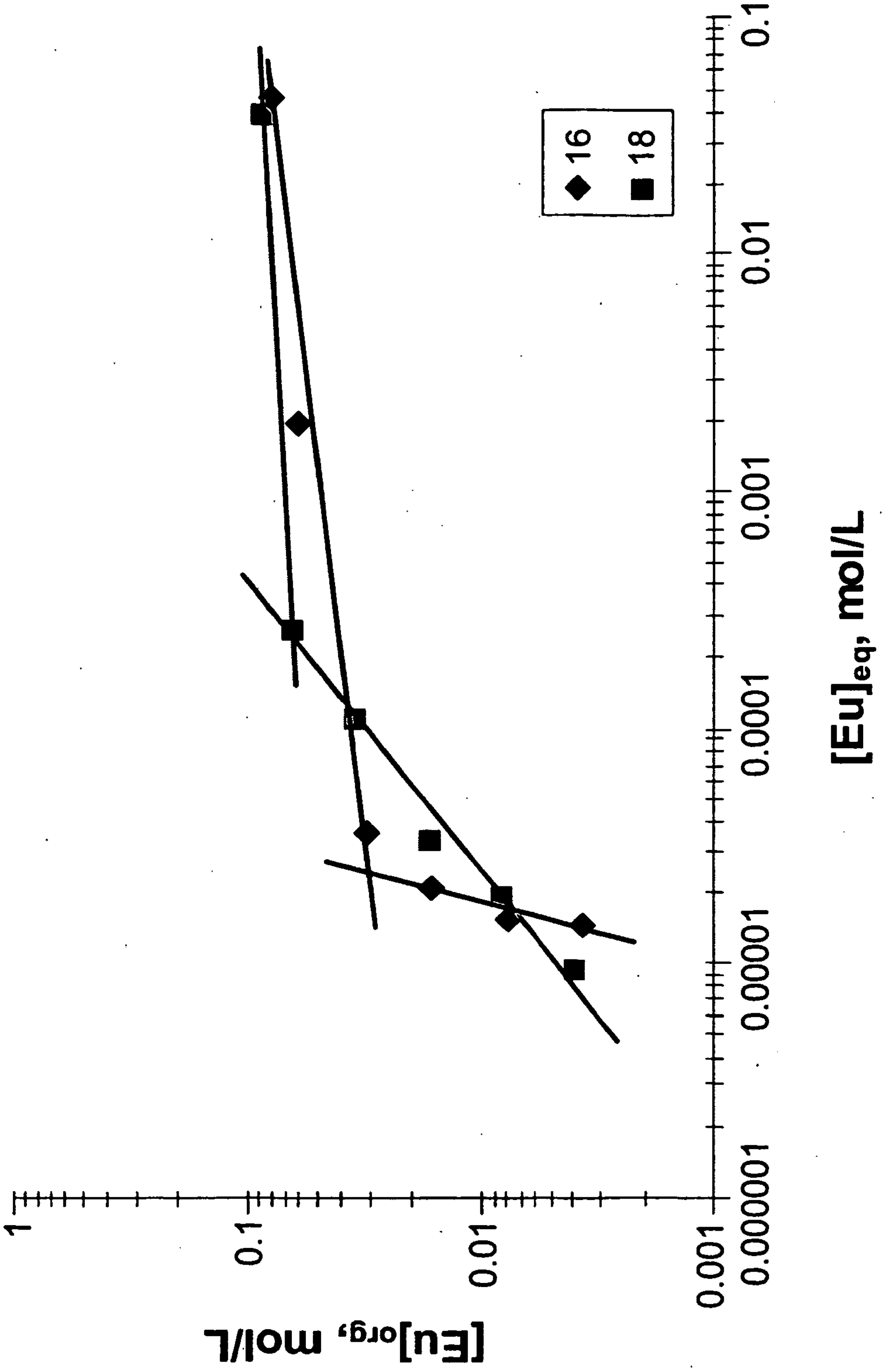
[Eu]$_{org}$, mol/L
1
0.1
0.01
0.001
0.0000001
0.000001
0.00001
0.0001
0.001
0.01
0.1
[Eu]$_{eq}$, mol/L
16
18

FIG. 12

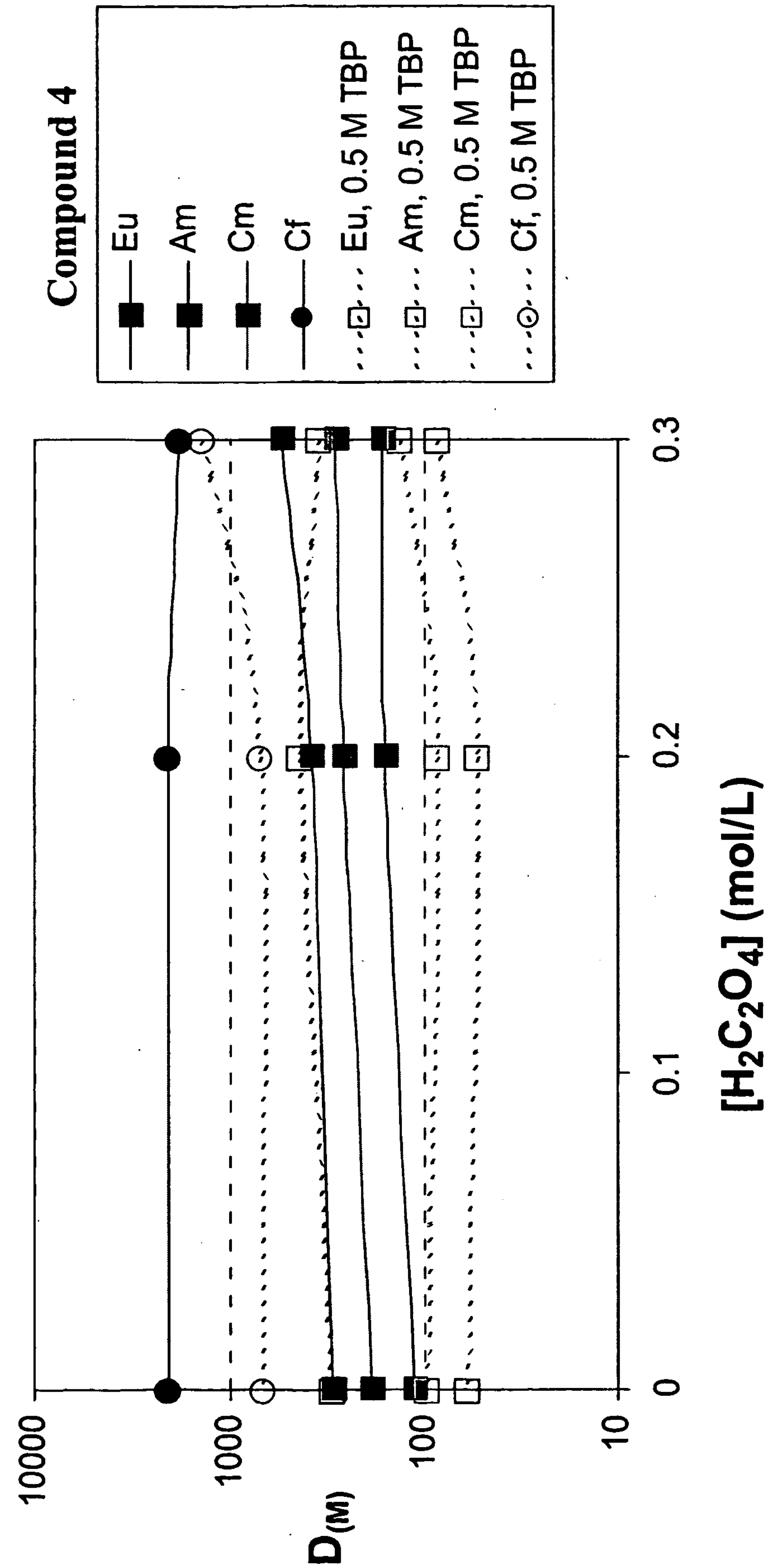
Compound 4
Eu
Am
Cm
Cf
Eu, 0.5 M TBP
Am, 0.5 M TBP
Cm, 0.5 M TBP
Cf, 0.5 M TBP
$D_{(M)}$
10000
1000
100
10
0
0.1
0.2
0.3
$[H_2C_2O_4]$ (mol/L)

FIG. 13

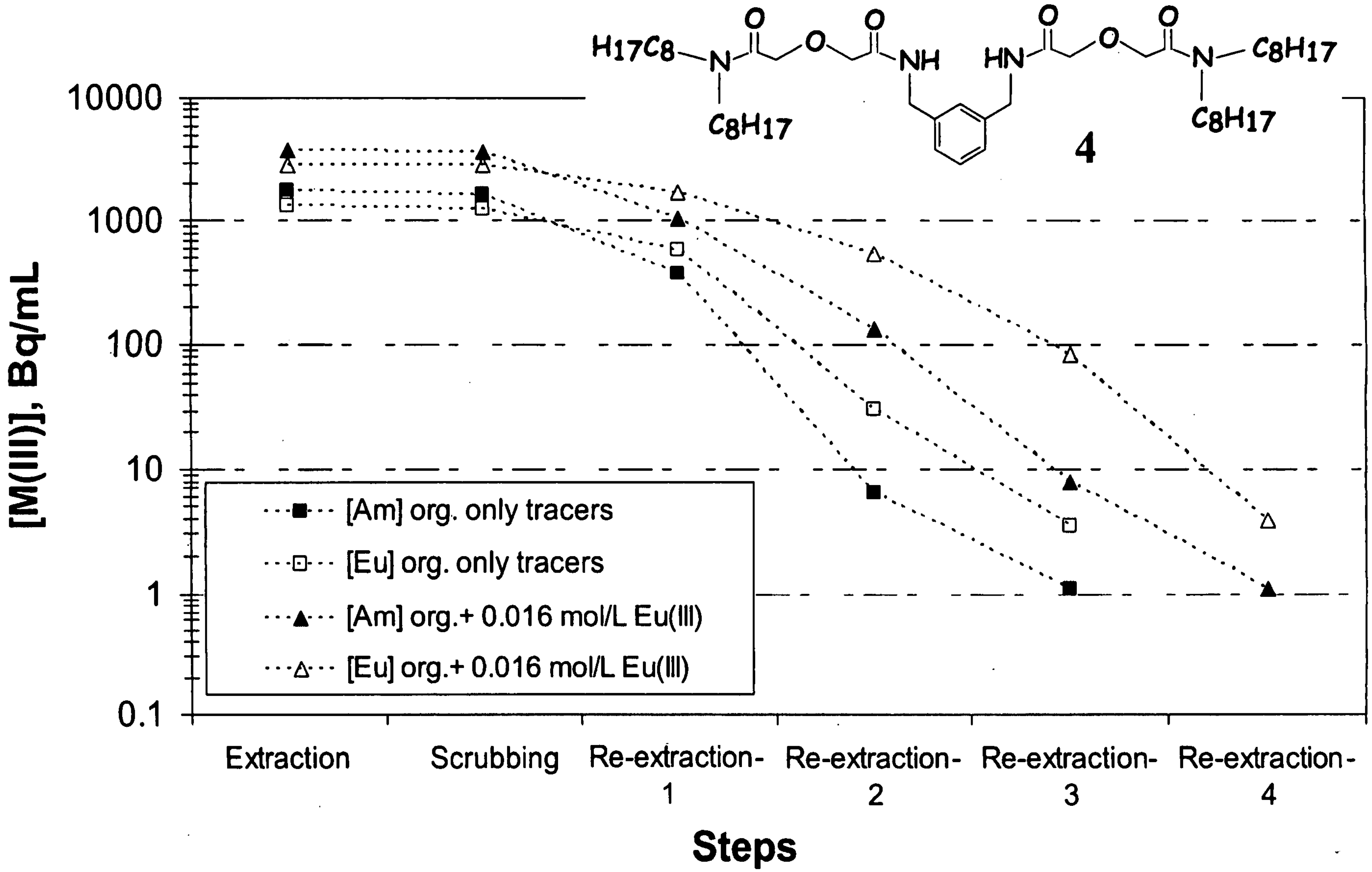
[M(III)], Bq/mL
10000
1000
100
10
1
0.1
[Am] org. only tracers
[Eu] org. only tracers
[Am] org.+ 0.016 mol/L Eu(III)
[Eu] org.+ 0.016 mol/L Eu(III)
Extraction
Scrubbing
Re-extraction-1
Re-extraction-2
Re-extraction-3
Re-extraction-4
Steps

4

FIG. 14

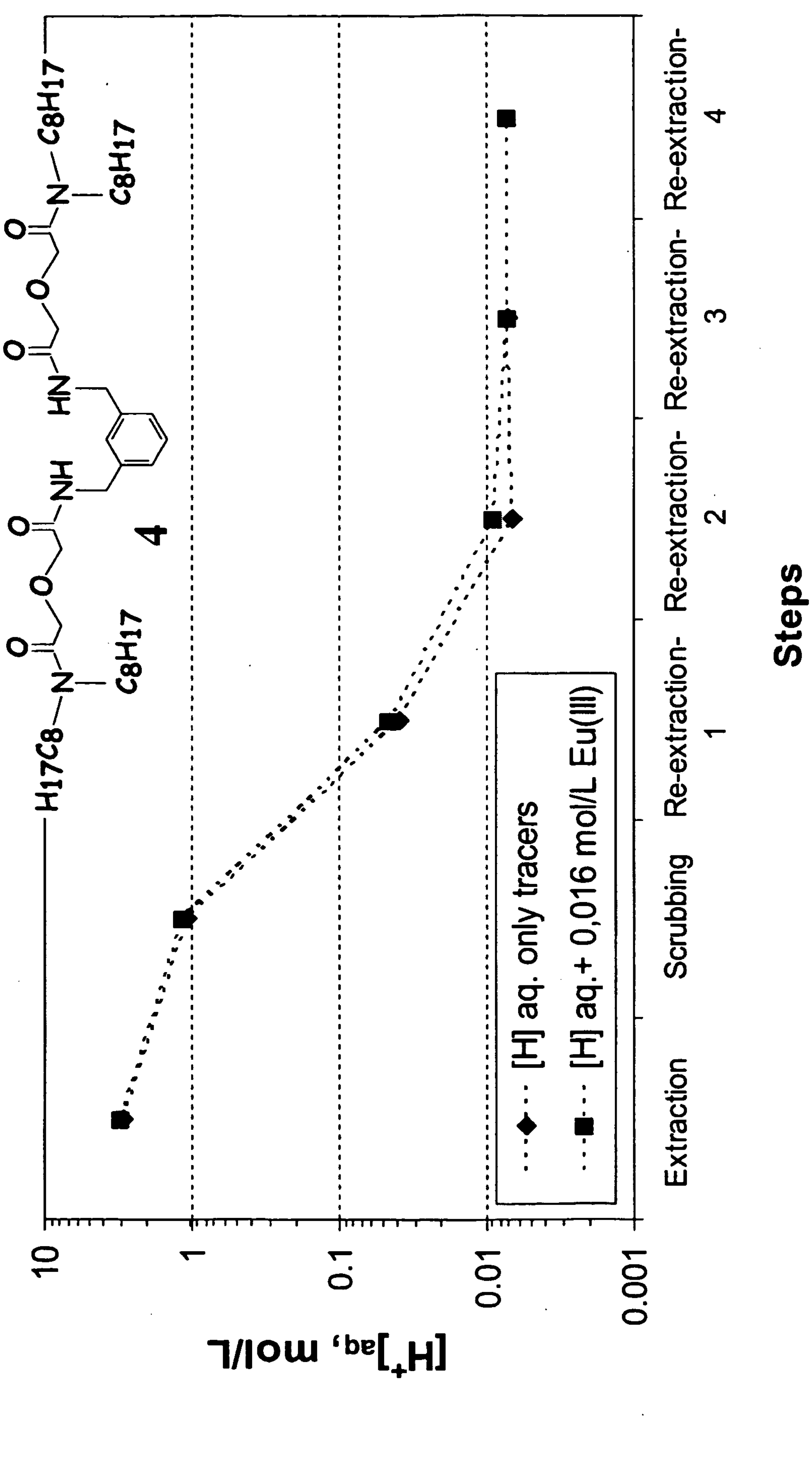
4
[H+]aq, mol/L
10
1
0.1
0.01
0.001
[H] aq. only tracers
[H] aq.+ 0,016 mol/L Eu(III)
Extraction
Scrubbing
Re-extraction-1
Re-extraction-2
Re-extraction-3
Re-extraction-4
Steps

FIG. 15

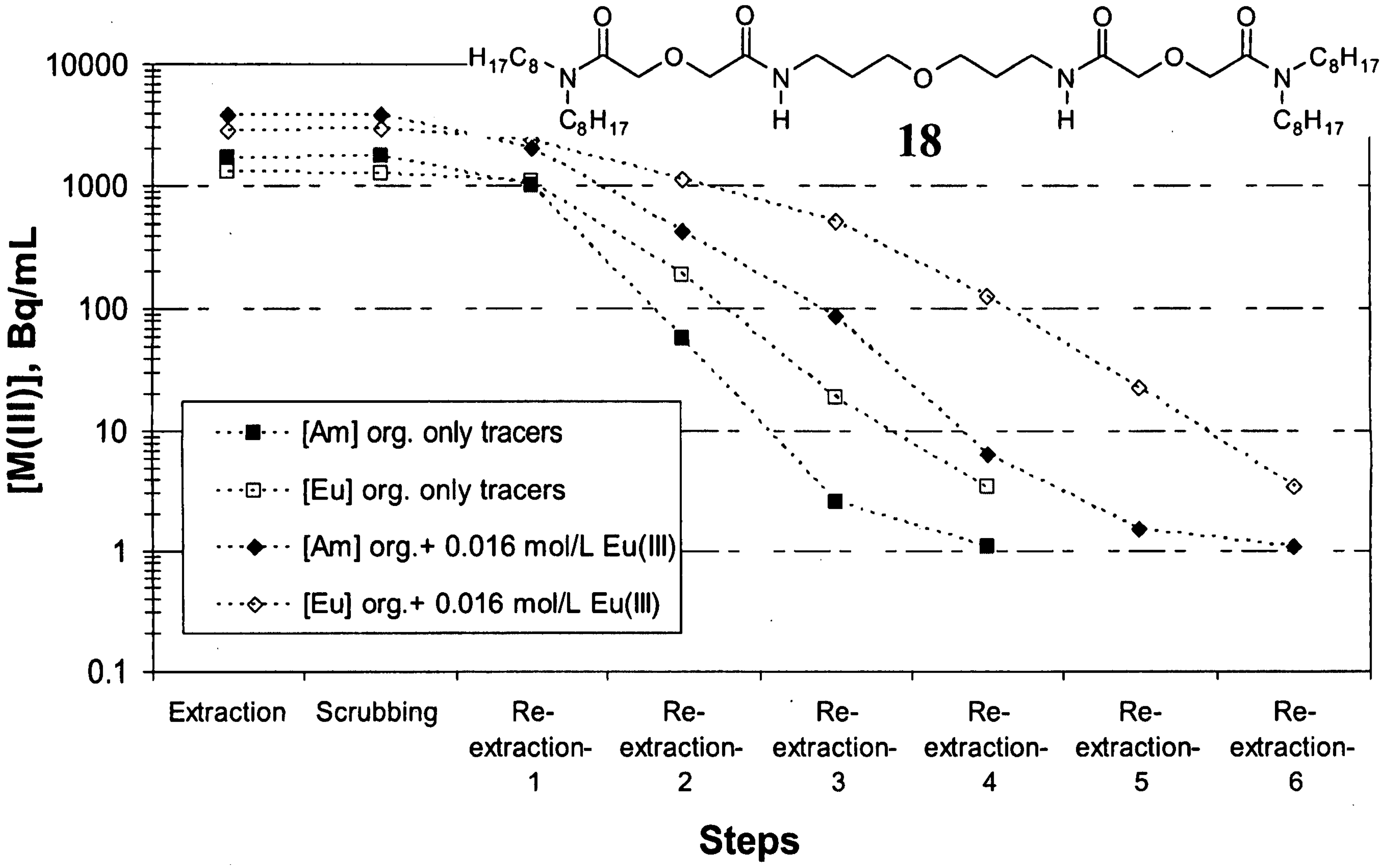
18
10000
1000
100
10
1
0.1
[M(III)], Bq/mL
[Am] org. only tracers
[Eu] org. only tracers
[Am] org.+ 0.016 mol/L Eu(III)
[Eu] org.+ 0.016 mol/L Eu(III)
Extraction
Scrubbing
Re-extraction-1
Re-extraction-2
Re-extraction-3
Re-extraction-4
Re-extraction-5
Re-extraction-6
Steps

FIG. 16

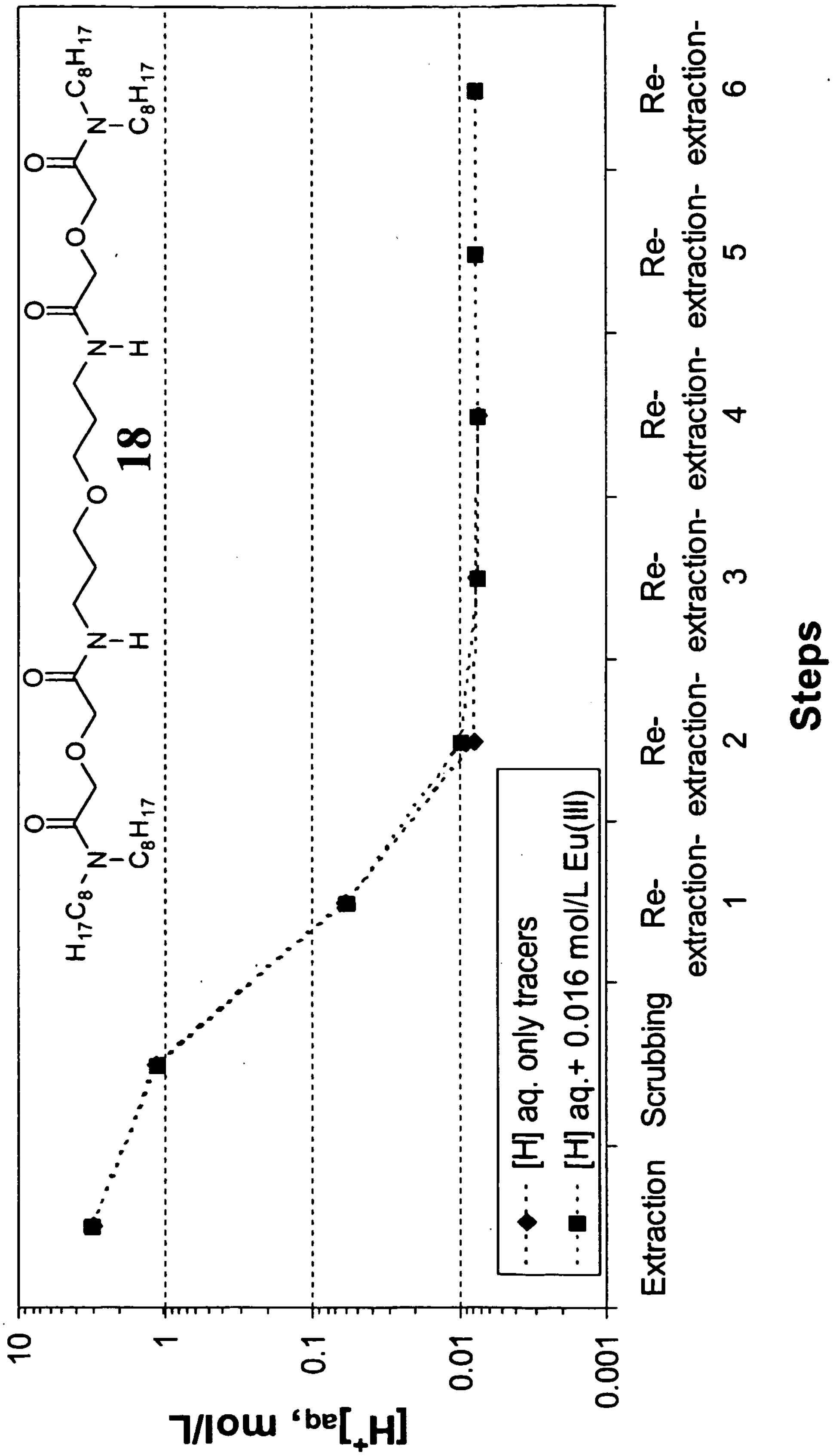
[H+]aq, mol/L
10
1
0.1
0.01
0.001
Extraction
Scrubbing
Re-extraction-1
Re-extraction-2
Re-extraction-3
Re-extraction-4
Re-extraction-5
Re-extraction-6
Steps
[H] aq. only tracers
[H] aq.+ 0.016 mol/L Eu(III)
18

FIG. 17

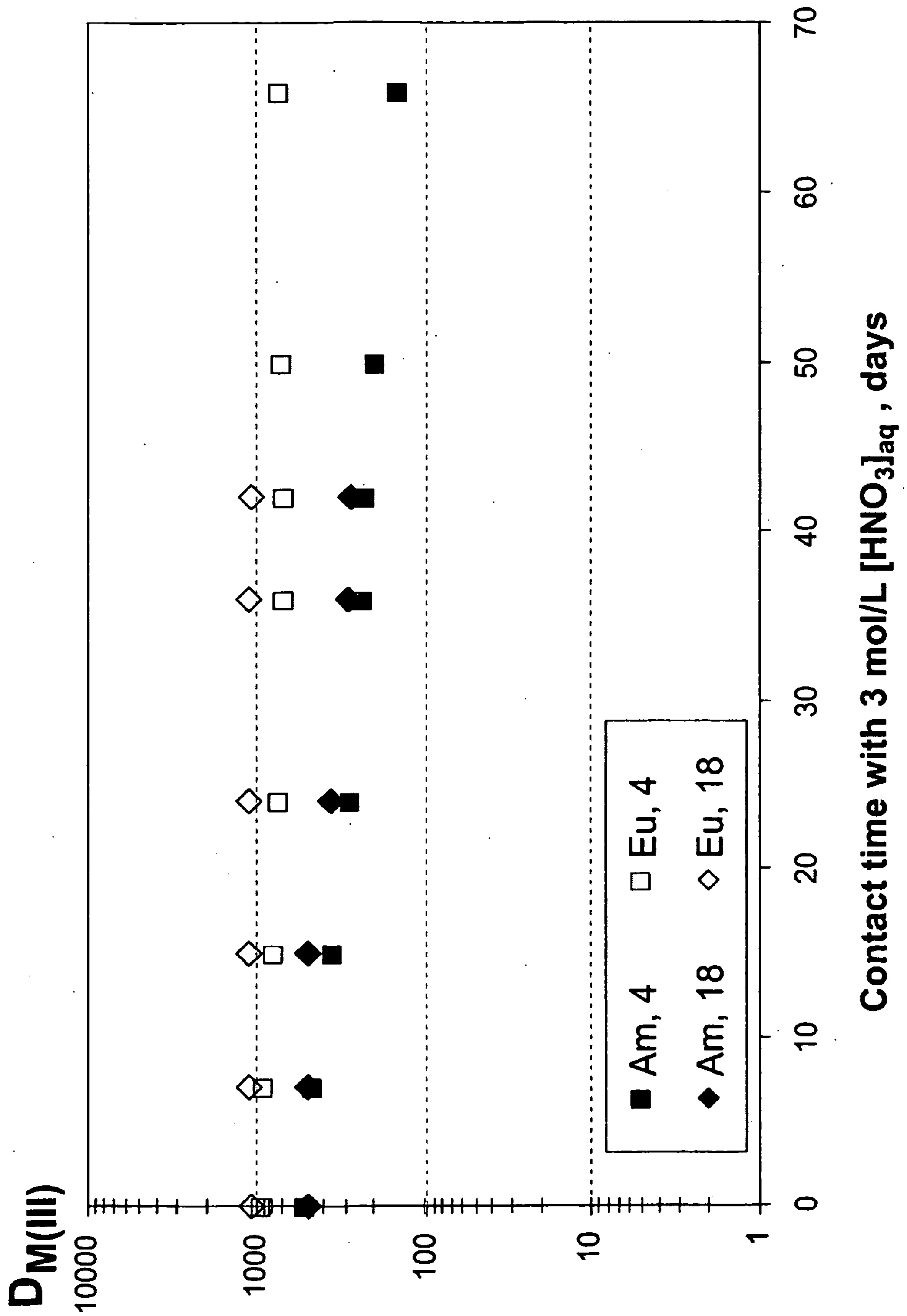

$D_{M(III)}$
10000
1000
100
10
1
0
10
20
30
40
50
60
70
Am, 4
Eu, 4
Am, 18
Eu, 18
Contact time with 3 mol/L $[HNO_3]_{aq}$ , days


FIG. 18

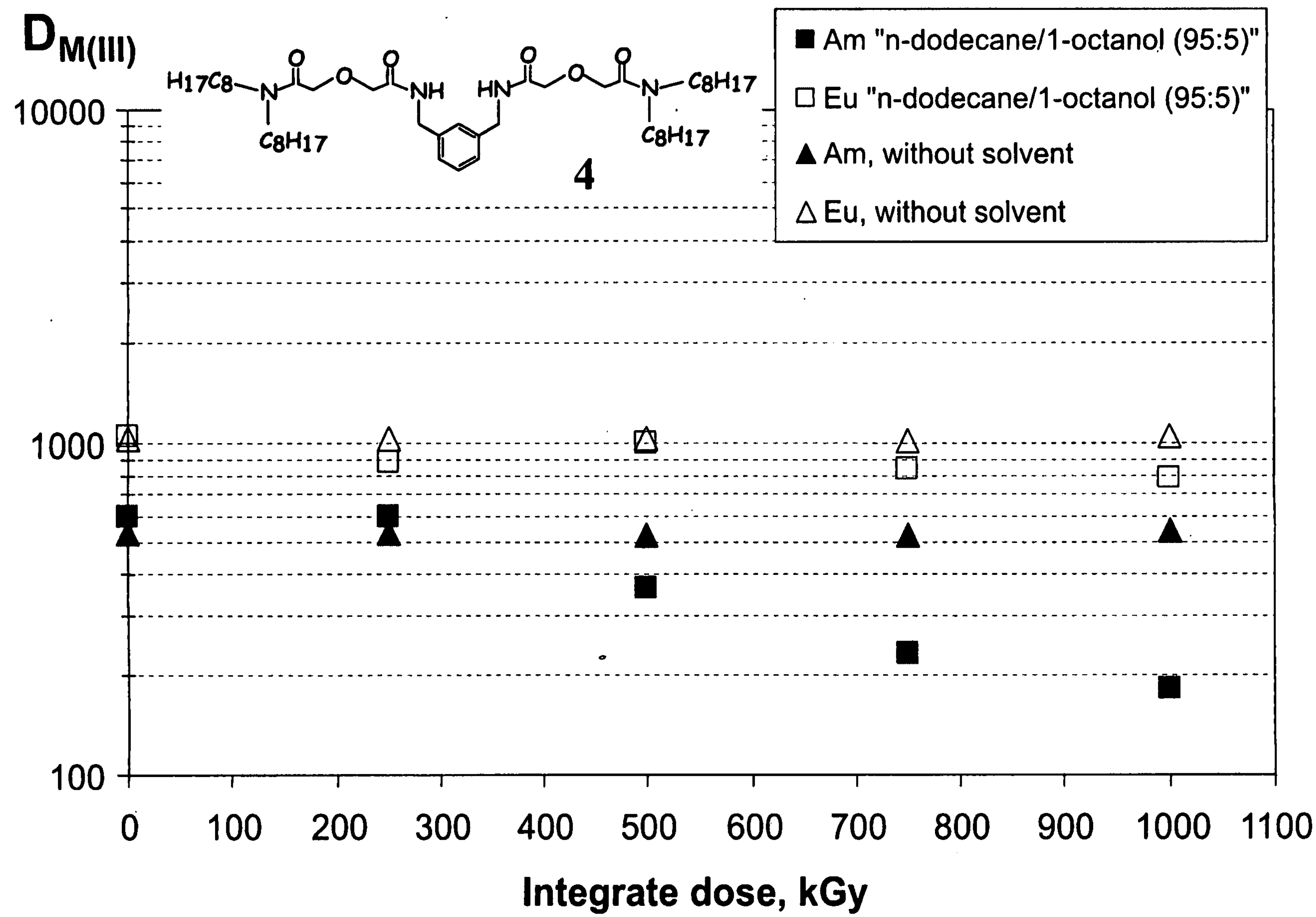
D_M(III)
10000
1000
100
4
Am "n-dodecane/1-octanol (95:5)"
Eu "n-dodecane/1-octanol (95:5)"
Am, without solvent
Eu, without solvent
0
100
200
300
400
500
600
700
800
900
1000
1100
Integrate dose, kGy

FIG. 19

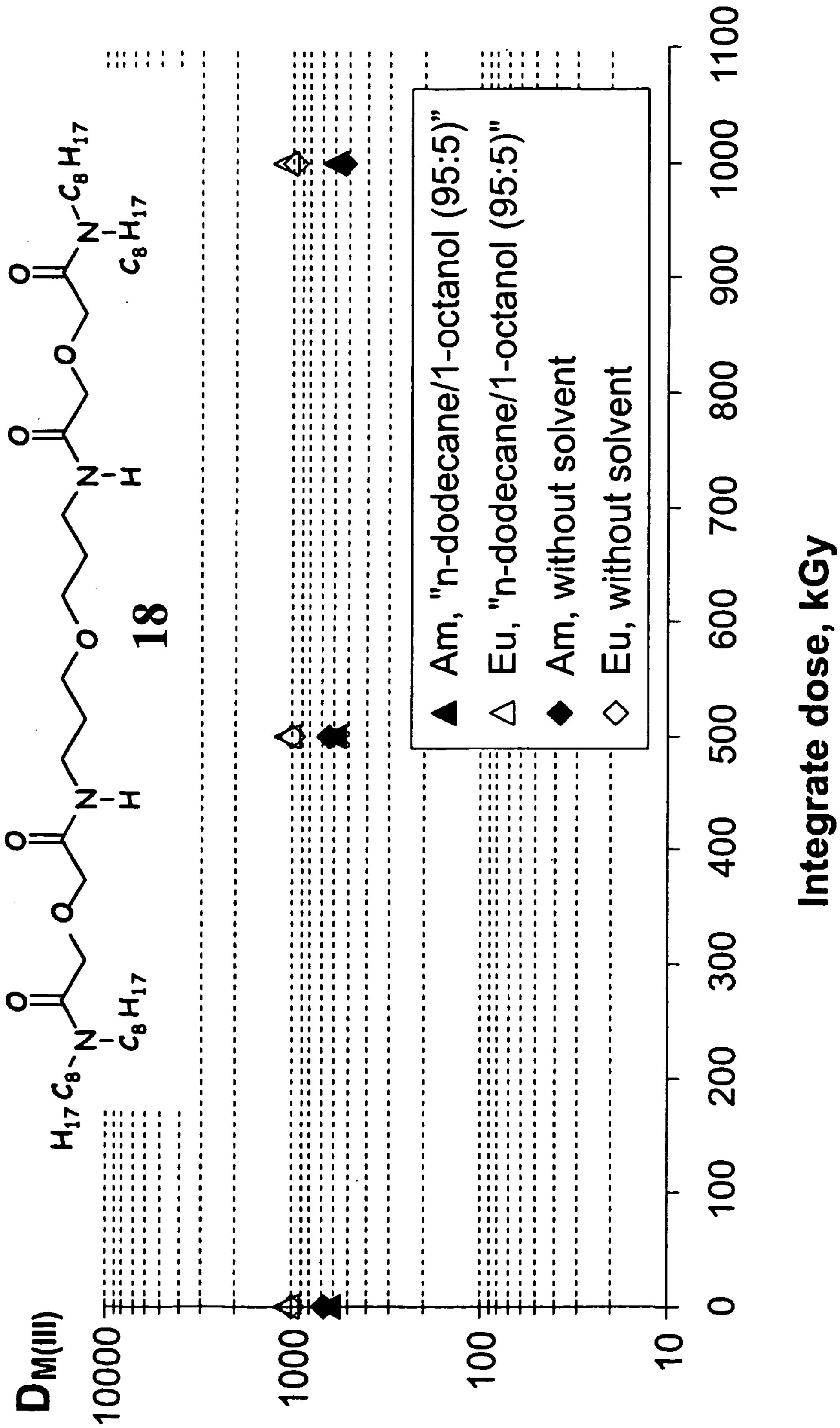
D_M(III)
10000
1000
100
10
0
100
200
300
400
500
600
700
800
900
1000
1100
Am, "n-dodecane/1-octanol (95:5)"
Eu, "n-dodecane/1-octanol (95:5)"
Am, without solvent
Eu, without solvent
Integrate dose, kGy

18

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5093795 B
- US 4572802 A
- FR 2585700
- US 4938871 A
- EP 0043765 A
- US 4461747 A
- CA 1197382
- US 4867951 A
- US 5256383 A
- FR 2810679
- JP 2003322699 B
- JP 2005114448 B


### Non-patent literature cited in the description


- **MCKAY, H. A. C.** The PUREX Process, Part I, Introduction in Science and Technology of Tributyl Phosphate. CRC Press, 1984
- **MILES, J. H.** The PUREX Process, Part II, Separation of Plutonium and Uranium in Science and Technology of Tributyl Phosphate. CRC Press, 1984
- **MCKIBBEN, J. M.** *Radiochim. Acta,* 1984, vol. 36, 3
- **SPJUTH, L ; LILJENZIN, J. O ; HUDSON, M. J ; DREW, M. G. B ; IVESON, P. B ; MADIC, C.** *Solvent Extr. Ion Exch,* 2000, vol. 18, 1
- **SASAKI, Y ; SUGO, Y ; SUZUKI, S ; TACHIMORI, S.** *Solvent Extraction and Ion Exchange,* 2001, vol. 19 (1), 91-103
- **SASAKI, Y ; TACHIMORI, S.** *Solvent Extraction and Ion Exchange,* 2002, vol. 20 (1), 21-34
- **TACHIMORI, S ; SASAKI, Y ; SUZUKI, S.** *Solvent Extraction and Ion Exchange,* 2002, vol. 20 (6), 687-699
- **YE, G. -A ; HE, J. -Y. ; JIANG, Y. -Q.** *He Huaxue Yu Fangshe Huaxue,* 2000, vol. 22 (2), 65-72
- **YE, G.-A ; HE, J.-Y ; LUO, F.-X.** *He Huaxue Yu Fangshe Huaxue,* 2000, vol. 22 (3), 136-143
- **CHEN, W.-J.; ; ZHU, L ; DING, S.-D ; LIU, Z.-Q ; CHEN, S.-J ; JIN, Y.-D.** *Gaodeng Xuexiao Huaxue Xuebao,* 1998, vol. 19 (11), 1724-1726
- **CHEN, W ; WEN, Y ; FAN, Q.** *He Huaxue Yu Fangshe Huaxue,* 1999, vol. 21 (3), 136-141
- **DING, S.-D ; CHEN, W.J ; XIAO, C.-J ; LU, Q ; LUO, H.-Y ; LIU, J.-F ; YE, G.-A ; ZHU, Z.-X ; TANG, H.-B ; LUO, F.-X.** *He Huaxue Yu Fangshe Huaxue,* 2003, vol. 25 (3), 188-192
- **STILL, W. C ; KAHN, M ; MITRA, A.** *J. Org. Chem.,* 1978, vol. 43, 2923
- **PERRIN, D. D ; ARMAREGO, W. L. F ; PERRIN, D. R.** Purification of Laboratory Chemicals. Pergamon Press, 1980
- **STEPHAN, H ; GLOE, K ; BEGER, J ; MUEHL, P.** *Solvent Ext. and Ion Exch.,* 1991, vol. 9, 435
- **STEPHAN, H ; GLOE, K ; BEGER, J ; MUEHL, P.** *Solvent Ext. and Ion Exch.,* 1991, vol. 9, 459
- **LE STANG, S ; MEIER, R ; ROCABOY, C ; GLADYSZ, J. A.** *J. Fluorine Chem.,* 2003, vol. 119, 141